# EUROPEAN PATENT APPLICATION

(11) **EP 4 806 920 A1**
(43) Date of publication of application: **16.09.2026**
(21) Application number: 25163979.5
(22) Date of filing: 14.03.2025
(51) Int. Cl.: C07K 16/28, C07K 16/40, A61P 35/00

(54) **CD38 ANTIBODIES AND FRAGMENTS THEREOF**

(71) Applicant: Georg-August-Universität Göttingen Stiftung Öffentlichen Rechts, 37073 Göttingen (DE)
(72) Inventor: The designation of the inventor has not yet been filed
(74) Representative: Fuchs Patentanwälte Partnerschaft mbB

(57) **Abstract**

The present disclosure relates to anti-CD38 antibodies and fragments thereof, therapeutic, diagnostic and surgical uses of such antibodies or fragments thereof, as well as methods of making these antibodies or fragments thereof. Further, this disclosure includes nucleic acids encoding the antibodies and fragments thereof, and host cells comprising such nucleic acids.

## Description

This disclosure relates to anti-CD38 antibodies and fragments thereof, therapeutic, diagnostic and surgical uses of such antibodies or fragments thereof, as well as methods of making these antibodies or fragments thereof. Further, this disclosure includes nucleic acids encoding the antibodies and fragments thereof, and host cells comprising such nucleic acids.

### Background

The glycoprotein CD38 ("Cluster of Differentiation 38") is a cell surface membrane protein widely expressed across various hematopoietic cells, including bone marrow progenitors, monocytes and activated immune cells like T-lymphocytes, natural killer cells, and plasma cells (which are derived from B-lymphocytes). It plays a crucial role in cell adhesion, signaling and enzymatic activity.

Functioning as a multifunctional ectoenzyme with hydrolase and cyclase activity, CD38 is involved in calcium signaling and immune regulation and plays an important role in the regulation of immune cell activation and proliferation.

CD38 is highly expressed on plasma cells, which are essential for immune defense through antibody production. Due to its pivotal involvement in hematopoiesis and immune responses, CD38 has become a target of interest in various hematologic diseases.

One such disease is multiple myeloma, a cancer of the hematopoietic system that originates in plasma cells, which are differentiated, antibody producing B-cells. These cancerous plasma cells grow uncontrollably, disrupting the body's ability to fight infections and leading to an overproduction of antibodies and to the production of abnormal antibodies. Multiple myeloma often remains asymptomatic until it has advanced significantly, which complicates early diagnosis.

As a form of blood cancer, multiple myeloma falls within the broader category of hematologic cancers, which include leukemia, lymphoma, and myeloma. These types of cancer impact blood cells in various ways, affecting normal blood cell function and leading to a range of symptoms.

Accordingly, CD38 is expressed in high concentrations on the surface of myeloma, lymphoma, and leukemic cells, and has therefore sparked significant interest in developing therapeutic strategies aiming at it as a target. Tagging these cancer cells with antibodies helps the immune system recognize and neutralize them and could also be used to improve the poor chances for early diagnosis.

Fully differentiated B-cells show a high expression of the CD38 receptor on their surface. Therefore, antibodies against CD38 are of particular interest for developing treatment and diagnosis methods of blood cancer types, the treatment of multiple myeloma having become one of the most important applications of anti-CD38 antibodies.

An example of an antibody that is used to target CD38 on multiple myeloma cells is daratumumab. Daratumumab is a human IgG1-kappa antibody, which mainly neutralizes cancer cells by inducing complement-dependent cytotoxicity (CDC) and antibody-dependent cellular phagocytosis (ADCP).

Isatuximab is another example of an anti-CD38 antibody used in the treatment of multiple myeloma. It is a IgG1-kappa antibody, whose main mode of action against multiple myeloma cells involves cross-linking CD38 and completely inhibiting its ectoenzymatic activity, seriously affecting the cell's energy metabolism and division. Thus, isatuximab mainly relies on inducing antibody-dependent cellular toxicity (ADCC).

An effective method to treat cancer cells are combination therapies, desirable in the treatment of cancer because of the advantages they bring.

Cancer cells, including those in multiple myeloma, can develop resistance to single therapies over time. By using different drugs with distinct mechanisms of action, combination therapies reduce the likelihood that the cancer will develop a resistance to all of them, enhancing the effectiveness of the treatment and improving long-term outcomes.

However, daratumumab and isatuximab cannot be used simultaneously in a combination therapy. Even though they do not bind to the exact same epitope on the CD38 receptor, their epitopes are close enough to result in sterical hindrance of the bulky antibody formats, as both the heavy and the light chains are needed for a specific binding to the epitopes.

Furthermore, the affinity of an antibody to its target has a strong influence on the amount of antibody that needs to be used to achieve therapeutical effects. The higher the affinity of the antibody, the lower the amount of antibody that needs to be employed to achieve therapeutical effects and accordingly the lower the price of the therapy is for the patient.

Currently, antibody treatments with daratumumab and isatuximab are expensive and unfortunately, not all patients in need of these therapeutic drugs can benefit from them.

The currently used methods for antibody screening focus on identifying antibodies that specifically bind to certain target proteins. In order to achieve this, however, the proteins are often isolated or even denatured, alienating them from their natural, physiological three-dimensional shape. Therefore, when implemented in practice, the antibodies identified by current screening methods tend to have a lower affinity for their designated target in living organisms than they had in the performed screening procedure. This significantly increases the concentration of therapeutical antibodies needed in order to achieve therapeutical effects, and consequently also the cost of the therapy for the patients.

### Brief Description of the Disclosure

The following aspects of this disclosure overcome at least some of the drawbacks of the prior art.

In a first aspect, this disclosure relates to an antibody or fragment thereof binding to CD38, selected from
(i) an antibody or fragment thereof competing with a reference antibody for binding to CD38, and
(ii) the reference antibody, wherein
the reference antibody comprises the sequence of SEQ ID No. 2.

In a second aspect, this disclosure relates to an antibody or fragment thereof comprising
(i) complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8, optionally having framework regions, FR1, FR2, FR3 and FR4, wherein FR1 has a sequence identity of at least 90% with SEQ ID No. 3, FR2 has a sequence identity of at least 90% with SEQ ID No. 5, FR3 has a sequence identity of at least 90% with SEQ ID No. 7, and/or FR4 has a sequence identity of at least 90% with SEQ ID No. 9,
(ii) complementarity-determining regions CDR1, CDR2 and CDR3, wherein CDR1 has an amino acid sequence SEQ ID No. 13, CDR2 has an amino acid sequence SEQ ID No. 15, and CDR3 has an amino acid sequence SEQ ID No. 17, optionally having framework regions, FR1, FR2, FR3 and FR4, wherein FR1 has a sequence identity of at least 90% with SEQ ID No. 12, FR2 has a sequence identity of at least 90% with SEQ ID No. 14, FR3 has a sequence identity of at least 90% with SEQ ID No. 16, and/or FR4 has a sequence identity of at least 90% with SEQ ID No. 18,
(iii) complementarity-determining regions CDR1, CDR2 and CDR3, wherein CDR1 has an amino acid sequence SEQ ID No. 22, CDR2 has an amino acid sequence SEQ ID No. 24, and CDR3 has an amino acid sequence SEQ ID No. 26, optionally having framework regions, FR1, FR2, FR3 and FR4, wherein FR1 has a sequence identity of at least 90% with SEQ ID No. 21, FR2 has a sequence identity of at least 90% with SEQ ID No. 23, FR3 has a sequence identity of at least 90% with SEQ ID No. 25, and/or FR4 has a sequence identity of at least 90% with SEQ ID No. 27,
(iv) complementarity-determining regions CDR1, CDR2 and CDR3, wherein CDR1 has an amino acid sequence SEQ ID No. 31, CDR2 has an amino acid sequence SEQ ID No. 33, and CDR3 has an amino acid sequence SEQ ID No. 35, optionally having framework regions, FR1, FR2, FR3 and FR4, wherein FR1 has a sequence identity of at least 90% with SEQ ID No. 30, FR2 has a sequence identity of at least 90% with SEQ ID No. 32, FR3 has a sequence identity of at least 90% with SEQ ID No. 34, and/or FR4 has a sequence identity of at least 90% with SEQ ID No. 36,
(v) complementarity-determining regions CDR1, CDR2 and CDR3, wherein CDR1 has an amino acid sequence SEQ ID No. 40, CDR2 has an amino acid sequence SEQ ID No. 42, and CDR3 has an amino acid sequence SEQ ID No. 44, optionally having framework regions, FR1, FR2, FR3 and FR4, wherein FR1 has a sequence identity of at least 90% with SEQ ID No. 39, FR2 has a sequence identity of at least 90% with SEQ ID No. 41, FR3 has a sequence identity of at least 90% with SEQ ID No. 43, and/or FR4 has a sequence identity of at least 90% with SEQ ID No. 45,
(vi) complementarity-determining regions CDR1, CDR2 and CDR3, wherein CDR1 has an amino acid sequence SEQ ID No. 51, CDR2 has an amino acid sequence SEQ ID No. 53, and CDR3 has an amino acid sequence SEQ ID No. 55, optionally having framework regions, FR1, FR2, FR3 and FR4, wherein FR1 has a sequence identity of at least 90% with SEQ ID No. 50, FR2 has a sequence identity of at least 90% with SEQ ID No. 52, FR3 has a sequence identity of at least 90% with SEQ ID No. 54, and/or FR4 has a sequence identity of at least 90% with SEQ ID No. 56,
(vii) complementarity-determining regions CDR1, CDR2 and CDR3, wherein CDR1 has an amino acid sequence SEQ ID No. 60, CDR2 has an amino acid sequence SEQ ID No. 62, and CDR3 has an amino acid sequence SEQ ID No. 64, optionally having framework regions, FR1, FR2, FR3 and FR4, wherein FR1 has a sequence identity of at least 90% with SEQ ID No. 59, FR2 has a sequence identity of at least 90% with SEQ ID No. 61, FR3 has a sequence identity of at least 90% with SEQ ID No. 63, and/or FR4 has a sequence identity of at least 90% with SEQ ID No. 65, or
(viii) complementarity-determining regions CDR1, CDR2 and CDR3, wherein CDR1 has an amino acid sequence SEQ ID No. 71, CDR2 has an amino acid sequence SEQ ID No. 73, and CDR3 has an amino acid sequence SEQ ID No. 75, optionally having framework regions, FR1, FR2, FR3 and FR4, wherein FR1 has a sequence identity of at least 90% with SEQ ID No. 70, FR2 has a sequence identity of at least 90% with SEQ ID No. 72, FR3 has a sequence identity of at least 90% with SEQ ID No. 74, and/or FR4 has a sequence identity of at least 90% with SEQ ID No. 76.

In a third aspect, this disclosure relates to an antibody or fragment thereof binding to CD38, comprising complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8.

Advantageously, the antibody or fragment according to the first, second and third bind CD38 at a position that allows for simultaneous binding of the antibody or fragment of this disclosure and isatuximab. The antibody or fragment of this disclosure may bind an epitope on CD38 that competes with daratumumab for binding and allows for various therapeutic and diagnostic functions and properties as discussed in more detail herein. Generally, the antibody or fragment thereof according to this disclosure may be an isolated antibody. It was found that the antibodies and fragments of this disclosure may inhibit the cyclase and/or hydrolase activity of human CD38. Further, the antibodies and fragments of this disclosure may induce complement-dependent cytotoxicity (CDC) and/or antibody-dependent cellular cytotoxicity (ADCC).

The antibody or fragment thereof binding to CD38 according to the first, second or third aspect may relate to a single-domain antibody ("nanobody"), a minibody, a bispecific antibody or minibody (able to bind to CD38 and to another epitope or molecular target), an antibody, a trimeric single-domain antibody and/or to humanized variants thereof.

The antibody or fragment thereof binding to CD38 according to the first, second or third aspect may relate to monovalent single-domain antibodies, multivalent single-domain antibodies, monovalent human recombinant heavy chain antibody, and multivalent human recombinant heavy chain antibody and/or humanized variants thereof.

In a fourth aspect, the antibody or fragment thereof according to the first aspect is used in a method of therapy, diagnosis *in vivo* or *in vitro* or surgery, wherein
(i) optionally the antibody or fragment thereof is for use in theranostics,
(ii) optionally the method comprises imaging by Positron Emission Tomography or Single-Photon Emission Computed Tomography, in particular when the antibody or fragment is conjugated to a radionuclide, and/or
optionally, the *in vitro* method is a method for analysis or diagnosis of a disease in a sample, comprising contacting the antibody or fragment thereof according to one or more of claims 1 to 9 with the sample.

The antibody or fragment thereof binding may be incorporated in CAR-T cell therapy.

The method of therapy may be administering an RNA vaccine, wherein the RNA is coding for an antibody or fragment thereof according to the present disclosure.

In a fifth aspect, the antibody or fragment is used in
(i) a method of treating hematologic diseases, such as CD38 positive cancer, cancer overexpressing CD38, Multiple Myeloma (MM), Chronic Lymphatic Leukemia (CLL), Acute Lymphoblastic Leukemia (ALL), Non-Hodgkin's Lymphoma (NHL), Diffuse Large B-cell Lymphoma (DLBCL) or Light Chain Amyloidosis;
(ii) a method of treating autoimmune diseases such as Rheumatoid Arthritis (RA);
(iii) a method of treating neurodegenerative diseases such as Alzheimer's Disease (AD), Parkinson's Disease (PD), Amyotrophic Lateral Sclerosis (ALS) or Multiple Sclerosis (MS); or
(iv) a method of immunosuppression, e.g. preventing transplant rejection following organ transplantation,
wherein
- optionally the antibody or fragment thereof is used in combination with isatuximab;
- optionally the antibody or fragment thereof is used as an RNA-vaccine; and/or
- optionally the antibody or fragment thereof is used in CAR T-cell therapy.

Methods of therapeutic, diagnostic, surgical or theranostic use may include administering to a subject in need thereof an effective amount of the antibody or fragment thereof, the pharmaceutical composition or the nucleic acid according to this disclosure.

Advantageously, the higher affinity of the antibodies or fragments thereof effectively reduces the concentration of the single-domain antibodies needed in therapy, diagnosis or surgery in order to achieve therapeutic or diagnostic effects and thus lowers the cost of the therapy for patients in need thereof. Further advantageously, the higher affinity effectively reduces the concentration of the antibody or fragments thereof needed in therapy to achieve therapeutic effects and thus lowers the risk of off-target binding, increasing the specificity of the single-domain antibodies. Further advantageously, the antibody or fragment thereof can penetrate into target tissue deeper and more easily than conventional antibodies due to its small size. In an embodiment, the antibody or fragment thereof can pass the blood-brain barrier due to its small size.

In a sixth aspect, this disclosure relates to a nucleic acid molecule comprising at least one nucleic acid sequence encoding at least partially or essentially completely the antibody or fragment thereof according to the first, second or third aspect of this disclosure.

In an eighth aspect, this disclosure relates to a method of manufacturing the antibody or fragment thereof according to the first, second or third aspect of this disclosure, comprising expressing the antibody or fragment thereof in a host cell, optionally in a host cell according to the seventh aspect, and isolating the antibody or fragment thereof.

In a ninth aspect, this disclosure relates to a pharmaceutical composition comprising the antibody or fragment thereof according to the first, second or third aspect, or the nucleic acid molecule according to the sixth aspect of this disclosure and a pharmaceutically acceptable excipient.

Whether an antibody competes with a reference antibody for binding to CD38 can be determined according to the experiment described in Example 8 & Example 15, wherein CD38 is first incubated with the sample antibody or fragment and then with a fluorescently labelled reference antibody. If the sample antibody or fragment blocks binding of the reference antibody, it is concluded that competition occurred. In some embodiments, competition is concluded, if the fluorescence signal is reduced by at least 25%, at least 50%, or at least 80% compared to control.

"Human CD38" comprises or consists of the amino acid sequence SEQ ID No. 77, or of a sequence having at least 96%, at least 98% or at least 99% sequence identity therewith.

As used herein, the "framework regions" form part of the antibodies and fragments thereof of this disclosure. For example, a single-domain antibody is composed of four framework regions (FRs) and three complementarity-determining regions (CDRs). The framework regions provide structural support and stability to the antibody or fragment thereof, while the CDRs are responsible for antigen binding and specificity. In some cases, the framework regions can participate in antigen binding, contributing to the specificity of the antibodies and fragments thereof. The positions of the CDRs and FRs within the single-domain antibody's sequence may be determined using the IMGT method (https://www.imgt.org/lMGTindex/FR.php, https://www.imgt.org/IMGTindex/CDR.php, February 8, 2024).

As used herein, the term "antibody or fragments thereof", "antibody or fragment thereof", or "antibodies and fragments thereof" comprises full-length antibodies, antibody fragments, multispecific antibodies or fragments thereof, bivalent and multivalent antibodies or fragments thereof, Antibody-Drug Conjugates (ADCs) and fusion proteins.

As used herein, the term "full-length antibodies" comprises immunoglobulin (Ig) classes and engineered IgG variants. As used herein, the term "immunoglobulin classes" comprises immunoglobulin isotypes such as IgG, IgA, IgM, IgD and IgE. These classes can be further divided into subclasses such as IgG1, IgG2, IgG3, IgG4, IgA1, IgA2, IgM, IgD and IgE. As used herein, the term "engineered IgG variants" comprises Fc (Fragment crystallizable)-engineered IgG, single-armed or monovalent IgG, pH-dependent antibodies, Fc-silenced IgGs and IgG fusion proteins.

As used herein, the term "antibody fragments" comprises monovalent fragments, bivalent and multivalent fragments, and inverted IgG fragments.

Preferably, as used herein, the term "antibody or fragment thereof" refers to monovalent or multivalent single-domain antibodies (sdAbs) and/or to monovalent or multivalent minibodies. Most preferably, as used herein, the term "antibody fragments" or "antibodies and fragments thereof" refers to single-domain antibodies (sdAbs) and/or to minibodies.

As used herein, the term "monovalent fragments" comprises Fab (Fragment antigen-binding), Fab' (Fab prime), Fv (Fragment variable), scFv (single-chain variable fragment) and sdAb (single-domain antibody). As used herein, the term "bivalent and multivalent fragments" may comprise one or more of F(ab')2, diabody, tandem scFv, BiTE (bispecific T-cell Engager), tandem diabody (TandAb), triabody, antibody tetramer, pentabody, bivalent minibody, trivalent minibody, tetravalent minibody, pentavalent minibody, hexavalent minibody, bivalent single-domain antibody, trivalent single-domain antibody, tetravalent single-domain antibody, pentavalent single-domain antibody, hexavalent single-domain antibody, bivalent Chimeric Antigen Receptor (CAR) for use in CAR T-cell therapy or trivalent Chimeric Antigen Receptor (CAR) for use in CAR T-cell therapy. Preferably, as used herein, the term "bivalent and multivalent fragments" refers to bivalent minibodies, trivalent minibodies, tetravalent minibodies, bivalent single-domain antibodies, trivalent single-domain antibodies or tetravalent single-domain antibodies.

As used herein, the term "bivalent single-domain antibody" refers to two single-domain antibodies linked together, optionally through a linker, such as a linker peptide or a flexible protein sequence, the construct possessing two binding domains.

As used herein, the term "trivalent single-domain antibody" refers to three single-domain antibodies linked together, optionally through a linker, such as a linker peptide or a flexible protein sequence, the construct possessing three binding domains.

As used herein, the term "tetravalent single-domain antibody" refers to four single-domain antibodies linked together, optionally through a linker, such as a linker peptide or a flexible protein sequence, the construct possessing four binding domains.

As used herein, the term "inverted IgG fragments" refer to antibody fragments resembling the overall shape of an IgG, but having an altered domain composition or connectivity, arising from controlled fragmentation of IgM antibodies or engineered re-assembly of immunoglobulin pieces.

As used herein, the term "multispecific antibodies or fragments thereof" comprises bispecific antibodies or antibody fragments, trispecific antibodies or antibody fragments, tetraspecific antibodies or antibody fragments and antibodies or antibody fragments with higher-order specificity.

As used herein, the term "bispecific antibodies or antibody fragments" may describe antibody formats or antibody fragment formats able to bind to two distinct targets. The two distinct targets may be two distinct epitopes or antigens on the same target molecule or on distinct target molecules.

As used herein, the term "bispecific antibodies or antibody fragments" may comprise bispecific IgG (heterodimeric IgG), bispecific full-length antibodies, BiTE (Bispecific T-cell Engager), bispecific diabody, DART (Dual Affinity ReTargeting), TandAb, DVD-Ig (Dual-Variable Domain Ig), Dual Action Fab, bispecific minibodies, bispecific single-domain antibodies, Quadroma, orthogonal Fab, bispecific Chimeric Antigen Receptors (CARs) for use in CAR T-cell therapy, half-antibodies or variations thereof.

As used herein, the term "trispecific antibodies or antibody fragments" may describe antibody formats or antibody fragment formats able to bind to three distinct targets. Of the three distinct targets, two or more may be distinct epitopes or antigens on the same target molecule or on distinct target molecules.

As used herein, the term "trispecific antibodies" may comprise trispecific IgG, trispecific full-length antibodies, TriKE (Trispecific Killer Engager), trispecific minibodies, trispecific single-domain antibodies or trispecific Chimeric Antigen Receptors (CARs) for use in CAR T-cell therapy.

As used herein, the term "tetraspecific antibodies and antibodies with higher-order specificity" may describe antibody formats or antibody fragment formats able to bind to four or more distinct targets. Of the four or more distinct targets, two or more may be distinct epitopes or antigens on the same target molecule or on distinct target molecules.

As used herein, the term "multivalent antibodies or fragments thereof" comprises bivalent antibodies or antibody fragments, trivalent antibodies or antibody fragments, tetravalent antibodies or antibody fragments, pentavalent antibodies or antibody fragments, hexavalent antibodies or antibody fragments, or antibody or antibody fragments of higher multivalency.

As used herein, the term "minibody" or "minibodies" refers to small multivalent antibody fragments that include at least one engineered oligomerization domain. The used oligomerization domain influences the valency of the antibody fragment. A classic example would be an scFv fused to the CH3 domain of an IgG1. As CH3 drives dimerization, the resulting antibody fragment is a bivalent dimer. If a trimerization domain is used instead (e.g. human collagen XIII domain or bacteriophage foldon domain), three scFvs may assemble into a trivalent trimer (sometimes termed a tribody). If a tetramerization domain (e.g. the p53 tetramerization domain or the streptavidin core) is used, four scFvs assemble into a tetravalent tetramer (sometimes termed a tetrabody). Tetravalent minibodies may also be formed by the dimerization of two bivalent scFv-Fc fusion antibody fragments. Minibodies are typically smaller than IgG but achieve multivalency. Another example of a minibody is a bivalent antibody fragment consisting of two single-domain antibodies (sdAbs) fused to the Fc (Fragment crystallizable) domain of an antibody, able to bind antigens bivalently. Another example of a minibody is a trivalent antibody fragment consisting of three sdAbs fused to a trimerization domain. Another example of a minibody is a tetravalent antibody fragment consisting of four sdAbs fused to a tetramerization domain. Another example of a minibody are two camelid sdAbs fused to human hinge and immunoglobulin Fc domain, resulting in functional homodimers, also called human recombinant heavy chain antibody (hrHcAb).

In preferred embodiments, the term "human recombinant heavy chain antibody" refers to an antibody-construct comprising one or more single-domain antibodies fused to an Fc region of an immunoglobulin isotype heavy chain, such as human IgG, optionally human IgG1.

In embodiments, the minibody consists of two single-domain antibodies fused to the Fc domain of an antibody selected from the immunoglobulin classes IgG, IgA, IgM, IgD and IgE.

In preferred embodiments, one "minibody" refers to two camelid single-domain antibodies fused to human hinge and immunoglobulin Fc domains, resulting in functional homodimers.

In preferred embodiments, one "minibody" refers to two camelid single-domain antibodies fused to human IgG1 hinge and immunoglobulin Fc domains, resulting in functional homodimers.

In some embodiments, the minibody refers to two camelid sdAbs fused to immunoglobulin hinge and Fc domains, resulting in functional heterodimers.

In preferred embodiments, one "minibody" is a human recombinant heavy chain antibody (hrHcAb).

Minibodies show improved tumor uptake, both in therapy and in imaging applications, due to their intermediate size. Minibodies are sufficiently small to enable efficient penetration and sufficiently large to demonstrate slower renal clearance than scFv. Multispecific minibodies can be made by including different scFv or sdAb specificities on a multimerizing scaffold. The minibody format greatly increases the avidity of the antibody construct.

In embodiments, the antibodies or fragments thereof according to the present disclosure may be multivalent and multispecific.

In embodiments, the antibodies or fragments thereof may display a combination of their multivalency and their multispecificity. For example, in embodiments, bivalent antibodies or fragments thereof may be monospecific or bispecific. For example, in embodiments, trivalent antibodies or fragments thereof may be monospecific, bispecific or trispecific. For example, in embodiments, tetravalent antibodies may be monospecific, bispecific, trispecific or tetraspecific.

As used herein, the term "Antibody-Drug Conjugates" (ADCs) refers to antibodies or antibody fragments chemically conjugated to cytotoxic drugs (also referred to as "payload"). Examples of drugs include but are not limited to: microtubule inhibitors (e.g. auristatins like MMAE, MMAF; maytansinoids like DM1, DM4), DNA-damaging agents (e.g. calicheamicins, pyrrolobenzodiazepine dimers, duocarmycins), topoisomerase I inhibitors (e.g. SN-38), RNA polymerase II inhibitors (e.g, amanitin) and immune stimulators.

Microtubule inhibitors disrupt cell division. DNA-damaging agents such as calicheamicin induce DNA double-strand breaks. DNA-damaging agents such as pyrrolobenzodiazepine dimers and duocarmycins alkylate/crosslink DNA.

The payload in ADCs must be able to kill or impair the function of the target cell once released. Example mechanisms include but are not limited to causing apoptosis via DNA destruction, causing apoptosis via mitotic apparatus destruction or causing cell arrest.

As used herein, the term "fusion proteins" refers to antibodies or antibody fragments fused to functional proteins. Examples include but are not limited to: immunocytokines, antibody-enzyme fusion proteins, antibody-drug-metabolizing enzymes and immunotoxins.

As used herein, the term "nanobody" refers to a single-domain antibody (sdAb) consisting of a single monomeric variable domain with high stability and small size, able to bind to a specific antigen.

As used herein, the term "nanobody" and "single-domain antibody" are used synonymously.

As used herein, the term "antibodies or fragments thereof" may relate to a nanobody.

The nanobody according to the present disclosure may relate to a monomeric, dimeric, trimeric, tetrameric, pentameric, hexameric or multimeric antibody fragment.

In embodiments, the antibodies or fragments thereof may be incorporated in CAR-T cell therapy, adopting the form of chimeric antigen receptors (CARs). In embodiments, the antibodies or fragments thereof may be humanized - as is known in the art -, converting the FRs to human FRs.

In preferred embodiments, the antibody or fragment thereof according to the present disclosure is recombinant.

In preferred embodiments, the antibody or fragment thereof according to the present disclosure is humanized

The affinity of the antibodies or fragments thereof according to the present disclosure to specific targets can be measured by methods known in the art. In this disclosure, the affinity (dissociation constant K_{D}) of a single-domain antibody may be determined using surface plasmon resonance (at 25°C, e.g. Biacore), or mean fluorescence intensity (MFI) methods, such as the method described in **Example 5.** As used herein, an affinity is indicated by reference to the dissociation constant, meaning that a lower value represents higher affinity.

A "conservative amino acid substitution" or "conservative replacement" is one in which an amino acid residue is substituted by another amino acid residue having a side chain (R group) with similar chemical properties (e.g., same charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art. Examples of groups of amino acids that have side chains with similar chemical properties, and whose substitution for each other constitutes conservative substitutions, include 1) aliphatic side chains: glycine, alanine, valine, leucine and isoleucine; 2) aliphatic hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartate and glutamate, and 7) sulfur-containing side chains: cysteine and methionine. Particular conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256: 1443 45. In an embodiment, all charged amino acids are considered similar.

In an embodiment, a "conservative" replacement is any change having a value in the PAM250 log-likelihood matrix differing by not more than 50% of the "no-replacement" value. A "moderately conservative" replacement is any change having a nonnegative value in the PAM250 log-likelihood matrix. A "strictly conservative" replacement is any change having a value in the PAM250 log-likelihood matrix differing by maximum of 1.

An "empirical amino acid substitution" or "empirical replacement" is one which does not substantially alter the relevant properties of the antibody or fragment thereof, particularly its affinity to human CD38.

"Sequence similarity" or "sequence identity" for polypeptides is typically measured using sequence analysis software. Protein analysis software matches similar sequences using measures of similarity assigned to various substitutions, deletions and other modifications, including conservative amino acid substitutions. For instance, GCG software contains programs such as GAP and BESTFIT which can be used with default parameters to determine sequence homology or sequence identity between closely related polypeptides, such as homologous polypeptides from different species of organisms or between a wild type protein and a mutein thereof. Polypeptide sequences also can be compared using FASTA with default or recommended parameters; a program in GCG Version 6.1. FASTA (e.g., FASTA2 and FASTA3) provides alignments and percent sequence identity of the regions of the best overlap between the query and search sequences (Pearson (2000) supra). Another preferred algorithm when comparing a sequence of this disclosure to a database containing a large number of sequences from different organisms is the computer program BLAST, especially BLASTP or TBLASTN, using default parameters. See, e.g., Altschul et al. (1990) J. Mol. Biol. 215: 403 410 and (1997) Nucleic Acids Res. 25:3389 402. Similarly, when referring to a nucleic acid sequence identity or similarity can be determined - as is known in the art - by any well-known algorithm, such as FASTA, BLAST or GAP, as discussed.

In the context of binding to CD38, the antigen binding "specificity" refers to the ability of the antibodies or fragments thereof according to the present disclosure to specifically bind the CD38 protein and/or its homologs in various species, excluding the binding to other types of proteins different from CD38. In particular embodiments of the present disclosure, the antibodies and fragments thereof are able to bind to both human CD38 and rodent CD38.

### Brief Description of the Figures

- **Figures 1 A - E:**: Immunoprecipitation Using Candidate Nanobodies
- **Figures 2 A - E:**: Validation of Single-Domain Antibodies Binding to Human CD38 in Transfected Cos-7 Cells
- **Figure 2 F:**: Dot-blot Assay to validate binding to hCD38 of other Single-Domain Antibody candidates
- **Figures 3 A** - **E:**: Specificity Checks in Live Cells
- **Figures 4 A** - **E:**: Thermostability Analysis of Single-Domain Antibodies
- **Figure 5:**: Affinity Measurement of Single-Domain Antibody Candidates
- **Figure 6:**: Affinity comparison with Therapeutic Antibodies Binding to Human CD38
- **Figures 7 A** - **F:**: FACS Analysis of Single-Domain Antibody Candidates Used to Stain Primary Immune Cells from the Blood of 2 Different Donors
- **Figure 8:**: Evaluating the Ability of NbC6 to Block or Affect the Binding of Daratumumab or Isatuximab on CD38
- **Figure 9A:**: Measuring Cyclase Activity of Purified CD38 over Time
- **Figure 9B:**: Cyclase Activity of Purified CD38 when control reaches plateau (8 minutes)
- **Figure 9C:**: Cyclase Activity of Purified CD38 after Combinatorial Inhibition
- **Figure 10A:**: Measuring Hydrolase Activity of Purified CD38 over Time
- **Figure 10B:**: Hydrolase Activity of Purified CD38 when control reaches plateau (12 minutes)
- **Figure 10C:**: Hydrolase Activity of Purified CD38 after Combinatorial Inhibition
- **Figure 11:**: Complement-Dependent Cytotoxicity (CDC) Comparison between Minibody C6 and Daratumumab
- **Figure 12A:**: Complement-Dependent Cytotoxicity (CDC) for Combinatorial Treatment at Different Concentrations
- **Figure 12B:**: Complement-Dependent Cytotoxicity (CDC) for Combinatorial Treatment at 0.25 nM
- **Figure 13A:**: Antibody-Dependent Cellular Cytotoxicity (ADCC) Assay Using RAMOS Cells Stably Expressing Yellow Fluorescent Protein (YFP)
- **Figure 13B:**: Statistical Analysis Comparing Antibody-Dependent Cellular Cytotoxicity Efficacy of Different Treatments at 10 pM
- **Figures 14 A** - **B:**: Live-Cell Staining of Transfected Cos-7 Cells Expressing Native or Glycomutant hCD38
- **Figure 15A:**: Evaluating the Ability of Single-Domain Antibodies to Block or Affect the Binding of Isatuximab
- **Figure 15B:**: Evaluating the Ability of Single-Domain Antibodies to Block or Affect the Binding of Daratumumab

### Detailed Description of the Disclosure

The antibodies and fragments thereof of this disclosure specifically bind to human CD38. The antibodies and fragments thereof of the present disclosure bind to human CD38 with high specificity. **Example 3** illustrates the specific binding of single-domain antibodies according to the present disclosure to human B-cells, human T-cells and not mouse epithelial cells, human fibroblast cells or monkey fibroblast cells.

In an embodiment, the antibody or fragment thereof is a more potent inhibitor of the cyclase enzymatic function of CD38 than daratumumab. In an embodiment, the antibody or fragment thereof is more efficient than daratumumab in inducing CDC in cancer cells.

In an embodiment, the antibody or fragment thereof is a more potent inhibitor of the cyclase function of CD38 than daratumumab.

In an embodiment, the antibody or fragment thereof is a potent inhibitor of the cyclase function of CD38, comparable in strength to daratumumab.

In an embodiment, when used in combination with isatuximab, the antibody or fragment thereof is a more potent inhibitor of the hydrolase function of CD38 than daratumumab and isatuximab.

In an embodiment, the antibody or fragment thereof is a potent inhibitor of the hydrolase function of CD38, comparable in strength to daratumumab.

In an embodiment, the antibody or fragment thereof is more efficient than daratumumab in inducing complement-dependent cytotoxicity (CDC) in CD38-expressing cells and/or CD38-expressing cancer cells.

In an embodiment, the antibody or fragment thereof is comparable in efficiency to daratumumab in inducing complement-dependent cytotoxicity (CDC) in CD38-expressing cells and/or CD38-expressing cancer cells.

In an embodiment, the antibody or fragment thereof is more efficient than daratumumab in inducing antibody-dependent cellular cytotoxicity (ADCC) in CD38-expressing cells and/or CD38-expressing cancer cells. In an embodiment, the antibody or fragment thereof is more efficient than isatuximab in inducing antibody-dependent cellular cytotoxicity (ADCC) in CD38-expressing cells and/or CD38-expressing cancer cells. In an embodiment, the antibody or fragment thereof is more efficient than daratumumab and/or in inducing antibody-dependent cellular cytotoxicity (ADCC) in CD38-expressing cells and/or CD38-expressing cancer cells.

In embodiments, the combination of the antibody or fragment thereof and isatuximab demonstrates a strong synergistic effect in inhibiting the cyclase function of CD38, surpassing the sum of the individual effects in magnitude.

In embodiments, the combination of the antibody or fragment thereof and isatuximab demonstrates a synergistic effect in inhibiting the hydrolase function of CD38, surpassing the sum of the individual effects in magnitude.

In embodiments, the combination of the antibody or fragment thereof and isatuximab demonstrates a synergistic effect in inducing complement-dependent cytotoxicity (CDC) in living cells, surpassing the sum of the individual effects in magnitude.

In embodiments, the combination of the antibody or fragment thereof and isatuximab demonstrates a synergistic effect in inducing antibody-dependent cellular cytotoxicity (ADCC) in living cells, surpassing the sum of the individual effects in magnitude.

In an embodiment, the antibody or fragment thereof is independently chosen from the list consisting of: a monovalent single-domain antibody, a multivalent single-domain antibody, a monovalent human recombinant heavy chain antibody, and a multivalent human recombinant heavy chain antibody.

In an embodiment, the antibody or fragment thereof is independently chosen from the list consisting of: IgG1, IgG2, IgG3, IgG4, IgA1, IgA2, IgM, IgD, IgE, Fc-engineered IgG, single-armed or monovalent IgG, pH-dependent antibodies or fragments, Fc-silenced IgGs, IgG fusion proteins, monovalent single-domain antibodies, multivalent single-domain antibodies, sdAb (single-domain antibodies), Fab (Fragment antigen-binding), Fab' (Fab prime), Fv (Fragment variable), scFv (single-chain variable fragment), F(ab')2, diabodies, tandem scFv, BiTE (bispecific T-cell Engager), TandAb (tandem diabody), triabody, antibody tetramer, pentabody, bivalent single-domain antibody, trivalent single-domain antibody, tetravalent single-domain antibody, pentavalent single-domain antibody, hexavalent single-domain antibody, hrHcAb (human recombinant heavy chain antibody), monovalent hrHcAb, multivalent hrHcAb, bivalent hrHcAb, trivalent hrHcAb, tetravalent hrHcAb, pentavalent hrHcAb, hexavalent hrHcAb, bispecific hrHcAb, trispecific hrHcAb, tetraspecific hrHcAb, monovalent minibodies, multivalent minibodies, bivalent minibodies, trivalent minibodies, tetravalent minibodies, pentavalent minibodies, hexavalent minibodies, bispecific minibodies, trispecific minibodies, tetraspecific minibodies, bivalent Chimeric Antigen Receptor (CAR), trivalent Chimeric Antigen Receptor (CAR), inverted IgG fragments, bispecific IgG (heterodimeric IgG), bispecific IgG1, bispecific IgG2, bispecific IgG3, bispecific IgG4, bispecific IgA1, bispecific IgA2, bispecific IgM, bispecific IgD, bispecific IgE, bispecific Fc-engineered IgG, bispecific pH-dependent antibodies or fragments, bispecific Fc-silenced IgG, bispecific IgG fusion proteins, bispecific diabodies, DART (Dual Affinity ReTargeting), DVD-Ig (Dual-Variable Domain Ig), Dual Action Fab, bispecific single-domain antibodies, Quadroma, orthogonal Fab, bispecific Chimeric Antigen Receptor (CAR), half-antibodies, trispecific IgG1, trispecific IgG2, trispecific IgG3, trispecific IgG4, trispecific IgA1, trispecific IgA2, trispecific IgM, trispecific IgD, trispecific IgE, TriKE (Trispecific Killer Engager), trispecific single-domain antibodies, trispecific Chimeric Antigen Receptor (CAR), trispecific Fc-engineered IgG, trispecific pH-dependent antibodies or fragments, trispecific Fc-silenced IgG, trispecific IgG fusion proteins, tetraspecific single-domain antibody, antibody-drug conjugates carrying MMAF, antibody-drug conjugates carrying MMAE, antibody-drug conjugates carrying DM1, antibody-drug conjugates carrying DM4, antibody-drug conjugates carrying pyrrolobenzodiazepine dimers, antibody-drug conjugates carrying duocarmycins, antibody-drug conjugates carrying SN-38, antibody-drug conjugates carrying amanitin, antibody-drug conjugates carrying immune stimulators, antibody-drug conjugates carrying microtubule inhibitors, antibody-drug conjugates carrying auristatins, antibody-drug conjugates carrying maytansinoids, antibody-drug conjugates carrying topoisomerase I inhibitors, antibody-drug conjugates carrying RNA polymerase II inhibitors, immunocytokines fusion proteins, antibody-enzyme fusion proteins, antibody-drug-metabolizing enzymes fusion proteins, immunotoxin fusion proteins, monomeric single-domain antibody, dimeric single-domain antibody, trimeric single-domain antibody, tetrameric single-domain antibody, pentameric single-domain antibody, hexameric single-domain antibody, multimeric single-domain antibody, bivalent scFv and CH3 domain of IgG fusion, bispecific scFv and CH3 domain of IgG fusion, bivalent scFv Fc domain fusion, bispecific scFv Fc domain fusion, trivalent scFv and trimerization domain fusion, trispecific scFv and trimerization domain fusion, tetravalent scFv and tetramerization domain fusion, tetraspecific scFv and tetramerization domain fusion, bivalent sdAb and CH3 domain of IgG fusion, bispecific sdAb and CH3 domain of IgG fusion, bivalent sdAb Fc domain fusion, bispecific sdAb Fc domain fusion, trivalent sdAb and trimerization domain fusion, trispecific sdAb and trimerization domain fusion, tetravalent sdAb and tetramerization domain fusion, tetraspecific sdAb and tetramerization domain fusion and/or variations thereof and/or recombinant variations thereof and/or humanized variations thereof;

Optionally, the antibody or fragment thereof is humanized, recombinant and/or isolated.

For example, the CD38 is human CD38 and/or comprises SEQ ID No. 77, or a sequence of at least 95%, 97% or at least 98% sequence identity therewith.

In an embodiment, the antibody or fragment thereof is independently chosen from the list consisting of: monovalent single-domain antibody, multivalent single-domain antibody, bivalent single-domain antibody, trivalent single-domain antibody, tetravalent single-domain antibody, pentavalent single-domain antibody, hexavalent single-domain antibody, hrHcAb (human recombinant heavy chain antibody), monovalent hrHcAb, multivalent hrHcAb, bivalent hrHcAb, trivalent hrHcAb, tetravalent hrHcAb, pentavalent hrHcAb, hexavalent hrHcAb, bispecific hrHcAb, trispecific hrHcAb, tetraspecific hrHcAb, monovalent minibodies, multivalent minibodies, bivalent minibodies, trivalent minibodies, tetravalent minibodies, pentavalent minibodies, hexavalent minibodies, bispecific minibodies, trispecific minibodies, tetraspecific minibodies, bispecific single-domain antibodies, trispecific single-domain antibodies, tetraspecific single-domain antibody, dimeric single-domain antibody, trimeric single-domain antibody, tetrameric single-domain antibody, pentameric single-domain antibody, BiTE, hexameric single-domain antibody, multimeric single-domain antibody, bivalent sdAb and CH3 domain of IgG fusion, bispecific sdAb and CH3 domain of IgG fusion, bivalent sdAb Fc domain fusion, bispecific sdAb Fc domain fusion, trivalent sdAb and trimerization domain fusion, trispecific sdAb and trimerization domain fusion, tetravalent sdAb and tetramerization domain fusion, tetraspecific sdAb and tetramerization domain fusion and/or variations thereof and/or recombinant variations thereof and/or humanized variations thereof;

Optionally, the antibody or fragment thereof is humanized, recombinant and/or isolated.

For example, the CD38 is human CD38 and/or comprises SEQ ID No. 77, or a sequence of at least 95%, 97% or at least 98% sequence identity therewith.

In an embodiment, the antibody or fragment thereof is independently chosen from the list consisting of: multivalent single-domain antibody, bivalent single-domain antibody, trivalent single-domain antibody, tetravalent single-domain antibody, monovalent hrHcAb, multivalent hrHcAb, bivalent hrHcAb, trivalent hrHcAb, tetravalent hrHcAb,bispecific hrHcAb, trispecific hrHcAb, tetraspecific hrHcAb, bispecific single-domain antibodies, trispecific single-domain antibodies, tetraspecific single-domain antibody, dimeric single-domain antibody, BiTE, trimeric single-domain antibody, tetrameric single-domain antibody, multimeric single-domain antibody, bivalent sdAb and CH3 domain of IgG fusion, bispecific sdAb and CH3 domain of IgG fusion, bivalent sdAb Fc domain fusion, bispecific sdAb Fc domain fusion, trivalent sdAb and trimerization domain fusion, trispecific sdAb and trimerization domain fusion, tetravalent sdAb and tetramerization domain fusion, tetraspecific sdAb and tetramerization domain fusion and/or variations thereof and/or recombinant variations thereof and/or humanized variations thereof;

Optionally, the antibody or fragment thereof is humanized, recombinant and/or isolated.

For example, the CD38 is human CD38 and/or comprises SEQ ID No. 77, or a sequence of at least 95%, 97% or at least 98% sequence identity therewith.

In an embodiment, the antibody or fragment thereof is independently chosen from the list consisting of: multivalent single-domain antibody, bivalent single-domain antibody, trivalent single-domain antibody, tetravalent single-domain antibody, monovalent hrHcAb, multivalent hrHcAb, bivalent hrHcAb, trivalent hrHcAb, tetravalent hrHcAb,bispecific hrHcAb, trispecific hrHcAb, tetraspecific hrHcAb, bispecific single-domain antibodies, trispecific single-domain antibodies, tetraspecific single-domain antibody, dimeric single-domain antibody, BiTE, Chimeric Antigen Receptor, multimeric Chimeric Antigen Receptor, multivalent Chimeric Antigen Receptor, multispecific Chimeric Antigen Receptor, trimeric single-domain antibody, tetrameric single-domain antibody, multimeric single-domain antibody, bivalent sdAb and CH3 domain of IgG fusion, bispecific sdAb and CH3 domain of IgG fusion, bivalent sdAb Fc domain fusion, bispecific sdAb Fc domain fusion, trivalent sdAb and trimerization domain fusion, trispecific sdAb and trimerization domain fusion, tetravalent sdAb and tetramerization domain fusion, tetraspecific sdAb and tetramerization domain fusion and/or variations thereof and/or recombinant variations thereof and/or humanized variations thereof;

Optionally, the antibody or fragment thereof is humanized, recombinant and/or isolated.

For example, the CD38 is human CD38 and/or comprises SEQ ID No. 77, or a sequence of at least 95%, 97% or at least 98% sequence identity therewith.

In some preferred embodiments, the antibody or fragment thereof is a single-domain antibody or a minibody. In some preferred embodiments, the antibody or fragment thereof is a single-domain antibody or a hrHcAb. In some preferred embodiments, the antibody or fragment thereof is a single-domain antibody. In some preferred embodiments, the antibody or fragment thereof is a minibody. In some preferred embodiments, the antibody or fragment thereof is a hrHcAb.

In some preferred embodiments, the minibody consists of camelid single-domain antibodies fused to human hinge and immunoglobulin Fc domains, resulting in functional homodimers. In some preferred embodiments, the minibody consists of two single-domain antibodies fused to an immunoglobulin hinge and Fc domains, e.g. human IgG Fc domain. In some preferred embodiments, the antibody or fragment thereof consists of two single-domain antibodies fused to an Fc domain, e.g human IgG Fc. In some preferred embodiments, the minibody consists of two single-domain antibodies fused to an Fc domain, e.g. human IgG Fc, preferably human IgG1 Fc. In some preferred embodiments, the minibody consists of two single-domain antibodies fused to a complete Fc domain, e.g. complete human IgG Fc. In some embodiments, the minibody may consist of two-single domain antibodies fused to an incomplete Fc domain, e.g. an incomplete human IgG Fc. In some preferred embodiments, the minibody is a human recombinant heavy chain antibody (hrHcAb).

In preferred embodiments, the term "human recombinant heavy chain antibody" refers to an antibody-construct comprising one or more single-domain antibodies fused to an Fc region of an immunoglobulin isotype heavy chain, such as human IgG, optionally human IgG1.

In some embodiments, the antibody or fragment thereof is bispecific. In some embodiments, the antibody or fragment thereof is a BiTE. In some embodiments, the antibody or fragment thereof is a bispecific single-domain antibody.

In some embodiments, the antibody or fragment thereof is a bispecific single-domain antibody able to bind to CD38 and to another epitope or molecular target.

In some embodiments, the antibody or fragment thereof is a trimeric single-domain antibody. In some embodiments, the antibody or fragment thereof is a bispecific minibody.

In some embodiments, one or more of the following is true for the antibody or fragment thereof of this disclosure
(i) the antibody or fragment thereof competes with daratumumab for binding to CD38, in particular to human CD38,
(ii) the antibody or fragment thereof does not compete with isatuximab for binding to CD38, in particular to human CD38,
(iii) the size of the antibody or fragment thereof ranges from 10.0 to 18.0 kDa, or from 70.0 kDa to 100.0 kDa, or from 130.0 kDa to 170.0 kDa,
(i) the antibody or fragment thereof is stable at temperatures of up to 65 to 70, preferred 70 to 75, most preferred 75 °C or higher, and/or
(ii) the antibody or fragment thereof is conjugated to at least one effector molecule, optionally selected from the group consisting of: a detectable label, a radionuclide, a cytotoxic drug or combinations thereof.

Optionally, the antibody or fragment thereof binds to human CD38 with an affinity of at most 1 µM, or at most 500 nM, or at most 250 nM, or at most 1 nM, or at most 100 pM, preferably at most 75 pM, most preferred or at most 50 pM.

In an embodiment, the antibody or fragment thereof according to this disclosure binds to CD38 with an affinity of at most 1 nM, or at most 100 pM. For example, the antibody or fragment thereof may bind to human CD38 with an affinity of at most 1 nM, at most 200 pM, at most 100 pM, at most 70 pM, or at most 50 pM. In some embodiments, the antibody or fragment thereof may bind to CD38 with an affinity of at least 1 pM, at least 10 pM, at least 30 pM, or at least 40 pM. For example, the antibody or fragment thereof may bind to CD38 with an affinity of from 1 pM to 1 nM, from 10 pM to 200 pM, from 30 pM to 100 pM, or from 40 pM to 70 pM. It was found that this affinity is useful for therapeutic, diagnostic and surgical applications as the strong affinity provides for ideal targeting capabilities towards CD38 positive cells.

In some embodiments, the antibodies or fragments thereof according to this disclosure bind to CD38 with an affinity of at most 1 µM, or at most 900 nM, or at most 500 nM, or at most 300 nM, or at most 250 nM, or at most 1nM, or at most 200 pM, or at most 100 pM, or at most 75 pM, or at most 50 pM. In some embodiments, the antibodies or fragments thereof may bind to CD38 with an affinity ranging from 1 pM to 1 µM, or from 500 pM to 900 nM, or from 100 nM to 800 nM. In some embodiments, the antibodies or fragments thereof may bind to CD38 with an affinity ranging from 1 nM to 800 nM, or from 200 nM to 700 nM, or from 300 nM to 600 nM. In some embodiments, the antibodies or fragments thereof may bind to CD38 with an affinity ranging from 200 pM to 300 nM. In some embodiments, the antibodies or fragments thereof according to the present disclosure bind to CD38 with an affinity of at most 1 µM.

In some embodiments, the antibodies or fragments thereof according to the present disclosure compete with daratumumab but not with isatuximab for binding to CD38. Example 8 and Example **15** demonstrate this. Advantageously, this allows combination therapies comprising isatuximab and antibodies or fragments thereof according to the present disclosure.

Advantageously, the antibody or fragment thereof binds to at least one epitope of CD38, such as human CD38.

In an embodiment, the antibody or fragment comprises
(i) complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8, optionally having framework regions, FR1, FR2, FR3 and FR4, wherein FR1 has a sequence identity of at least 90% with SEQ ID No. 3, FR2 has a sequence identity of at least 90% with SEQ ID No. 5, FR3 has a sequence identity of at least 90% with SEQ ID No. 7, and/or FR4 has a sequence identity of at least 90% with SEQ ID No. 9,
(ii) complementarity-determining regions CDR1, CDR2 and CDR3, wherein CDR1 has an amino acid sequence SEQ ID No. 13, CDR2 has an amino acid sequence SEQ ID No. 15, and CDR3 has an amino acid sequence SEQ ID No. 17, optionally having framework regions, FR1, FR2, FR3 and FR4, wherein FR1 has a sequence identity of at least 90% with SEQ ID No. 12, FR2 has a sequence identity of at least 90% with SEQ ID No. 14, FR3 has a sequence identity of at least 90% with SEQ ID No. 16, and/or FR4 has a sequence identity of at least 90% with SEQ ID No. 18,
(iii) complementarity-determining regions CDR1, CDR2 and CDR3, wherein CDR1 has an amino acid sequence SEQ ID No. 22, CDR2 has an amino acid sequence SEQ ID No. 24, and CDR3 has an amino acid sequence SEQ ID No. 26, optionally having framework regions, FR1, FR2, FR3 and FR4, wherein FR1 has a sequence identity of at least 90% with SEQ ID No. 21, FR2 has a sequence identity of at least 90% with SEQ ID No. 23, FR3 has a sequence identity of at least 90% with SEQ ID No. 25, and/or FR4 has a sequence identity of at least 90% with SEQ ID No. 27,
(iv) complementarity-determining regions CDR1, CDR2 and CDR3, wherein CDR1 has an amino acid sequence SEQ ID No. 31, CDR2 has an amino acid sequence SEQ ID No. 33, and CDR3 has an amino acid sequence SEQ ID No. 35, optionally having framework regions, FR1, FR2, FR3 and FR4, wherein FR1 has a sequence identity of at least 90% with SEQ ID No. 30, FR2 has a sequence identity of at least 90% with SEQ ID No. 32, FR3 has a sequence identity of at least 90% with SEQ ID No. 34, and/or FR4 has a sequence identity of at least 90% with SEQ ID No. 36,
(v) complementarity-determining regions CDR1, CDR2 and CDR3, wherein CDR1 has an amino acid sequence SEQ ID No. 40, CDR2 has an amino acid sequence SEQ ID No. 42, and CDR3 has an amino acid sequence SEQ ID No. 44, optionally having framework regions, FR1, FR2, FR3 and FR4, wherein FR1 has a sequence identity of at least 90% with SEQ ID No. 39, FR2 has a sequence identity of at least 90% with SEQ ID No. 41, FR3 has a sequence identity of at least 90% with SEQ ID No. 43, and/or FR4 has a sequence identity of at least 90% with SEQ ID No. 45,
(vi) complementarity-determining regions CDR1, CDR2 and CDR3, wherein CDR1 has an amino acid sequence SEQ ID No. 51, CDR2 has an amino acid sequence SEQ ID No. 53, and CDR3 has an amino acid sequence SEQ ID No. 55, optionally having framework regions, FR1, FR2, FR3 and FR4, wherein FR1 has a sequence identity of at least 90% with SEQ ID No. 50, FR2 has a sequence identity of at least 90% with SEQ ID No. 52, FR3 has a sequence identity of at least 90% with SEQ ID No. 54, and/or FR4 has a sequence identity of at least 90% with SEQ ID No. 56,
(vii) complementarity-determining regions CDR1, CDR2 and CDR3, wherein CDR1 has an amino acid sequence SEQ ID No. 60, CDR2 has an amino acid sequence SEQ ID No. 62, and CDR3 has an amino acid sequence SEQ ID No. 64, optionally having framework regions, FR1, FR2, FR3 and FR4, wherein FR1 has a sequence identity of at least 90% with SEQ ID No. 59, FR2 has a sequence identity of at least 90% with SEQ ID No. 61, FR3 has a sequence identity of at least 90% with SEQ ID No. 63, and/or FR4 has a sequence identity of at least 90% with SEQ ID No. 65, or
(viii) complementarity-determining regions CDR1, CDR2 and CDR3, wherein CDR1 has an amino acid sequence SEQ ID No. 71, CDR2 has an amino acid sequence SEQ ID No. 73, and CDR3 has an amino acid sequence SEQ ID No. 75, optionally having framework regions, FR1, FR2, FR3 and FR4, wherein FR1 has a sequence identity of at least 90% with SEQ ID No. 70, FR2 has a sequence identity of at least 90% with SEQ ID No. 72, FR3 has a sequence identity of at least 90% with SEQ ID No. 74, and/or FR4 has a sequence identity of at least 90% with SEQ ID No. 76.

In an embodiment, the antibody or fragment comprises
(i) complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8, optionally having framework regions, FR1, FR2, FR3 and FR4, wherein FR1 has a sequence identity of at least 95% with SEQ ID No. 3, FR2 has a sequence identity of at least 95% with SEQ ID No. 5, FR3 has a sequence identity of at least 95% with SEQ ID No. 7, and/or FR4 has a sequence identity of at least 95% with SEQ ID No. 9,
(ii) complementarity-determining regions CDR1, CDR2 and CDR3, wherein CDR1 has an amino acid sequence SEQ ID No. 13, CDR2 has an amino acid sequence SEQ ID No. 15, and CDR3 has an amino acid sequence SEQ ID No. 17, optionally having framework regions, FR1, FR2, FR3 and FR4, wherein FR1 has a sequence identity of at least 95% with SEQ ID No. 12, FR2 has a sequence identity of at least 95% with SEQ ID No. 14, FR3 has a sequence identity of at least 95% with SEQ ID No. 16, and/or FR4 has a sequence identity of at least 95% with SEQ ID No. 18,
(iii) complementarity-determining regions CDR1, CDR2 and CDR3, wherein CDR1 has an amino acid sequence SEQ ID No. 22, CDR2 has an amino acid sequence SEQ ID No. 24, and CDR3 has an amino acid sequence SEQ ID No. 26, optionally having framework regions, FR1, FR2, FR3 and FR4, wherein FR1 has a sequence identity of at least 95% with SEQ ID No. 21, FR2 has a sequence identity of at least 95% with SEQ ID No. 23, FR3 has a sequence identity of at least 95% with SEQ ID No. 25, and/or FR4 has a sequence identity of at least 95% with SEQ ID No. 27,
(iv) complementarity-determining regions CDR1, CDR2 and CDR3, wherein CDR1 has an amino acid sequence SEQ ID No. 31, CDR2 has an amino acid sequence SEQ ID No. 33, and CDR3 has an amino acid sequence SEQ ID No. 35, optionally having framework regions, FR1, FR2, FR3 and FR4, wherein FR1 has a sequence identity of at least 95% with SEQ ID No. 30, FR2 has a sequence identity of at least 95% with SEQ ID No. 32, FR3 has a sequence identity of at least 95% with SEQ ID No. 34, and/or FR4 has a sequence identity of at least 95% with SEQ ID No. 36,
(v) complementarity-determining regions CDR1, CDR2 and CDR3, wherein CDR1 has an amino acid sequence SEQ ID No. 40, CDR2 has an amino acid sequence SEQ ID No. 42, and CDR3 has an amino acid sequence SEQ ID No. 44, optionally having framework regions, FR1, FR2, FR3 and FR4, wherein FR1 has a sequence identity of at least 95% with SEQ ID No. 39, FR2 has a sequence identity of at least 95% with SEQ ID No. 41, FR3 has a sequence identity of at least 95% with SEQ ID No. 43, and/or FR4 has a sequence identity of at least 95% with SEQ ID No. 45,
(vi) complementarity-determining regions CDR1, CDR2 and CDR3, wherein CDR1 has an amino acid sequence SEQ ID No. 51, CDR2 has an amino acid sequence SEQ ID No. 53, and CDR3 has an amino acid sequence SEQ ID No. 55, optionally having framework regions, FR1, FR2, FR3 and FR4, wherein FR1 has a sequence identity of at least 95% with SEQ ID No. 50, FR2 has a sequence identity of at least 95% with SEQ ID No. 52, FR3 has a sequence identity of at least 95% with SEQ ID No. 54, and/or FR4 has a sequence identity of at least 95% with SEQ ID No. 56,
(vii) complementarity-determining regions CDR1, CDR2 and CDR3, wherein CDR1 has an amino acid sequence SEQ ID No. 60, CDR2 has an amino acid sequence SEQ ID No. 62, and CDR3 has an amino acid sequence SEQ ID No. 64, optionally having framework regions, FR1, FR2, FR3 and FR4, wherein FR1 has a sequence identity of at least 95% with SEQ ID No. 59, FR2 has a sequence identity of at least 95% with SEQ ID No. 61, FR3 has a sequence identity of at least 95% with SEQ ID No. 63, and/or FR4 has a sequence identity of at least 95% with SEQ ID No. 65, or
(viii) complementarity-determining regions CDR1, CDR2 and CDR3, wherein CDR1 has an amino acid sequence SEQ ID No. 71, CDR2 has an amino acid sequence SEQ ID No. 73, and CDR3 has an amino acid sequence SEQ ID No. 75, optionally having framework regions, FR1, FR2, FR3 and FR4, wherein FR1 has a sequence identity of at least 95% with SEQ ID No. 70, FR2 has a sequence identity of at least 95% with SEQ ID No. 72, FR3 has a sequence identity of at least 95% with SEQ ID No. 74, and/or FR4 has a sequence identity of at least 95% with SEQ ID No. 76.

In an embodiment, the antibody or fragment comprises
(i) a sequence of SEQ ID No. 2,
(ii) a sequence of SEQ ID No. 11,
(iii) a sequence of SEQ ID No. 20,
(iv) a sequence of SEQ ID No. 29,
(v) a sequence of SEQ ID No. 38,
(vi) a sequence of SEQ ID No. 47,
(vii) a sequence of SEQ ID No. 49,
(viii) a sequence of SEQ ID No. 58,
(ix) a sequence of SEQ ID No. 67,
(x) a sequence of SEQ ID No. 69,or
(xi) a sequence identity of at least 90%, 95%, 97%, or 99% with one of the above-mentioned sequences (i) to (x).

In an embodiment, cumulatively across all CDRs, optionally excluding CDR3, up to 3, up to 2 or up to 1 amino acid is eliminated or substituted, wherein the substitution optionally is an empirical or conservative substitution.

Optionally, cumulatively across all FRs up to 5, up to 3, up to 2 or up to 1 amino acid is eliminated or substituted, wherein the substitution optionally is an empirical or conservative substitution.

In an embodiment, the antibody or fragment thereof according to this disclosure has four framework regions, FR1, FR2, FR3 and FR4, wherein
FR1 has a sequence identity of at least 97% with SEQ ID No. 3,
FR2 has a sequence identity of at least 97% with SEQ ID No. 5,
FR3 has a sequence identity of at least 97% with SEQ ID No. 7, and/or
FR4 has a sequence identity of at least 97% with SEQ ID No. 9.

In another embodiment, the antibody or fragment thereof according to this disclosure has four framework regions, FR1, FR2, FR3 and FR4, wherein
FR1 has a sequence identity of at least 99% with SEQ ID No. 3,
FR2 has a sequence identity of at least 99% with SEQ ID No. 5,
FR3 has a sequence identity of at least 99% with SEQ ID No. 7, and/or
FR4 has a sequence identity of at least 99% with SEQ ID No. 9.

In further embodiment, the antibody or fragment thereof according to this disclosure has four framework regions, FR1, FR2, FR3 and FR4, wherein FR1 comprises SEQ ID No. 3, FR2 comprises SEQ ID No. 5, FR3 comprises SEQ ID No. 7, and/or FR4 comprises SEQ ID No. 9.

In another embodiment, the antibody or fragment thereof according to this disclosure has four framework regions, FR1, FR2, FR3 and FR4, wherein FR1 consists of SEQ ID No. 3, FR2 consists of SEQ ID No. 5, FR3 consists of SEQ ID No. 7, and/or FR4 consists of SEQ ID No. 9.

Optionally, one or more amino acids within the framework regions may be substituted. For example, cumulatively across all framework regions up to 3, up to 2 or up to 1 amino acid is eliminated or substituted, wherein the substitution may be an empirical or conservative substitution.

Optionally, one amino acid within the framework regions is substituted. For example, cumulatively across all framework regions up to 1 amino acid is eliminated or substituted, wherein the substitution is a conservative substitution.

Optionally, one amino acid within the framework regions is substituted. For example, cumulatively across all framework regions up to 2 amino acids are eliminated or substituted, wherein the substitution is a conservative substitution.

In an embodiment, the residues of the framework regions may participate in antigen binding, contributing to the outstanding performance of the antibody and/or fragments thereof disclosed herein in terms of affinity, specificity and epitope.

As discussed above, the antibody or fragment thereof comprises complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8. These CDR sequences contribute to the outstanding performance of the antibody and fragments thereof disclosed herein in terms of affinity, specificity and epitope.

In some embodiments, residues of both the framework regions and the complementarity-determining regions may participate in antigen binding.

Optionally, one or more amino acids within the CDRs may be substituted. For example, cumulatively across all CDRs up to 3, up to 2 or up to 1 amino acid is eliminated or substituted, wherein the substitution may be an empirical or conservative substitution. In an embodiment, there is no substitution in CDR3, i.e. CDR3 comprises or consists of SEQ ID No. 8, whereas cumulatively across CDRs 1 and 2 up to 3, up to 2 or up to 1 amino acid is eliminated or substituted.

Optionally, one amino acid within the CDRs is substituted. For example, cumulatively across all CDRs up to 1 amino acid is eliminated or substituted, wherein the substitution is a conservative substitution. In an embodiment, there is no substitution in CDR3, i.e. CDR3 comprises or consists of SEQ ID No. 8, whereas cumulatively across CDRs 1 and 2 up to 1 amino acid is eliminated or substituted.

Optionally, one amino acid within the CDRs is substituted. For example, cumulatively across all CDRs up to 2 amino acids are eliminated or substituted, wherein the substitution is a conservative substitution. In an embodiment, there is no substitution in CDR3, i.e. CDR3 comprises or consists of SEQ ID No. 8, whereas cumulatively across CDRs 1 and 2 up to 2 amino acids are eliminated or substituted.

Advantageously, in an embodiment, the antibody or fragment thereof is more stable than traditional antibodies. Further advantageously, in an embodiment, the antibody or fragment thereof is more thermally stable than traditional antibodies.

In an embodiment, **Example 4** demonstrates the higher stability or thermal stability of the antibody or fragment thereof.

Moreover, in an embodiment, the antibody or fragment thereof is stable for a longer storage period than traditional antibodies due to its higher resistance to temperature and pressure. The inventors assume that this property is brought about by the amino acid sequences as discussed herein.

In an embodiment, the antibody or fragment thereof comprises the amino acid sequence of SEQ ID No. 2. Optionally, the antibody or fragment thereof comprises an amino acid having a sequence identity of at least 95%, 97%, 98% or 99% with SEQ ID No. 2. For example, the antibody or fragment thereof consists essentially of the amino acid sequence of SEQ ID No. 2. Optionally, the antibody or fragment thereof consists essentially of an amino acid having a sequence identity of at least 95%, 97%, 98% or 99% with SEQ ID No. 2.

In an embodiment, the antibody or fragment thereof comprises the amino acid sequence of SEQ ID No. 11. Optionally, the antibody or fragment thereof comprises an amino acid having a sequence identity of at least 95%, 97%, 98% or 99% with SEQ ID No. 11. For example, the antibody or fragment thereof consists essentially of the amino acid sequence of SEQ ID No. 11. Optionally, the antibody or fragment thereof consists essentially of an amino acid having a sequence identity of at least 95%, 97%, 98% or 99% with SEQ ID No. 11.

In an embodiment, the antibody or fragment thereof comprises the amino acid sequence of SEQ ID No. 20. Optionally, the antibody or fragment thereof comprises an amino acid having a sequence identity of at least 95%, 97%, 98% or 99% with SEQ ID No. 20. For example, the antibody or fragment thereof consists essentially of the amino acid sequence of SEQ ID No. 20. Optionally, the antibody or fragment thereof consists essentially of an amino acid having a sequence identity of at least 95%, 97%, 98% or 99% with SEQ ID No. 20.

In an embodiment, the antibody or fragment thereof comprises the amino acid sequence of SEQ ID No. 29. Optionally, the antibody or fragment thereof comprises an amino acid having a sequence identity of at least 95%, 97%, 98% or 99% with SEQ ID No. 29. For example, the antibody or fragment thereof consists essentially of the amino acid sequence of SEQ ID No. 29. Optionally, the antibody or fragment thereof consists essentially of an amino acid having a sequence identity of at least 95%, 97%, 98% or 99% with SEQ ID No. 29.

In an embodiment, the antibody or fragment thereof comprises the amino acid sequence of SEQ ID No. 38. Optionally, the antibody or fragment thereof comprises an amino acid having a sequence identity of at least 95%, 97%, 98% or 99% with SEQ ID No. 38. For example, the antibody or fragment thereof consists essentially of the amino acid sequence of SEQ ID No. 38. Optionally, the antibody or fragment thereof consists essentially of an amino acid having a sequence identity of at least 95%, 97%, 98% or 99% with SEQ ID No. 38.

In an embodiment, the antibody or fragment thereof comprises the amino acid sequence of SEQ ID No. 47. Optionally, the antibody or fragment thereof comprises an amino acid having a sequence identity of at least 95%, 97%, 98% or 99% with SEQ ID No. 47. For example, the antibody or fragment thereof consists essentially of the amino acid sequence of SEQ ID No. 47. Optionally, the antibody or fragment thereof consists essentially of an amino acid having a sequence identity of at least 95%, 97%, 98% or 99% with SEQ ID No. 47.

In an embodiment, the antibody or fragment thereof comprises the amino acid sequence of SEQ ID No. 49. Optionally, the antibody or fragment thereof comprises an amino acid having a sequence identity of at least 95%, 97%, 98% or 99% with SEQ ID No. 49. For example, the antibody or fragment thereof consists essentially of the amino acid sequence of SEQ ID No. 49. Optionally, the antibody or fragment thereof consists essentially of an amino acid having a sequence identity of at least 95%, 97%, 98% or 99% with SEQ ID No. 49.

In an embodiment, the antibody or fragment thereof comprises the amino acid sequence of SEQ ID No. 58. Optionally, the antibody or fragment thereof comprises an amino acid having a sequence identity of at least 95%, 97%, 98% or 99% with SEQ ID No. 58. For example, the antibody or fragment thereof consists essentially of the amino acid sequence of SEQ ID No. 58. Optionally, the antibody or fragment thereof consists essentially of an amino acid having a sequence identity of at least 95%, 97%, 98% or 99% with SEQ ID No. 58.

In an embodiment, the antibody or fragment thereof comprises the amino acid sequence of SEQ ID No. 67. Optionally, the antibody or fragment thereof comprises an amino acid having a sequence identity of at least 95%, 97%, 98% or 99% with SEQ ID No. 67. For example, the antibody or fragment thereof consists essentially of the amino acid sequence of SEQ ID No. 67. Optionally, the antibody or fragment thereof consists essentially of an amino acid having a sequence identity of at least 95%, 97%, 98% or 99% with SEQ ID No. 67.

In an embodiment, the antibody or fragment thereof comprises the amino acid sequence of SEQ ID No. 69. Optionally, the antibody or fragment thereof comprises an amino acid having a sequence identity of at least 95%, 97%, 98% or 99% with SEQ ID No. 69. For example, the antibody or fragment thereof consists essentially of the amino acid sequence of SEQ ID No. 69. Optionally, the antibody or fragment thereof consists essentially of an amino acid having a sequence identity of at least 95%, 97%, 98% or 99% with SEQ ID No. 69.

In a preferable embodiment, the antibody or fragment thereof comprises the amino acid sequence of SEQ ID No. 2. Optionally, the antibody or fragment thereof comprises an amino acid having a sequence identity of at least 95%, 97%, 98% or 99% with SEQ ID No. 2. For example, the antibody or fragment thereof consists essentially of the amino acid sequence of SEQ ID No. 2. Optionally, the antibody or fragment thereof consists essentially of an amino acid having a sequence identity of at least 95%, 97%, 98% or 99% with SEQ ID No. 2.

In one embodiment, the size of the antibody or fragment thereof according to the present disclosure ranges from 7.0 kDa to 25.0 kDa, or from 8.0 kDa to 22.o kDa, or from 9.0 kDa to 21.0 kDa, or preferably from 10.0 kDa to 20.0 kDa, or more preferably from 10.0 kDa to 18.0 kDa.

In another embodiment, the size of the antibody or fragment thereof according to the present disclosure ranges from 50.0 kDa to 200.0 kDa, or from 60.0 kDa to 150.0 kDa, or from 70.0 kDa to 100.0 kDa, or from 75.0 kDa to 95.0 kDa, or from 80.0 kDa to 90.0 kDa, or preferably from 80.0 kDa to 85.0 kDa.

In an embodiment, the size of the minibody according to the present disclosure ranges from 60.0 kDa to 105.0 kDa, preferably from 70.0 kDa to 95.0 kDa, more preferably from 80.0 kDa to 85.0 kDa.

In an embodiment, the size of the minibody according to the present disclosure ranges from 70.0 kDa to 95.0 kDa.

In another embodiment, the size of the antibody or fragment thereof according to the present disclosure ranges from 100.0 kDa to 200.0 kDa, or from 110.0 kDa to 190.0 kDa, or from 120.0 kDa to 180.0 kDa, or preferably from 130.0 kDa to 170.0 kDa, or preferably from 140.0 kDa to 160.0 kDa.

In embodiments, the size of the antibody or fragments thereof according to the present disclosure is of at least 5.0 kDa, or of at least 10.0 kDa, or of at least 15.0 kDa, or of at least 20.0 kDa, or of at least 30.0 kDa, or of at least 40.0 kDa, or of at least 50.0 kDa, or of at least 75.0 kDa, or of at least 100.0 kDa, or of at least 150.0 kDa, or of at least 200.0 kDa, or of at least 300.0 kDa, or of at least 400.0 kDa.

In embodiments, the size of the antibody or fragments thereof according to the present disclosure is of at most 20.0 kDa, or of at most 18.0 kDa, or of at most 16.0 kDa, or of at most 14.0 kDa, or of at most 12.0 kDa.

In an embodiment, the size of the antibody or fragment thereof according to the present disclosure ranges from 10.0 to 18.0 kDa.

Advantageously, in an embodiment, the antibody or fragment thereof can penetrate into target tissue deeper and more easily than conventional antibodies due to its smaller size. Especially advantageously, in an embodiment, the antibody or fragment thereof can pass the blood-brain-barrier due to its small size.

Comprising two heavy chains and two light chains, full-length antibodies typically are too large to penetrate certain tissues. This is especially the case for tumors in the brain, as full-length antibodies, because of their size, cannot diffuse through the blood-brain-barrier.

Advantageously, because of its small size, the single-domain antibody according to the first aspect does not sterically prevent isatuximab from simultaneously binding to CD38.

In some preferred embodiments, the antibodies and fragments thereof according to the present disclosure are humanized. Advantageously, humanized therapeutic monoclonal antibodies are less immunogen. Advantageously, the humanized antibodies and fragments thereof according to the present disclosure are not immunogen when administered to a human patient.

In some embodiments, the antibody or fragment thereof according to this disclosure is conjugated to at least one effector molecule, optionally selected from the group consisting of: a detectable label, a radionuclide, a cytotoxic drug or combinations thereof. Conjugation may include conjugation by direct or indirect chemical and/or physical bonds, wherein indirect bonds are those mediated by additional chemical structures such as linkers or chelators, whereas direct bonding occurs without such additional chemical structures. Chemical bonds include covalent bonds and hydrogen bonds. Physical bonds include ionic bonds, coordination complexes and van der Waals interaction.

Optionally, the antibody or fragment thereof is a single-domain antibody , or an antibody-construct comprising a single-domain antibody fused to an Fc region of an immunoglobulin heavy chain, such as human IgG, optionally human IgG1 (also referred here to as "minibody"). In preferred embodiments, the term "minibody" refers to a human recombinant heavy chain antibody (hrHcAb).

An advantage of the present disclosure is the higher thermostability of the antibody or fragment when compared to traditional antibodies. In an embodiment, the antibody or fragment thereof is stable at temperatures of up to 75 °C, or up to 72°C, such as about 70°C. An antibody or fragment of this disclosure is considered stable at a given temperature, if the temperature is below its melting temperature (measured using TSA as used in example 4). **Example 4** shows 4 out of 5 single-domain antibodies of the present disclosure that have a melting temperature at approximately 70 °C, demonstrating robust stability and thermal resistance.

In embodiments, the antibody or fragment thereof according to the present disclosure has a melting temperature of up to 60 °C, or of up to most 70 °C, or of up to 80 °C, or preferably of between 70 °C and 80 °C.

The stability of the antibodies or fragments thereof according to the present disclosure can be measured by methods known in the art, preferably chosen from Differential Scanning Fluorometry (DSF) and Thermal Shift Assay (TSA).

The basic principle of the preferred methods consists in measuring the fluorescence signal of a molecular dye that becomes fluorescent upon binding to hydrophobic regions of a protein. At low temperatures, the protein is fully folded, usually hiding the hydrophobic regions in its interior, inaccessible to the molecular dye. Increasing temperature leads to protein denaturation, gradually exposing hydrophobic domains to the molecular dye which, upon binding to the hydrophobic regions, emits fluorescent signals.

DSF and TSA typically differ in the measuring of the melting temperature (Tₘ). While DSF uses the first derivative of the curve in order to determine Tₘ, TFA uses 50% of the maximum measured intensity to determine Tₘ.

In embodiments of the present disclosure, TSA in a Real-Time PCR device was used in order to measure the stability of the antibodies and fragments thereof according to the present disclosure.

According to the second aspect, the antibody or fragment thereof according to the first aspect is used in a method of therapy, diagnosis *in vivo* or *in vitro* or surgery.

The potential uses of the antibodies and fragments according to this disclosure are manifold. Due to the high affinities of the molecules disclosed herein, they find particular use *in vitro* or *in vivo* diagnostic and analytic applications. In diagnostic and analytic applications, the antibodies and fragments of this disclosure may require conjugation to a detectable label such as a fluorescent molecule, a dye or a radionuclide for detection of the antibody or fragment. For example, the antibody or fragment thereof may be very useful for detection of CD38 or CD38 expressing cells *in vitro* or *in vivo.* In an embodiment, the antibodies or fragments may be used to diagnose or detect cancer cells, e.g. melanoma cells, that express CD38. When used *in vivo,* the antibody or fragment may be conjugated to a radionuclide to facilitate detection from outside the body.

This disclosure includes an *in vitro* method for analysis or diagnosis of a disease in a sample, comprising contacting the antibody or fragment thereof with the sample. The sample can be a biological sample, such as a blood sample or a tissue sample.

The antibodies or fragments of the present disclosure are suitable for staining immune cells of subjects, providing the means for diagnosing conditions in patients or tracking the progress of therapies. Example 7 presents the successful staining of immune cells from blood samples using molecules according to the present disclosure.

In **Example 7,** T-cells, B-cells and monocytes can be successfully detected using antibodies or fragments thereof according to the present disclosure.

In an embodiment, the antibody or fragment thereof is used in the diagnosis of myeloma, such as multiple myeloma.

In some embodiments, the antibody or fragment thereof is used in the diagnosis of CD38 positive cancer.

In some embodiments, the antibody or fragment thereof is used in the diagnosis of hematologic diseases, such as Multiple Myeloma (MM), Chronic Lymphotic Leukemia (CLL), Acute Lymphoblastic Leukemia (ALL), Non-Hodgkin's Lymphoma (NHL), Diffuse Large B-Cell Lymphoma (DLBCL) and Light Chain Amyloidosis.

In some embodiments, the antibody or fragment thereof is used in the diagnosis of autoimmune diseases such as Rheumatoid Arthritis (RA).

In some embodiments, the antibody or fragment thereof is used in the diagnosis of neurodegenerative diseases such as Alzheimer's Disease (AD), Parkinson's Diseases (PD), Amyotrophic Lateral Sclerosis (ALS) or Multiple Sclerosis (MS).

In some embodiments, the antibody or fragment thereof is used in monitoring the activity of immune cells following organ transplantation.

In embodiments of the present disclosure, the antibodies and/or fragments thereof are conjugated to radionuclides for use in diagnostics.

Diagnostics can be performed by methods known in the art, e.g. by imaging using Positron Emission Tomography (PET) or Single-Photon Emission Computed Tomography (SPECT).

One or more radionuclides for imaging can be chosen from the list comprising 89Zr, 64Cu, 99mTc, 111In and 18F.

In embodiments, the antibodies or fragments of the present disclosure for use in diagnostics are conjugated with one or more radionuclides chosen from the list comprising 89Zr, 64Cu, 99mTc, 111In and 18F for use in PET or SPECT imaging.

Notably, the high affinities of the antibodies or fragments thereof according to the present disclosure are particularly beneficial for imaging for the use in diagnosis.

According to the second aspect, the antibody or fragment thereof according to the first aspect is used in a method of therapy, diagnosis *in vivo* or *in vitro* or surgery.

Thus in one embodiment, a method of therapy includes
- treating a subject, such as a human, in need thereof with an effective amount of a therapeutic principle for treatment of a disease or a condition,
- administering to the subject an effective amount of the antibody or fragment thereof according to this disclosure,
- determining whether the treatment was effective,

wherein the disease relates to CD38 positive or overexpressing cells, such as myeloma, including multiple myeloma and/or plasma cell myeloma;
wherein the disease may relate to hematologic diseases such as Multiple Myeloma (MM), Chronic Lymphotic Leukemia (CLL), Acute Lymphoblastic Leukemia (ALL), Non-Hodgkin's Lymphoma (NHL), Diffuse Large B-cell Lymphoma (DLBCL) or Light Chain Amyloidosis;
wherein the disease may relate to autoimmune diseases such as Rheumatoid Arthritis (RA);
wherein the disease may relate to neurodegenerative diseases such as Alzheimer's Disease (AD), Parkinson's Disease (PD), Amyotrophic Lateral Sclerosis (ALS) or Multiple Sclerosis (MS);
wherein the condition may relate to transplant rejection following organ transplantation;
wherein optionally the antibody or fragment is conjugated to a detectable label;
wherein optionally the antibody or fragment thereof is administered to the subject in the form of an RNA-vaccine;
wherein optionally the antibody or fragment thereof is administered to the subject in the form of a Chimeric Antigen Receptor for use in CAR T-cell therapy;
wherein optionally the method of therapy further comprises the administration of isatuximab; and
wherein optionally determining whether the treatment was effective includes evaluating the number, amount, density, existence and/or location of the CD38 positive or overexpressing cells.

In some embodiments, the antibody or fragment thereof is administered to the subject in the form of an RNA-vaccine by using lipid nanoparticles to deliver the RNA coding for the antibodies or fragments thereof. Upon successful administration to the subject's cells, the administered RNA will be translated to the antibodies or fragments thereof according to the present disclosure.

In one embodiment, the antibody or fragment thereof according to the present disclosure may be used in theranostics, i.e. the combination of therapy and diagnostics.

Optionally, the antibody or fragment thereof according to the present disclosure may be used in theranostics, e.g. by additionally determining whether the treatment was effective by evaluating the number, amount, density, existence and/or location of the CD38 positive or overexpressing cells.

Optionally, the antibody or fragment thereof according to the present disclosure may be used in theranostics, e.g. by monitoring the success of a method of therapy of a disease or a condition according to the present disclosure.

In embodiments of the present disclosure, the antibodies and/or fragments thereof are conjugated to radionuclides for use in theranostics, i.e. the combination of therapy and diagnostics.

In embodiments according to the present disclosure, when used in theranostics, the antibodies of fragments thereof are conjugated to radioactive isotopes that are able to both enable visualization of the therapy target as well as neutralize or impact the growth and proliferation of the tumor cells. One or more radionuclides for use in theranostics can be chosen from the list comprising 177Lu, 90Y, 225Ac and 213Bi.

In one embodiment, the therapy according to the second aspect comprises a CAR T-cell therapy.

In one embodiment, the therapy according to the second aspect comprises a CAR (Chimeric Antigen Receptor) T-cell therapy, wherein the antibody or fragment thereof according to the present disclosure is a Chimeric Antigen Receptor (CAR) protein.

In one embodiment, the therapy according to the second aspect comprises the administration of an RNA-vaccine, wherein the RNA is coding for an antibody or fragment thereof according to the present disclosure.

In one embodiment, the therapy according to the second aspect relates to a combination therapy of isatuximab and the antibody or fragment thereof according to the present disclosure. In one embodiment, the therapy according to the second aspect relates to a combination therapy of isatuximab and the antibody or fragment thereof according to the present disclosure, wherein optionally the antibody or fragment thereof is administered to the subject in the form of an RNA-vaccine; wherein optionally the antibody or fragment thereof is administered to the subject in the form of a Chimeric Antigen Receptor (CAR); or wherein optionally determining whether the treatment was effective includes evaluating the number, amount, density, existence and/or location of the CD38 positive or overexpressing cells.

In preferred embodiments, the antibodies or fragments thereof for use in CAR T-cell therapy are bivalent or multivalent.

According to the third aspect, the antibody or fragment thereof according to the first aspect is used in
- a method of treating hematologic diseases CD38 positive cancer, such as cancer overexpressing CD38, including diseases from the hematological field, such as Multiple Myeloma (MM), Chronic Lymphotic Leukemia (CLL), Acute Lymphoblastic Leukemia (ALL), Non-Hodgkin's Lymphoma (NHL), Diffuse Large B-cell Lymphoma (DLBCL) and Light Chain Amyloidosis;
- a method of treating autoimmune diseases such as Rheumatoid Arthritis (RA);
- a method of treating neurodegenerative diseases such as Alzheimer's Disease (AD), Parkinson's Disease (PD), Amyotrophic Lateral Sclerosis (ALS) or Multiple Sclerosis (MS); or
- a method of immunosuppression, e.g. preventing transplant rejection following organ transplantation.

As mentioned above, the antibody or fragment thereof may be used in the treatment of cancer, such as CD38 positive cancer or cancer wherein CD38 is overexpressed. Examples of cancers that can be treated with the antibodies and fragments of this disclosure are hematological malignancies.

In particular, the antibody or fragment thereof may be used in the treatment of Multiple Myeloma.

In Chronic Lymphocytic Leukemia, CD38 expression correlates with poor prognosis and aggressive disease progression. In embodiments of the present disclosure, the antibody or fragment thereof can be used in the treatment of Chronic Lymphocytic Leukemia.

In Acute Lymphoblastic Leukemia, some leukemic B-cells express CD38, able to be targeted in therapy. In embodiments of the present disclosure, the antibody or fragment thereof can be used in the treatment of Acute Lymphoblastic Leukemia.

CD38 may be involved in certain Non-Hodgkin's Lymphoma subtypes, particularly Diffuse Large B-Cell Lymphoma. In embodiments, the antibody or fragment thereof of the present disclosure is suitable for use in the treatment of Non-Hodgkin's Lymphoma. In particular embodiments, the antibody or fragment thereof of the present disclosure is suitable for use in the treatment of Diffuse Large B-Cell Lymphoma.

In embodiments, the antibody or fragment thereof may be used in the treatment of autoimmune diseases. In Rheumatoid Arthritis, CD38 is upregulated in synovial fibroblasts and contributes to the inflammation. In embodiments, the antibody or fragment thereof of the present disclosure can be used in the treatment of Rheumatoid Arthritis.

In embodiments, the antibody or fragment thereof may be used in the treatment of neurodegenerative diseases.

In Alzheimer's Disease, CD38 regulates NAD+ levels, which are implicated in cognitive decline and neuroinflammation. As such, in embodiments, the antibody or fragment thereof according to the present disclosure is suitable for use in the treatment of Alzheimer's Disease.

In Parkinson's Disease, CD38 dysfunction affects dopaminergic neuron survival. In embodiments, the antibody or fragment thereof according to the present disclosure is suitable for use in the treatment of Parkinson's Disease.

In Amyotrophic Lateral Sclerosis, CD38 is linked to neuroinflammation and motor neuron degeneration. In embodiments, the antibody or fragment thereof according to the present disclosure is suitable for use in the treatment of Amyotrophic Lateral Sclerosis.

In Multiple Sclerosis, CD38 is involved in neuroinflammatory pathways and demyelination. In embodiments, the antibody or fragment thereof according to the present disclosure is suitable for use in the treatment of Multiple Sclerosis.

In particular useful embodiments for use in the treatment of neurodegenerative diseases, the antibody or fragments thereof of the present disclosure are capable of passing the blood-brain barrier.

In embodiments, the antibody or fragment thereof according to the present disclosure is suitable for use in transplantation medicine, e.g. for treatment or prevention of transplant rejection. Targeting CD38 has a high chance of reducing rejection risk and of enhancing graft tolerance in organ transplantation of e.g. kidney, liver, heart, and lung.

Blocking the enzymatic activity of CD38 or binding of CD38 with a very high affinity increases the chance of reducing T-cell-mediated rejection, occurring when the new, transplanted organ is attacked by T-cells. Further effects are the suppression of antibody-mediated rejection, minimizing the amount of antibodies produced by B-cells (B-cell control), and the decrease of inflammation and fibrosis, with the blocking T-cells and B-cells resulting in lower cytokine levels.

Thus, another therapeutic use of the antibody or fragment thereof binding to CD38 according to the present disclosure is the prevention of transplant rejection following organ transplantation.

In the context of the therapeutic methods disclosed herein, the methods may include administering an effective amount of the antibody or fragment thereof to a subject. The subject may be a mammal such as a human. The subject may be in need of therapy. Similarly, in a diagnostic method *in vivo* the method may include administering an effective amount of the antibody or fragment of this disclosure to a subject.

Without wishing to be bound by this theory, the inventors hypothesize that one effect of the antibodies and fragments thereof of this disclosure is that CD38 cyclase activity is inhibited. Thus, in one embodiment, the methods of therapy of this disclosure may include inhibiting the cyclase activity of CD38. Optionally, the antibody or fragment thereof is used in combination with isatuximab thus increasing the inhibiting activity on CD38 enzyme functions. Optionally, the combination of the antibody or fragment thereof according to the present disclosure and isatuximab demonstrate a synergistic effect in inhibiting CD38 cyclase activity. Optionally, the combination of the antibody or fragment thereof according to the present disclosure and isatuximab demonstrate a synergistic effect in inhibiting CD38 hydrolase activity. Optionally, the combination of the antibody or fragment thereof according to the present disclosure and isatuximab demonstrate a synergistic effect in inhibiting CD38 cyclase and/or hydrolase activity. Notably, when used in combination, the antibody or fragment thereof according to the present disclosure and isatuximab demonstrate a synergistic effect in inhibiting the CD38 cyclase and/or hydrolase enzymatic activity, far exceeding the magnitude of the mere addition of the individual effects. Particularly, when used in combination, the minibody according to the present disclosure and isatuximab demonstrate a synergistic effect in inhibiting the CD38 cyclase and/or hydrolase enzymatic activity, far exceeding the magnitude of the mere addition of the individual effects. In preferred embodiments, the single-domain antibodies according to the present disclosure are efficient in inhibiting the cyclase activity of CD38. In preferred embodiments, the minibodies according to the present disclosure are efficient in inhibiting the cyclase activity of CD38. In some embodiments, the antibodies or fragments thereof according to the present disclosure are more efficient than daratumumab in inhibiting the cyclase activity of CD38. In preferred embodiments, the minibodies according to the present disclosure are more efficient than daratumumab in inhibiting the cyclase activity of CD38. In preferred embodiments, the combination of isatuximab and the antibody or fragments thereof according to the present disclosure has a synergistic effect in inhibiting the cyclase activity of CD38. In preferred embodiments, the combination of isatuximab and the minibodies of the present disclosure has a strong synergistic effect in inhibiting the cyclase activity of CD38. In **Example 9,** the minibodies of the present disclosure are more efficient than daratumumab in inhibiting the cyclase activity of CD38. In **Example 9,** the combinatorial treatment comprising isatuximab and the minibodies according to the present disclosure demonstrate a synergistic effect in inhibiting the cyclase activity of CD38. In preferred embodiments, the combination of isatuximab and the antibody or fragments thereof according to the present disclosure has a synergistic effect in inhibiting the hydrolase activity of CD38. In preferred embodiments, the combination of isatuximab and the minibodies of the present disclosure has a strong synergistic effect in inhibiting the hydrolase activity of CD38. In **Example 10,** the combinatorial treatment comprising isatuximab and the minibodies according to the present disclosure demonstrate a synergistic effect in inhibiting the hydrolase activity of CD38.Without wishing to be bound by this theory, the inventors hypothesize that one effect of the antibodies and fragments thereof of this disclosure is that they induce complement-dependent cytotoxicity. Thus, in one embodiment, the methods of therapy of this disclosure may include inducing complement-dependent cytotoxicity.

Notably, the antibodies or fragments thereof according to the present disclosure are more efficient than daratumumab at inducing complement-dependent cytotoxicity at low concentrations.

In **Example 11,** the minibodies according to the present disclosure are more efficient than daratumumab at inducing complement-dependent cytotoxicity at low concentrations.

Advantageously, the antibodies or fragments thereof according to the present disclosure are more cost-effective than therapeutic antibodies of the prior art, as they achieve better therapeutic effects at lower concentrations.

Notably, the combination of isatuximab and the antibodies or fragments thereof according to the present disclosure has a synergistic effect in inducing complement-dependent cytotoxicity at low concentrations, far exceeding the magnitude of the mere addition of the individual effects of isatuximab or the antibodies or fragments thereof according to the present disclosure.

In **Example 12,** the combination of isatuximab and the minibodies according to the present disclosure has a strong synergistic effect in inducing complement-dependent cytotoxicity at low concentrations, far exceeding the magnitude of the mere addition of the individual effects of isatuximab or the minibody according to the present invention.

Without wishing to be bound by this theory, the inventors hypothesize that one effect of the antibodies and fragments thereof of this disclosure is that they induce antibody-dependent cellular cytotoxicity. Thus, in one embodiment, the methods of therapy of this disclosure may include inducing antibody-dependent cellular cytotoxicity.

Notably, the antibodies or fragments thereof according to the present disclosure are more efficient than daratumumab at inducing antibody-dependent cellular cytotoxicity, especially at low concentrations. Notably, the antibodies or fragments thereof according to the present disclosure are more efficient than isatuximab at inducing antibody-dependent cellular cytotoxicity, especially at low concentrations. Notably, the antibodies or fragments thereof according to the present disclosure are more efficient than daratumumab and isatuximab at inducing antibody-dependent cellular cytotoxicity, especially at low concentrations. Advantageously, the antibodies or fragments thereof according to the present disclosure are more cost-effective than therapeutic antibodies of the prior art, as they achieve better therapeutic effects at lower concentrations. Notably, the combination of isatuximab and antibodies or fragments thereof according to the present disclosure is more efficient than daratumumab at inducing antibody-dependent cellular cytotoxicity. Notably, the combination of isatuximab and antibodies or fragments thereof according to the present disclosure is more efficient than isatuximab at inducing antibody-dependent cellular cytotoxicity. Notably, the combination of isatuximab and antibodies or fragments thereof according to the present disclosure is comparable or better compared with the antibodies or fragments thereof according to the present disclosure at inducing antibody-dependent cellular cytotoxicity. Notably, the high affinities of the antibodies or fragments thereof according to the present disclosure are particularly beneficial in inducing antibody-dependent cellular cytotoxicity.

In **Example 13,** the minibodies according to the present disclosure are more efficient than daratumumab and isatuximab at inducing antibody-dependent cellular cytotoxicity, especially at low concentrations. Furthermore, the combination of isatuximab and the minibodies according to the present disclosure is comparable or better compared with the minibodies according to the present disclosure at inducing antibody-dependent cellular cytotoxicity.

In embodiments, the antibodies or fragments thereof are able to specifically bind CD38, independent of glycosylation.

In **Example 14,** a single-domain antibody according to the present disclosure is able to bind a human CD38 glycomutant. Alternatively or in addition, therapeutic effects may be achieved by conjugating the antibodies or fragments thereof to an effector molecule such as a drug molecule, e.g. a cytotoxic drug or radionuclide. In this context, the antibody or fragment is used as a targeting molecule to deliver the effector molecule to a target cell, e.g. a CD38 expressing cell or a cell that overexpresses CD38.

Examples for cytotoxic drugs include microtubule inhibitors, DNA-damaging agents, topoisomerase I inhibitors, RNA polymerase II inhibitors and immune stimulators but are not limited thereto. One or more microtubule inhibitors can be chosen from the list consisting of monomethyl auristatin F (MMAF), monomethyl auristatin E (MMAE), DM1 and DM4. One or more DNA-damaging agents can be chosen from the list consisting of pyrrolobenzodiazepine dimers, duocarmycins and calicheamicins. One or more topoisomerase I inhibitors can be chosen from the list consisting of Camptothecin and its derivates, e.g. SN-38. An example of an RNA polymerase II inhibitor is amanitin. Examples for radionuclides include 89Zr, 64Cu, 99mTc, 111In, 18F, 177Lu, 90Y, 225Ac and 213Bi, without being limited thereto. One or more radionuclides can be chosen from the list consisting of include 89Zr, 64Cu, 99mTc, 111In, 18F, 177Lu, 90Y, 225Ac and 213Bi.

### Sequences

A sequence listing is filed herewith. Below are the sequences comprised within the sequence listing and their respective SEQ IDs.
**SEQ ID No. 1 (NbC6)** 
GAGGTGCAGCTGGTGGAGTCTGGGGGAGGCTTGGTGCAGGCTGGGGGGTCTCTGAGACTCT
CCTGTGCAGTCTCTGGAGGCGCGTTCAATAACTTTGCCATGGGCTGGTTCCGCCAGGCTCCA
GGGAAGGAGCGTGAGTTTGTTGCAGCTATTACCTGGGGTTCTGGCACACTCTATGCAGACTC
CGTGAAGGGCCGATTCACCCTCTCCAGAGACAACCCCAAAAACACGGCGTATCTGCAAATGG
ACAGTCTGAAACCTGAGGACACGGCCGTTTATTTCTGTGCAGGAAAGTATTTCACGACGGCC
CGACTACCGGCGGAATATAAGTACTGGGGTCAGGGGACCCAGGTCACCGTCTCCTCA
**SEQ ID No. 2 (NbC6)**
   Translation 122 a.a. MW=13194.24 
EVQLVESGGGLVQAGGSLRLSCAVSGGAFNNFAMGWFRQAPGKEREFVAAITWGSGTLYADSV
KGRFTLSRDNPKNTAYLQMDSLKPEDTAVYFCAGKYFTTARLPAEYKYWGQGTQVTVSS

<--------- FR1-----------><-CDR1><-----FR2--------><CDR2>
EVQLVESGGGLVQAGGSLRLSCAVSGGAFNNFAMGWFRQAPGKEREFVAAITWGSGT
<----------------FR3------------------><----CDR3---->
 
LYADSVKGRFTLSRDNPKNTAYLQMDSLKPEDTAVYFCAGKYFTTARLPAEYKY
<---FR4--->
WGQGTQVTVSS
**SEQ ID No. 3**
   EVQLVESGGGLVQAGGSLRLSCAVSG
**SEQ ID No. 4**
   GAFNNF
**SEQ ID No. 5**
   AMGWFRQAPGKEREFVAAI
**SEQ ID No. 6**
   TWGSGT
**SEQ ID No. 7**
   LYADSVKGRFTLSRDN PKNTAYLQM DSLKPEDTAVYFCAG
**SEQ ID No. 8**
   KYFTTARLPAEYKY
**SEQ ID No. 9**
   WGQGTQVTVSS
**SEQ ID No. 10 (NbB2)** 
GAGGTGCAGCTGGAGGAGTCTGGGGGAGGATTGGTGCAGGCTGGGGGCTCGCTAAGACTCT
CCTGTGCAGCCTCTGGACGCACCCTCAGTAACTATCACATGGGCTGGTTCCGCCAGGCTCCA
GGGAAGGAGCGTGAGTTTGTAGCAGCTATTAACTGGAGCGGTCTTAACACATACTATATGGAC
TCCGTGAAGGGCCGATTCACCGTCTCCAGAGACAACGCCAAGAACACGGTGTATCTGCAAAT
GAACAGCCTGAAACCTGAGGACACGGCCGTTTATTACTGTGCAGCACAATTCTTCGCGGCAG
CAGTTGGTAGGGACTCTGCGAAGTATGCGTACTGGGGCCAGGGGACCCAGGTCACCGTCTC
CTCA
**SEQ ID No. 11 (NbB2)**
   Translation 125 a.a. MW=13670.71 
EVQLEESGGGLVQAGGSLRLSCAASGRTLSNYHMGWFRQAPGKEREFVAAINWSGLNTYYMDS
VKGRFTVSRDNAKNTVYLQMNSLKPEDTAVYYCAAQFFAAAVGRDSAKYAYWGQGTQVTVSS
**SEQ ID No. 12**
   EVQLEESGGGLVQAGGSLRLSCAASG
**SEQ ID No. 13**
   RTLSNYH
**SEQ ID No. 14**
   MGWFRQAPGKEREFVAAI
**SEQ ID No. 15**
   NWSGLN
**SEQ ID No. 16**
   TYYMDSVKGRFTVSRDNAKNTVYLQMNSLKPEDTAVYYCAA
**SEQ ID No. 17**
   QFFAAAVGRDSAKYAY
**SEQ ID No. 18**
   WGQGTQVTVSS
**SEQ ID No. 19 (NbE11)** 
GAGGTGCAGCTGCTGGAGTCTGGGGGAGGATTGGTGCAGGCTGGGGCCTCTCTGAGACTCT
CCTGTGCAGCCTCTGGACGCACCTTCAGAAACTCTGCCATGGGCTGGTTCTGCCAGGCTCCA
GGGAAGGGGCGTGAGTTTGTAGCAGGCATGAGCTGGAGTGGCAGCACACTCTATGCAGACT
CCGTGAAGGGCCGATTCACTATCTCCAGAGACAACGCCAAGAACATGGTGTATCTGCAAATG
AACAGCCTGGCACCTGAGGACACGGCCGTTTATTTCTGTGCAGCAAGGAGCCGTATGTTTGT
AACCAGATCACCGGACGAGTATGACTACTGGGGCCAGGGGACCCAGGTCACCGTCTCCTCA
**SEQ ID No. 20 (NbE11)**
   Translation 123 a.a. MW=13359.43 
EVQLLESGGGLVQAGASLRLSCAASGRTFRNSAMGWFCQAPGKGREFVAGMSWSGSTLYADS
VKGRFTISRDNAKNMVYLQMNSLAPEDTAVYFCAARSRMFVTRSPDEYDYWGQGTQVTVSS
**SEQ ID No. 21**
   EVQLLESGGGLVQAGASLRLSCAASG
**SEQ ID No. 22**
   RTFRNSA
**SEQ ID No. 23**
   MGWFCQAPGKGREFVAGM
**SEQ ID No. 24**
   SWSGST
**SEQ ID No. 25**
   LYADSVKGRFTISRDNAKNMVYLQMNSLAPEDTAVYFCAA
**SEQ ID No. 26**
   RSRMFVTRSPDEYDY
**SEQ ID No. 27**
   WGQGTQVTVSS
**SEQ ID No. 28 (NbD11)** 
GAGGTGCAGCTGGAGGAGTCTGGGGGAGGATTGGTGCAGGCTGGGGGCTCTCTAAGACTCT
CCTGTGCAGCCTCCGAACGCACCTTCGGTAACTACCCCATGGGCTGGTTCCGCCAGGTTCCA
GGGAAGGAACGTGAGTTTGTAGCAGCTATTACTGGGCTTGCGGGTAGCCGACTCTATGCAGA
CTCCGCGAAGGGCCGCTTCACCATCTCCCGAGACAACGCCAAGAACACGGTGTATCTGCAAA
TGAACAACCTACAACCTGAGGACACGGGCGTTTATTACTGTGCAGCGGATCGACGAATAGGT
GGAGTAACGAGGGAGTCGAGGGAGTACGAGTACTGGGGTCAGGGGACCCAGGTCACCGTCT
CCTCA
**SEQ ID No. 29 (NbD11)**
   Translation 125 a.a. MW=13714.64 
EVQLEESGGGLVQAGGSLRLSCAASERTFGNYPMGWFRQVPGKEREFVAAITGLAGSRLYADSA
KGRFTISRDNAKNTVYLQMNNLQPEDTGVYYCAADRRIGGVTRESREYEYWGQGTQVTVSS
**SEQ ID No. 30**
   EVQLEESGGGLVQAGGSLRLSCAASE
**SEQ ID No. 31**
   RTFGNYP
**SEQ ID No. 32**
   MGWFRQVPGKEREFVAAI
**SEQ ID No. 33**
   TGLAGS
**SEQ ID No. 34**
   RLYADSAKGRFTISRDNAKNTVYLQMNNLQPEDTGVYYCAA
**SEQ ID No. 35**
   DRRIGGVTRESREYEY
**SEQ ID No. 36**
   WGQGTQVTVSS
**SEQ ID No. 37 (NbH11)** 
GAGGTGCAGCTGCTGGAGTCTGGGGGAGGATTGGTGCAGGCTGGGGACTCTCTGCGACTCT
CCTGTGTAGCCTCTGGACGTACTTCTAGTTTCTATACCATGGCCTGGTTCCGCCAGGCTCCAG
GGAAGGAGCGTGAGTTTGTAGCGTCGGTTGGGTGGAGTGGTGGAAGCACATTATATGCTGAC
TCCGTGAAGGGCCGAAGCACCATCTCCAGAGACAACGCCAACGCCAAGAATATGTTGTATTT
GCAAGTGGACAGGCTGAAAGTTGAGGACACGGCCGTTTATTACTGTGCAACCAGAAGGGGC
GTCAGTAGTTCGCGGTTGGTCAGTGAGTATGACTACTGGGGCCAGGGGACCCAGGTCACCG
TCTCCTCA
**SEQ ID No. 38 (NbH11)**
   Translation 126 a.a. MW=13739.71 
EVQLLESGGGLVQAGDSLRLSCVASGRTSSFYTMAWFRQAPGKEREFVASVGWSGGSTLYADS
VKGRSTISRDNANAKNMLYLQVDRLKVEDTAVYYCATRRGVSSSRLVSEYDYWGQGTQVTVSS
**SEQ ID No. 39**
   EVQLLESGGGLVQAGDSLRLSCVASG
**SEQ ID No. 40**
   RTSSFYT
**SEQ ID No. 41**
   MAWFRQAPGKEREFVASV
**SEQ ID No. 42**
   GWSGGS
**SEQ ID No. 43**
   TLYADSVKGRSTISRDNANAKNMLYLQVDRLKVEDTAVYYCAT
**SEQ ID No. 44**
   RRGVSSSRLVSEYDY
**SEQ ID No. 45**
   WGQGTQVTVSS
**SEQ ID No. 46 (NbStar1)** 
GAGGTGCAGCTGCTGGAGTCTGGGGGAGGATTGGTGCAGGCTGGGGCCTCTCTGAGACTCT
CATGTGCAACTTCTGGACGCACACTCAGTAACTTTGCCATGGGCTGGTTCCGCCAGGCTCCA
GGTAAGGGGCGTGAGTTTGTAGCAGCTGTGAGCTGGGCTGCAAGCACACTCTATGCGGACT
CCGTGAAGGGCCGATTCACCATCTCCAGAGACAACGCCAAAAACATGCTGTATCTCCAAATGA
ACAGCCTGAAACCTGAGGACACGGCCGTATATTTTTGTGCAGCAAGGAAGCGTATGTGGGTA
GACAGATCACCGGATGAGTATAACTACTGGGGCCAGGGGACCCAGGTCACCGTCTCCTCAGA
ACCCAAGAAACCAAAACCACAAACT
**SEQ ID No. 47 (NbStar1)**
   Translation 132 a.a. MW=14577.960000000008 
EVQLLESGGGLVQAGASLRLSCATSGRTLSNFAMGWFRQAPGKGREFVAAVSWAASTLYADSV
KGRFTISRDNAKNMLYLQMNSLKPEDTAVYFCAARKRMWVDRSPDEYNYWGQGTQVTVSSEPK
KPKPQT
**SEQ ID No. 48 (NbStar8)** 
GAGGTGCAGCTGGTGGAGTCTGGGGGAGGATTGGTGCAGGCTGGGGGCTCTCTGAGACTCT
CCTGTGCAGTCTCTGGAGGCACGTTCAATAACTTTGCCATGGGCTGGTTCCGCCAGGCTCCA
GGGGAGGAGCGTGAGTTTGTTGCAGCTATTACCTGGGGTTCTGGCACACTCTATGCAGACTC
CGTGAAGGGCCGATTCATCCTCTCCAGAAACAACGCCAAAAACACGGCGTATCTGCAAATGA
ACAGCCTGAAACCTGAGGACACGGCCGTTTATTACTGTGCAGGAAAGTATTTCACGGTTGACA
GACTACCAACCGAGTACAAGTACTGGGGCCAGGGGACCCAGGTCACCGTCTCCTCA
**SEQ ID No. 49 (NbStar8)**
   Translation 122 a.a. MW=13297.32 
EVQLVESGGGLVQAGGSLRLSCAVSGGTFNNFAMGWFRQAPGEEREFVAAITWGSGTLYADSV
KGRFILSRNNAKNTAYLQMNSLKPEDTAVYYCAGKYFTVDRLPTEYKYWGQGTQVTVSS
**SEQ ID No. 50**
   EVQLVESGGGLVQAGGSLRLSCAVSG
**SEQ ID No. 51**
   GTFNNF
**SEQ ID No. 52**
   MAWFRQAPGKEREFVASV
**SEQ ID No. 53**
   TWGSGT
**SEQ ID No. 54**
   LYADSVKGRFILSRNNAKNTAYLQMNSLKPEDTAVYYCAG
**SEQ ID No. 55**
   KYFTVDRLPTEYKY
**SEQ ID No. 56**
   WGQGTQVTVSS
**SEQ ID No. 57 (NbStar11)** 
GAGGTGCAGCTGCTGGAGTCTGGGGGAGGATTGGTGCAGGCTGGGGGCTCTCTGAGACTCT
CCTGTGCAGCCTCTGGACGTACTTCTACATTCTTTACCATGGCCTGGTTCCGCCAGGCTCCAG
GGAAGGAGCGTGAGTTTGTAGCAGCGGTTGGGTGGAGTGGTGGTAGTACATTATATGGTGAC
TCCGTGAAGGGCCGAAGCACCATCTCCAGAGACAACGCCAAGAACACACTGTATTTGCAAGT
GGACAGGCTGAAAGTTGAGGACACGGCCGATTATTACTGCGCAAGTAGGAGGGGCGTCAGT
 
ATTTCGCGGTTGGTCAGTGAGTATGACTACTGGGGCCAGGGGACCCAGGTCACCGTCTCCTC
A
**SEQ ID No. 58 (NbStar11)**
   Translation 124 a.a. MW=13434.34 
EVQLLESGGGLVQAGGSLRLSCAASGRTSTFFTMAWFRQAPGKEREFVAAVGWSGGSTLYGDS
VKGRSTISRDNAKNTLYLQVDRLKVEDTADYYCASRRGVSISRLVSEYDYWGQGTQVTVSS
**SEQ ID No. 59**
   EVQLLESGGGLVQAGGSLRLSCAASG
**SEQ ID No. 60**
   RTSTFF
**SEQ ID No. 61**
   TMAWFRQAPGKEREFVAAV
**SEQ ID No. 62**
   GWSGGS
**SEQ ID No. 63**
   TLYGDSVKGRSTISRDNAKNTLYLQVDRLKVEDTADYYCAS
**SEQ ID No. 64**
   RRGVSISRLVSEYDY
**SEQ ID No. 65**
   WGQGTQVTVSS
**SEQ ID No. 66 (NbStar15)** 
GAGGTGCAGCTGCTGGAGTCTGGGGGAGGATTGGTGCAGGCTGGGGGCTCTCTGAGACTCT
CCTGTGTAGCCTCTGAACGCACCGCCTCTGGACGCGCCGCCTATGTCATGGGCTGGTTCCG
CCAGGCCCCGGGGAAGGAGCGTGAGTTTGTGTCAGCCATTAGGTGGAGTGACGATAGTACA
TCGTATGCAGACTCCGTGAAGGGCAGATTCACCGTCTCCAGAGACAATGCCAGGAACATGGT
GTACCTGCAAATGAATACTGTGAAACCTGAGGACACGGCCAATTATTACTGTGCAGCTCGCTT
TCGATTTACTATAACTCCTATAACCAGTTCGCGTGACTATGACTACTGGGGCCAGGGGACCCA
GGTCACCGTCTCCTCAGAACCCAAGACACCAAAACCACAAACT
**SEQ ID No. 67 (NbStar15)**
   Translation 138 a.a. MW=15324.550000000012 
EVQLLESGGGLVQAGGSLRLSCVASERTASGRAAYVMGWFRQAPGKEREFVSAIRWSDDSTSY
ADSVKGRFTVSRDNARNMVYLQMNTVKPEDTANYYCAARFRFTITPITSSRDYDYWGQGTQVTV
SSEPKTPKPQT
**SEQ ID No. 68 (NbStar17)** 
GAGGTGCAGCTGGAGGAGTCTGGGGGAGGATTGGTGCAGGCTGGGGGCTCTCTGAGACTCT
CCTGTGTAGTCTCTGGAGGCGCGTTCAATAACTTTGCCATGGGCTGGTTCCGCCAGGCTCCA
GGGAAGGAGCGTGAGTTTGTTGCAGGCATGAGCTGGAGTGGCAGCACACTCTATGCAGACT
CCGTGAAGGGCCGATTCACCCTCTCCAGAGACAACCCCAAAAACACGGCGTATCTGCAAATG
GACAGTCTGAAACCTGAGGACACGGCCGTTTATTTCTGTGCAGGAAAGTATTTCACGACGGC
CCGACTACCGGCGGAGTATAAGTACTGGGGTCAGGGGACCCAGGTCACCGTCTCCTCA
**SEQ ID No. 69 (NbStar17)**
   Translation 122 a.a. MW=13272.26 
EVQLEESGGGLVQAGGSLRLSCVVSGGAFNNFAMGWFRQAPGKEREFVAGMSWSGSTLYADS
VKGRFTLSRDNPKNTAYLQMDSLKPEDTAVYFCAGKYFTTARLPAEYKYWGQGTQVTVSS
**SEQ ID No. 70**
   EVQLEESGGGLVQAGGSLRLSCVVSG
**SEQ ID No. 71**
   GAFNNFA
**SEQ ID No. 72**
   MGWFRQAPGKEREFVAGM
**SEQ ID No. 73**
   SWSGST
**SEQ ID No. 74**
   LYADSVKGRFTLSRDN PKNTAYLQM DSLKPEDTAVYFCA
**SEQ ID No. 75**
   KYFTTARLPAEYKY
**SEQ ID No. 76**
   WGQGTQVTVSS
**SEQ ID No. 77 (CD38)**

MANCEFSPVSGDKPCCRLSRRAQLCLGVSILVLILVVVLAVVVPRWRQQWSGPGTTKRFPETVLA
RCVKYTEIHPEMRHVDCQSVWDAFKGAFISKHPCNITEEDYQPLMKLGTQTVPCNKILLWSRIKDL
AHQFTQVQRDMFTLEDTLLGYLADDLTWCGEFNTSKINYQSCPDWRKDCSNNPVSVFWKTVSR
RFAEAACDVVHVMLNGSRSKIFDKNSTFGSVEVHNLQPEKVQTLEAWVIHGGREDSRDLCQDPTI
KELESIISKRNIQFSCKNIYRPDKFLQCVKNPEDSSCTSEI

### Examples

The antibodies and fragments of this disclosure were found using an unconventional screening method. The following examples show how this was achieved. The examples serve to illustrate the disclosure and work of the inventors, the scope of this disclosure is not limited to the examples.

### Example 1: Immunoprecipitation (IP) Using Candidate Nanobodies.

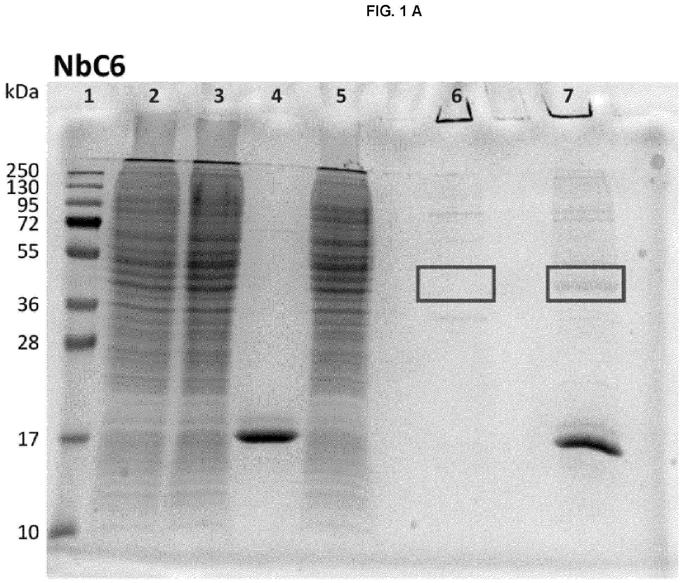

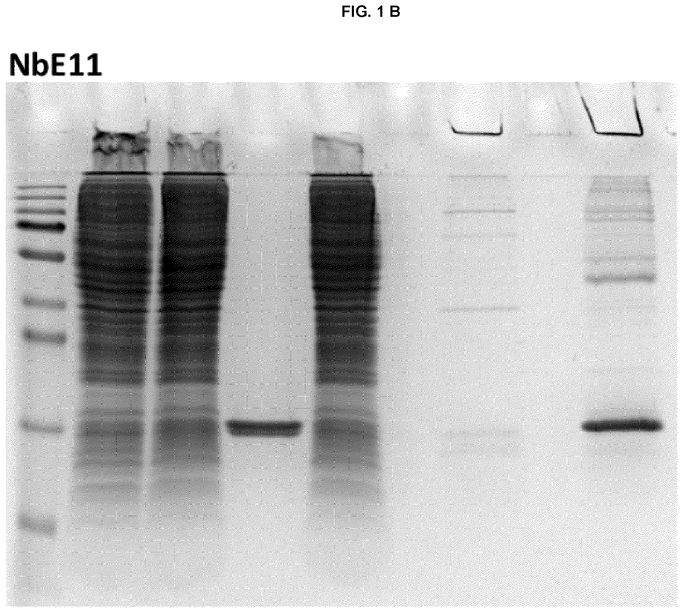

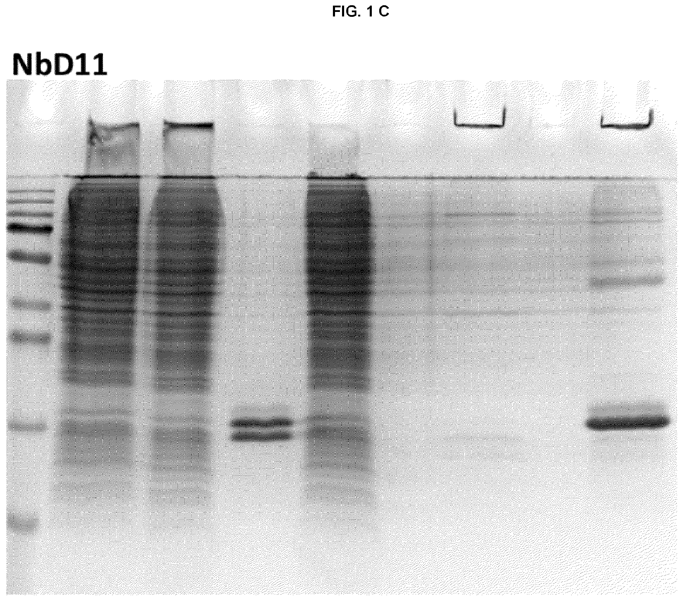

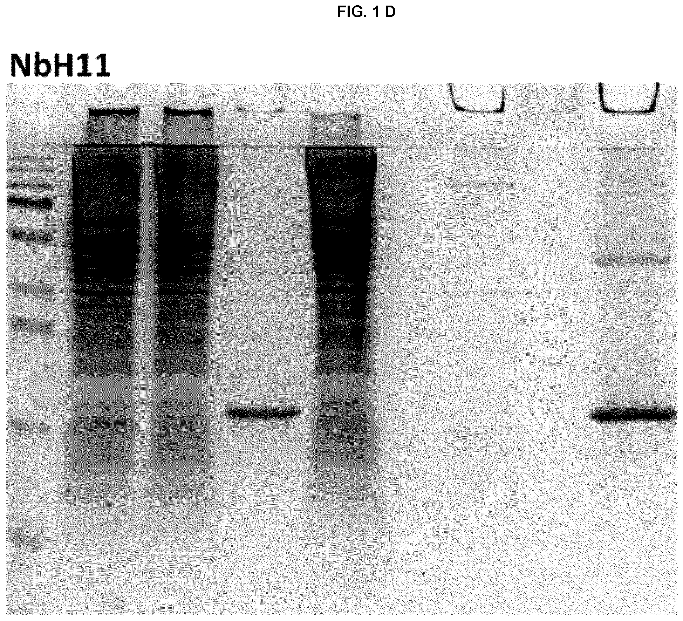

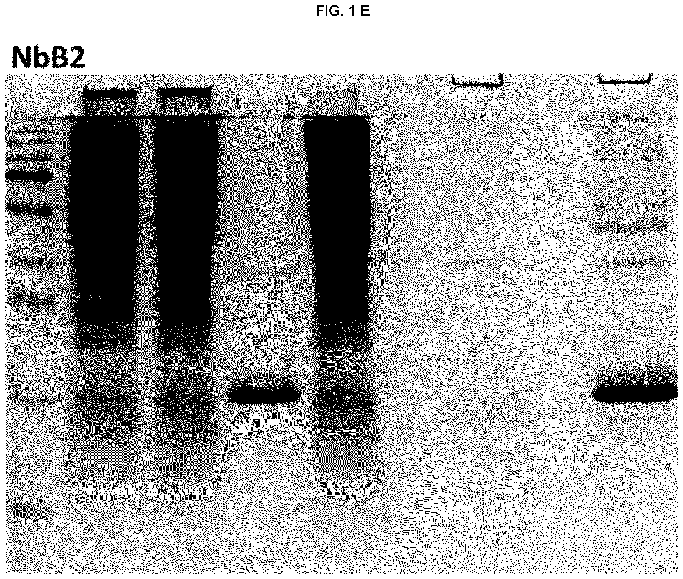

A single-domain antibody library was generated by immunization of alpacas with B-cell receptors. Reversed Phage-Display using live B-cells was employed to select single-domain antibodies binding surface proteins. Each candidate single-domain antibody was tagged with an ALFA-tag and immobilized on ALFA-beads. B-cell lysate was then used to pull down the single-domain antibody's target protein, followed by gel electrophoresis, as exemplified in **Figure 1** **A - E.** Highlighted candidate bands on the gels in **Figure 1** **A** and the corresponding ones in **Figures 1** **B to E** were then sent for Mass Spectrometry (MS) analysis. MS identified CD38 as the molecular target of the single-domain antibodies.

Without wishing to be bound by this theory, the inventors hypothesize that using the methods disclosed herein allows for the generation of a potentially unlimited number of further antibodies with the advantageous properties discussed in this disclosure.

**Figure 1** **A to E** shows the SDS-PAGE results of RAMOS lysate using candidate single-domain antibodies bearing an ALFA tag and loaded in ALFA-Selector magnetic resin. After several washing steps, single-domain candidates and proteins were eluted and run in a denaturing gel. The wells for all gels are respectively ladder (1), cell lysate input (2), flowthrough 1 (clean-lysate) (3), single-domain antibody input (4), flowthrough 2 from clean-lysate with resins loaded with candidate single-domain antibodies (5), background control IP (6), denaturation elution from the beads (7). The most prominent band (example in rectangle), not present in the control IP (no candidate single-domain antibody) or the candidate single-domain antibody lane, was cut and used for Mass Spectrometry (MS) analysis.

The lanes of Figure 1 B - E are numbered as in Figure 1 A. The ladder in Figure 1 B - E has fragments of the same kDa value as the ladder in Figure 1A.

MS analysis identified without a doubt that the protein in the gel band corresponds to CD38. The example for NbC6 shows 75 peptides derived from the band in PAGE, matching human CD38.

### Example 2: Validation of Single-Domain Antibodies Binding Human CD38

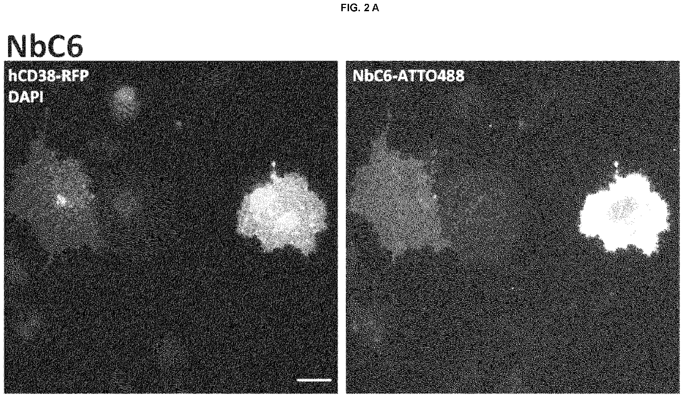

**Figures 2 A-E** show the validation of single-domain antibodies binding to human CD38 in transfected Cos-7 cells.

Cos-7 cells were transfected to express surface hCD38 tagged with RFP on their cytoplasmatic side (hCD38-RFP). Live-cell staining was performed with the single-domain antibody candidates conjugated with fluorophores (NbX-Atto488), followed by fixation with PFA, mounting, and imaging using an epifluorescence microscope. DAPI has been used for nuclei staining. The scale bars represent 20 µm.

**In** **Figures 2 A-E****,** first, the correct expression of human CD38 (fused to RFP) was controlled by expressing it in a cell line that does not express endogenously CD38. The exogenous expression of hCD38 is fused to RFP (hCD38-RFP). Subsequently, the cells were stained with different single-domain antibody candidates, which were conjugated with a Atto488.

**Figure 2 A-E** confirm the correct exogeneous expression of hCD38-RFP in a cell line that does not express CD38. The cells which did not possess the gene for CD38 expression after the transfection (only nuclei stained with DAPI) were not recognized by the fluorescently labeled single-domain antibodies. On the contrary, only the cells expressing CD38 after the transfection are recognized by the fluorescently labeled single-domain antibody candidates, gaining a green staining.

It is evident that only transfected cells emitting red light signals (expressing hCD38) are stained with green color upon staining with fluorescently labeled single-domain antibody candidates, leaving all other cells unmarked that do not express hCD38.

The results indicate effective binding of the single-domain antibody candidates to hCD38, as well as specific binding of the single-domain antibody candidates to CD38.

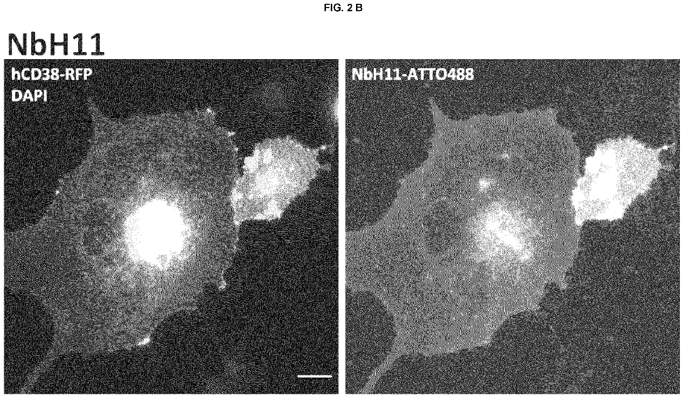

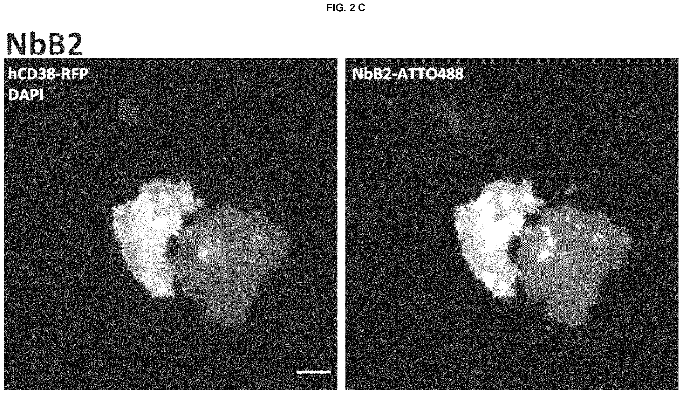

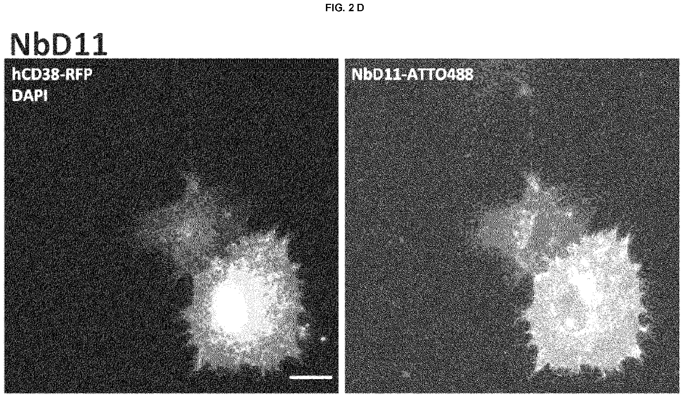

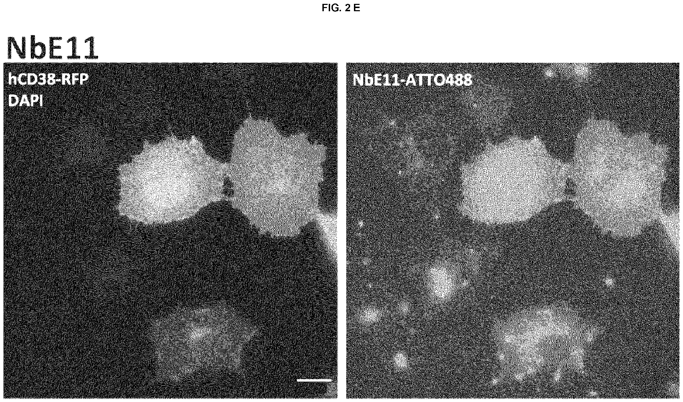

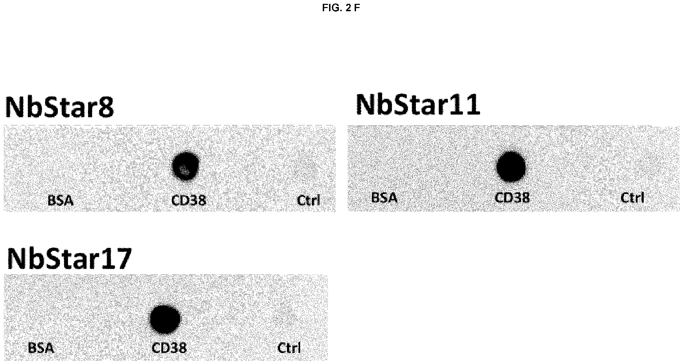

**Figure 2** **F** shows a dot-blot assay to validate binding to hCD38 of other single-domain antibody candidates. Human recombinant CD38, BSA, and purified hCD45 proteins were spotted on nitrocellulose membranes. After a blocking step using 5% BSA in PBS, candidate single-domain antibodies conjugated to a fluorophore (ATTO643) were incubated for one hour. After thorough washing, membranes were imaged. The signal was only present on spotted purified human CD38.

### Example 3: Specificity Checks in Live Cells

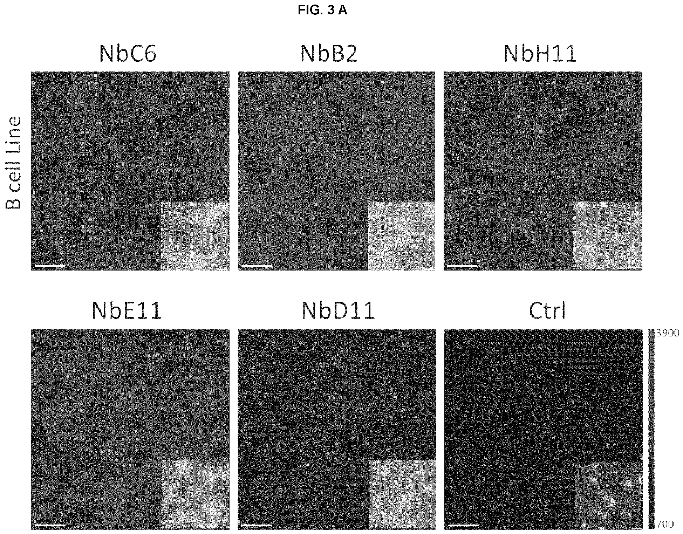

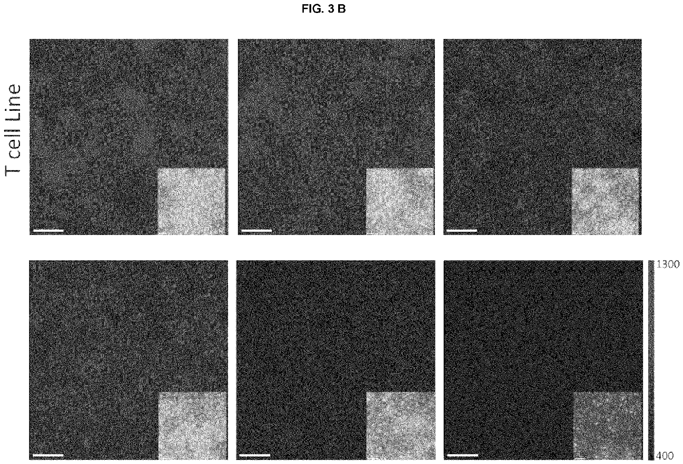

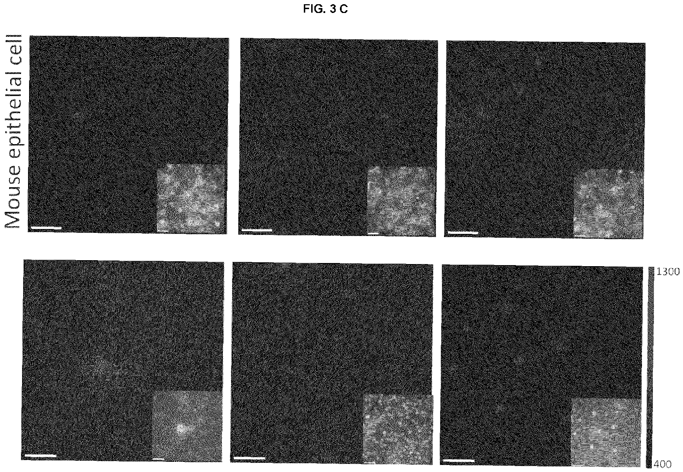

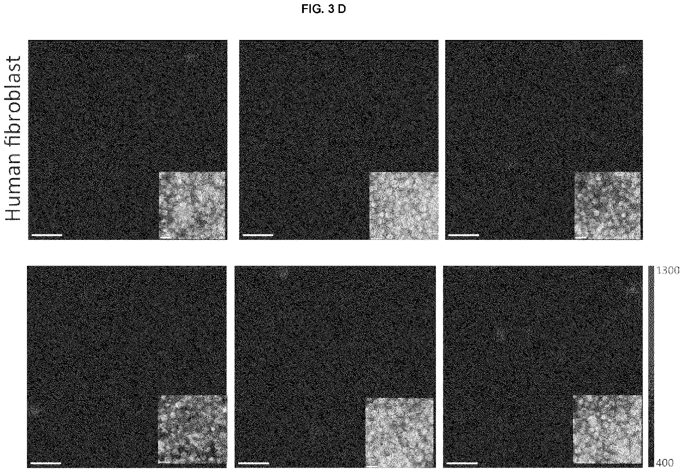

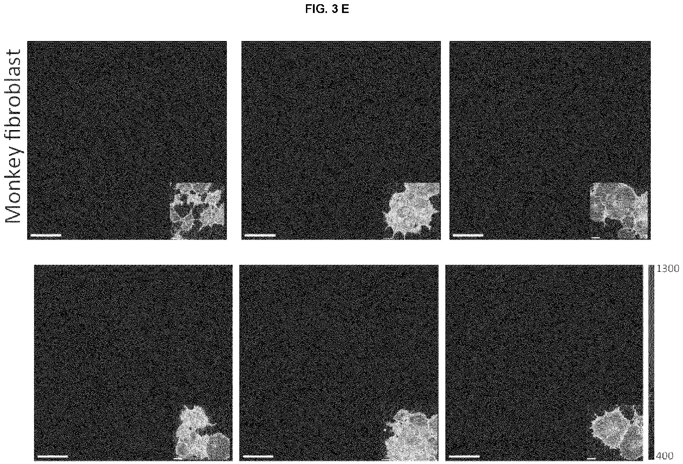

Live staining was performed using single-domain antibody candidates conjugated to ATTO643 on human, mouse, and monkey cell lines, for which some are known to express CD38 (B and T cells), while other fibroblasts from different species are not supposed to have high levels of CD38 or do not express CD38 at all. After live staining, cells were washed and immediately imaged live. The results are presented in **Figure 3 A-E.** Rows are equally scaled to allow direct comparison of the binding strength between different single-domain antibody candidates. The autofluorescence (insets) was used to confirm the presence of cells across all conditions. The inset image (bottom right of each image) represents the cells' autofluorescence to demonstrate the cells' presence, especially when black images are obtained from the single-domain antibodies. The scale bars represent 30 µm. In Figures 3 B-E, the single-domain antibody candidates are the same and in the same order as in Figure 3 A: NbC6 (first picture on the first row), NbB2 (second picture on the first row), NbH11 (third picture on the first row), NbE11 (first picture on the second row), NbD11 (second picture on the second row), Ctrl (third picture on the second row).

The results demonstrate the specificity of the single-domain antibody candidates, which bind specifically to the human B-cell line and to the human T-cell line and do not bind to mouse epithelial cells, nor to human fibroblasts or monkey fibroblasts.

Furthermore, NbALFA was used as a control, which binds to an artificial epitope with the same fluorophore and did not generate color signal in any cell line. This confirms that the signal on B and T cells is not an implicit feature of the single-domain antibody type, but a strict consequence of the specific binding of the single-domain antibody candidates of the present disclosure to CD38.

### Example 4: Thermostability Analysis of Single-Domain Antibodies

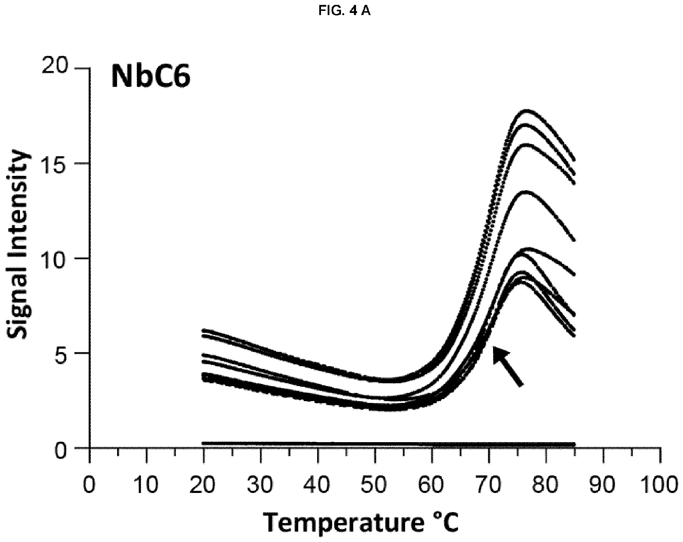

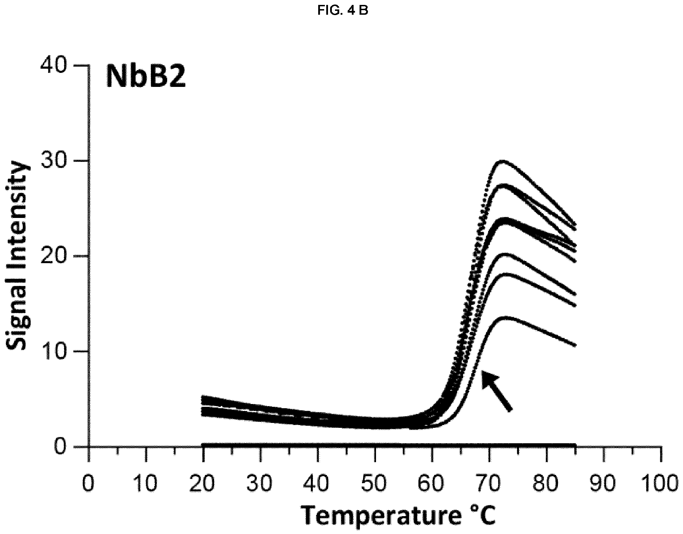

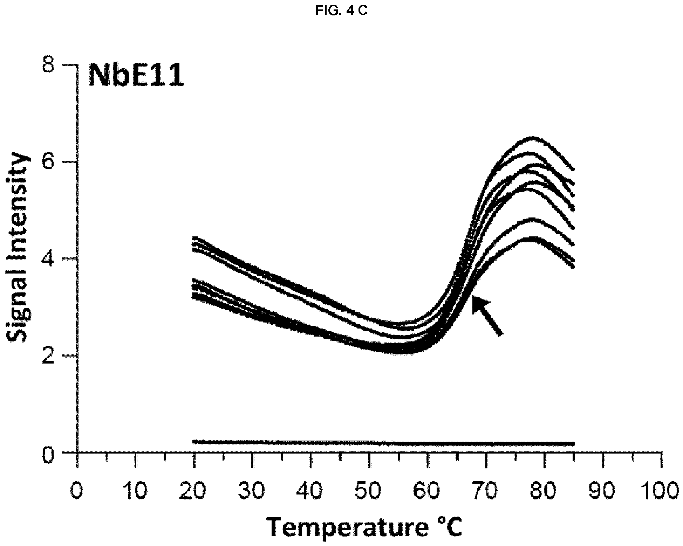

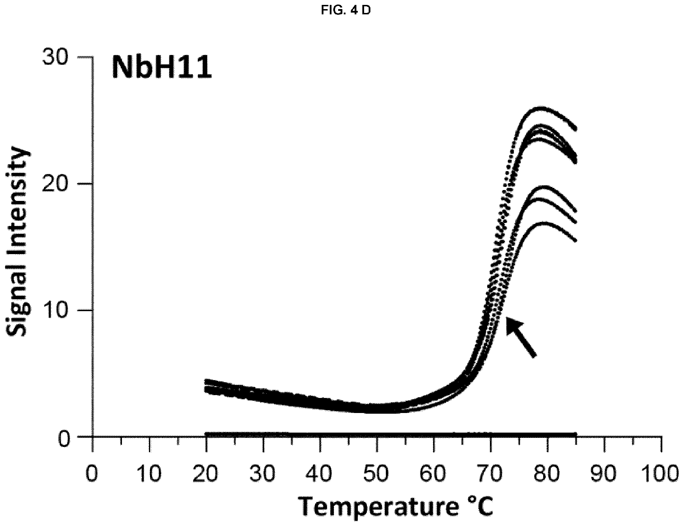

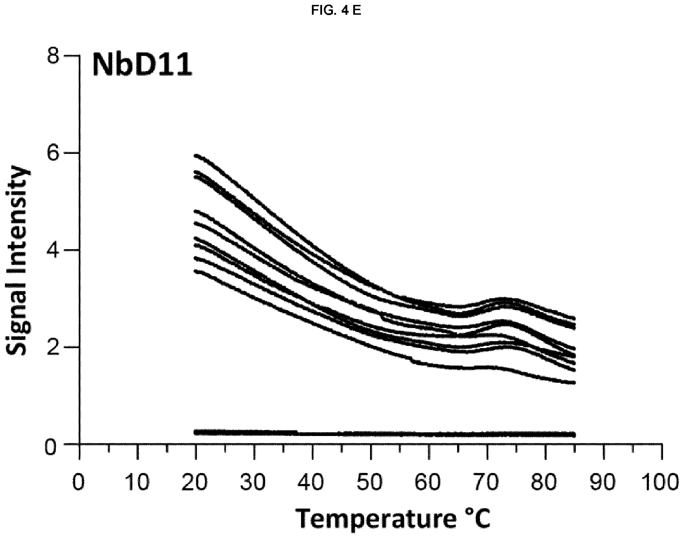

The thermostability of nanobodies was measured using a fluorescence-based thermal shift assay (Thermo Fisher, Cat.4461146). The results are presented in **Figures 4** **A** - **E.** As temperature increases, single-domain antibody unfolding exposes hydrophobic regions to which the dye binds, leading to an increased fluorescence signal. Four single-domain antibodies (C6, B2, H11, and E11) demonstrated high stability, with melting temperatures (Tₘ) at approximately 70°C (see arrows), indicating robust thermal resistance and scaffold stability.

Single-domain antibodies are more thermally stable than traditional antibodies. Except for the candidate NbD11, all single-domain antibody candidates start denaturating at a temperature of at least 65°C, displaying the structural stability of the molecules.

Example 5: Affinity Measurement of Single-Domain Antibody Candidates

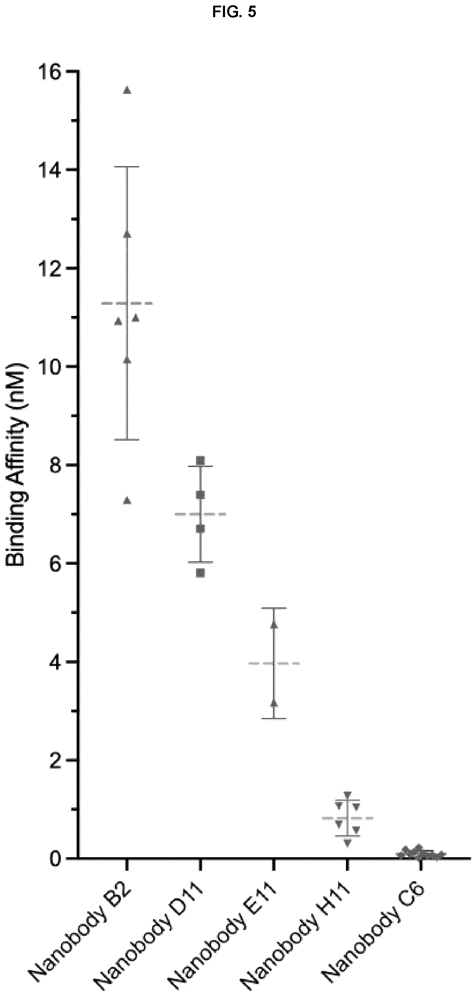

The affinity of single-domain antibodies was measured using Microscale Thermophoresis (MONOLITH, NanoTemper - measurements were conducted according to the manufacturer's instructions). For this, fluorescently labeled single-domain antibodies (conjugated to ATTO643) were combined at ramp concentration with purified human CD38. The results are presented in **Figure 5****.** Box-plot Data represents mean ± SD. Each dot represents an independent measurement.

All single-domain antibodies exhibited low nanomolar affinities, with the candidate NbC6 showing an outstanding binding affinity (K_{D}) of 41.45 pM. The results underline the significant superiority of the candidate NbC6 regarding affinity strength.

### Example 6: Comparative affinity measurements of single-domain antibodies and therapeutic antibodies binding to human CD38

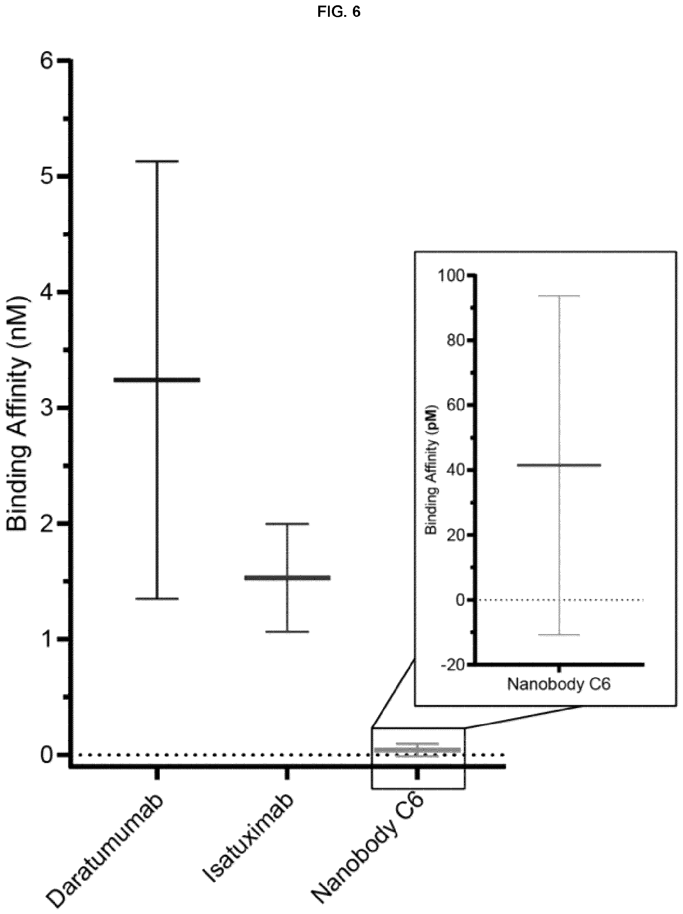

The affinities of the single-domain antibody candidate NbC6, of daratumumab, and of isatuximab were assessed using purified human CD38 protein in a consistent experimental setup. The results are presented in **Figure 6****.** Monovalent NbC6 exhibited an outstanding affinity of 41.45±52 pM, significantly surpassing that of daratumumab (3.24±1.9 nM) and that of isatuximab (1.53±0.47 nM). Data is represented as mean ± SD of 6, 7, and 8 independent measurements, respectively.

For the affinity measurements, the same technology as described in **Example 5** (Microscale Thermophoresis) was used to avoid comparison with literature values measured by using a different method. The obtained affinities obtained for daratumumab and isatuximab were comparable to the reported affinities measured by SPC or Octed (BLI), confirming the validity of the comparison.

### Example 7: FACS Analysis of Single-Domain Antibody Candidates Used to Stain Primary Immune Cells from the Blood of Two Different Donors.

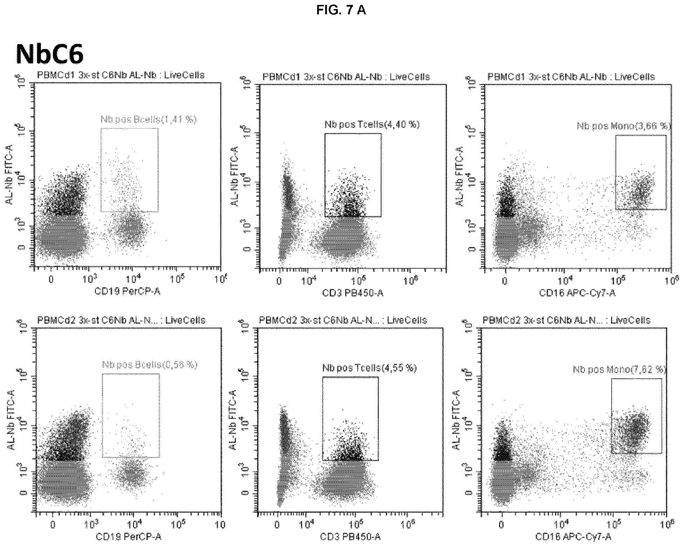

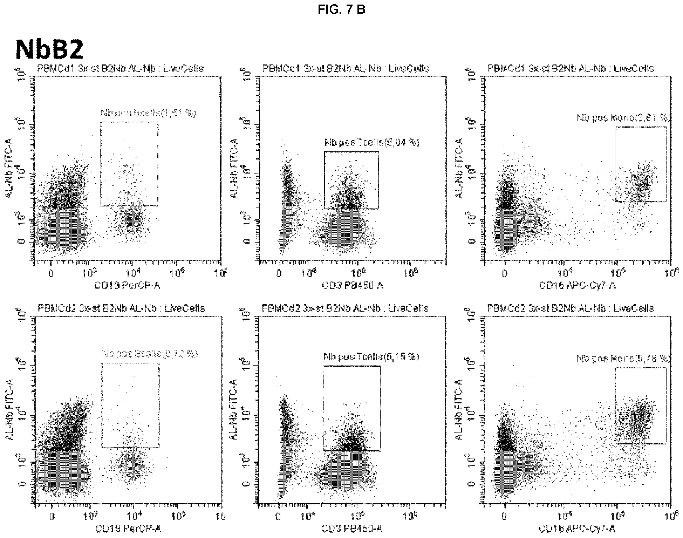

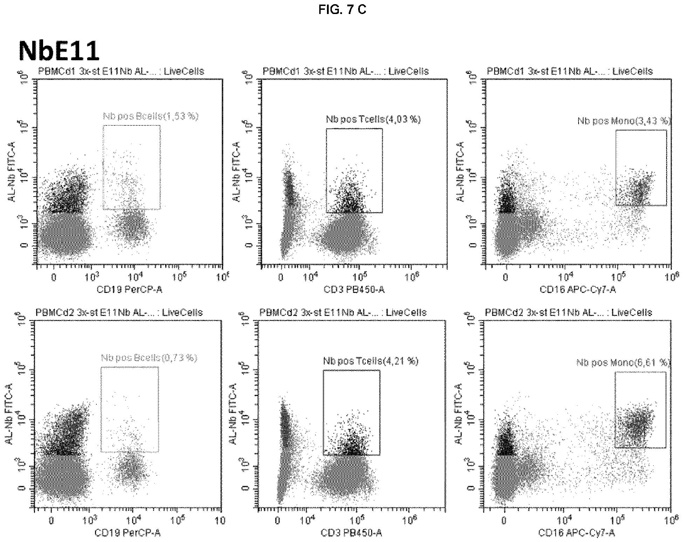

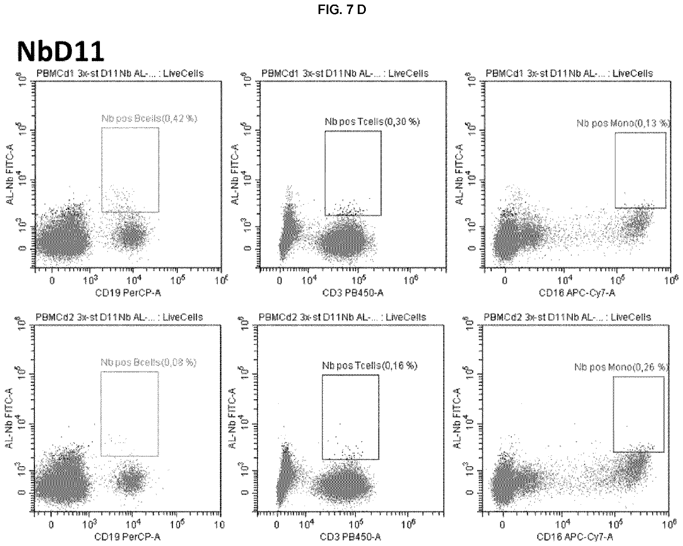

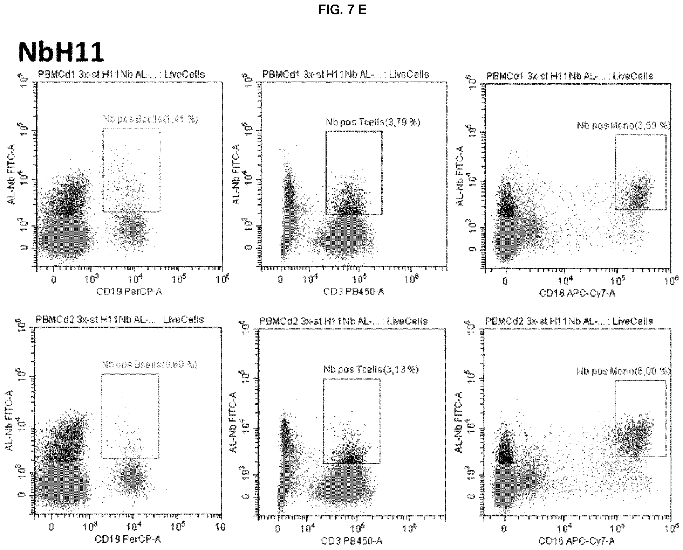

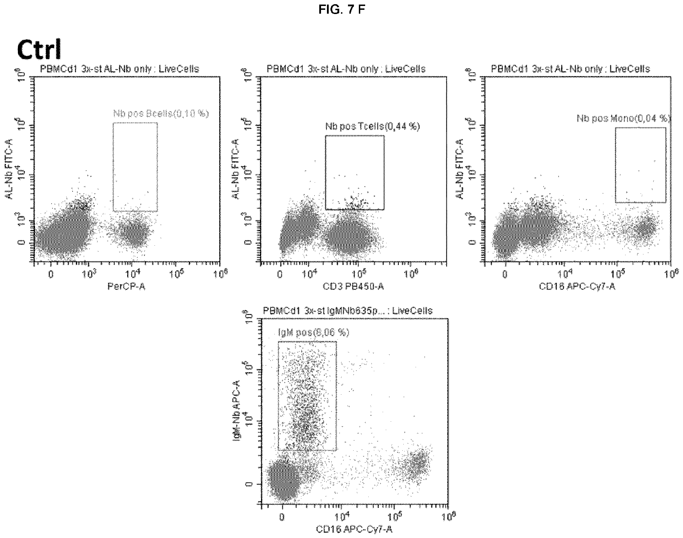

In **Figures 7** **A** - **E,** immune cells from blood samples of two human individuals were stained with different single-domain antibody candidates according to the present disclosure to assess their specificity and ability to bind native immune cells such as B-cells (box on left-panel for each patient/human), T-cells (box on mid-panel for each patient), and monocytes (box on right-panel for each patient/human) in Y-axis showing fluorescence intensity representing their binding. In Figures 7 A - E, the first row represents the analysis of the samples from first blood donor (Human 1) and the second row represents the analysis of the samples from the second blood donor (Human 2).

CD19, CD3, and CD16 were used respectively as markers for B cells, T cells, and monocytes shown in the X-axis with fluorescence intensity representing their binding. The control experiment (Figure 7 F) has been done by having cells not stained with single-domain antibodies and staining with single-domain antibodies against IgM (in brown).

Confirming the expectations, FACS analysis shows that a population of B-cells (anti-CD19), a population of T-cells (anti-CD3), and Monocytes (anti-CD16) are detected using the fluorescently labelled single-domain antibodies according to the present disclosure.

### Example 8: Evaluating the Ability of Single-Domain Antibody Candidates to Block or Affect the Binding of Daratumumab or Isatuximab on CD38

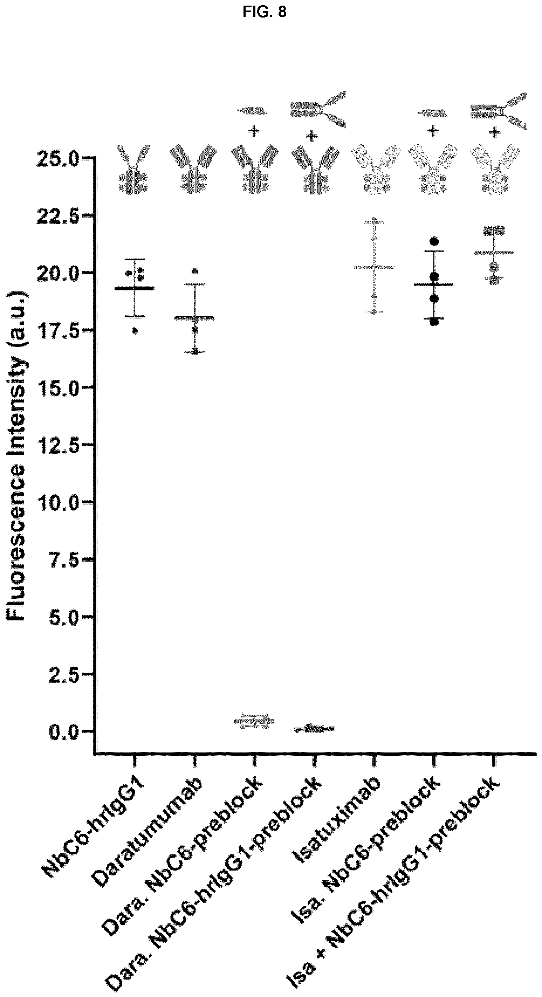

In this experiment, RAMOS cells, a cell line derived from a patient with Burkitt's Lymphoma which overexpresses CD38 on its surface, were stained with fluorescently labeled daratumumab, isatuximab, or NbC6-hrlgG1 (Minibody), either alone or after pre-blocking epitopes using non-fluorescent NbC6 or its recombinant minibody (recombinant Fc-fusion version). Pre-incubation with NbC6 or NbC6-hrlgG1 completely blocked daratumumab binding while not affecting and allowing full binding of isatuximab in the presence of the NbC6 and MinibodyC6 (results shown in **Figure 8****).** This is of significant relevance since it has been shown that daratumumab and isatuximab cannot bind the hCD38 at the same time (Lee et al., 2021, DOI: 10.1016/j.bbrc.2020.12.048).

The assay demonstrated that upon pre-labelling CD38 with non-fluorescent NbC6 or the minibody C6, subsequently added daratumumab can no longer bind CD38, while the binding of isatuximab to CD38 did not seem to be affected.

This suggests that isatuximab and the minibody C6, or isatuximab and NbC6 were able to bind simultaneously to the identical CD38 molecule on the cell surface of a human cell.

Notably, the minibody C6 demonstrated a better efficiency in blocking the daratumumab epitope. This was consistent with the expectations, as the minibody exhibited a higher avidity due to its bivalent nature.

### Example 9: Measuring Cyclase Activity of Purified CD38

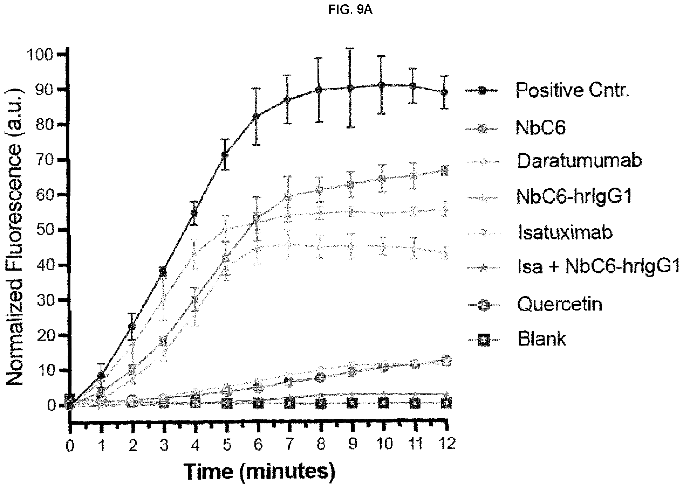

**Figure 9A** shows the cyclase enzymatic activity followed in time. A positive control is CD38 without any binding molecule, allowing cyclase activity to proceed. Other samples of hCD38 were preincubated with equal saturating amounts (100nM) of Single-Domain Antibody C6 (NbC6), NbC6-hrlgG1 (Minibody), daratumumab, isatuximab, or a combination of isatuximab and NbC6-hrlgG1. Finally, a known cyclase inhibitor, Quercetin (inhibition positive control), was used, and a blank condition where no enzyme (CD38) was present.

The enzymatic activity of CD38 was measured using a fluorescent substrate assay (Abcam ab284540). Untreated CD38 or CD38 treated with an irrelevant single-domain antibody showed the highest enzymatic activity, indicated by the steepest slope. Pre-incubation with daratumumab reduced the enzymatic activity, while isatuximab, used as a control, completely blocked the activity, as expected. Showing an enhanced inhibitory potential, the minibody C6 decreased CD38 activity more effectively than daratumumab.

Notably, the minibody C6 is more efficient than NbC6 in impairing the cyclase activity of CD38, favouring the affinity/avidity effect.

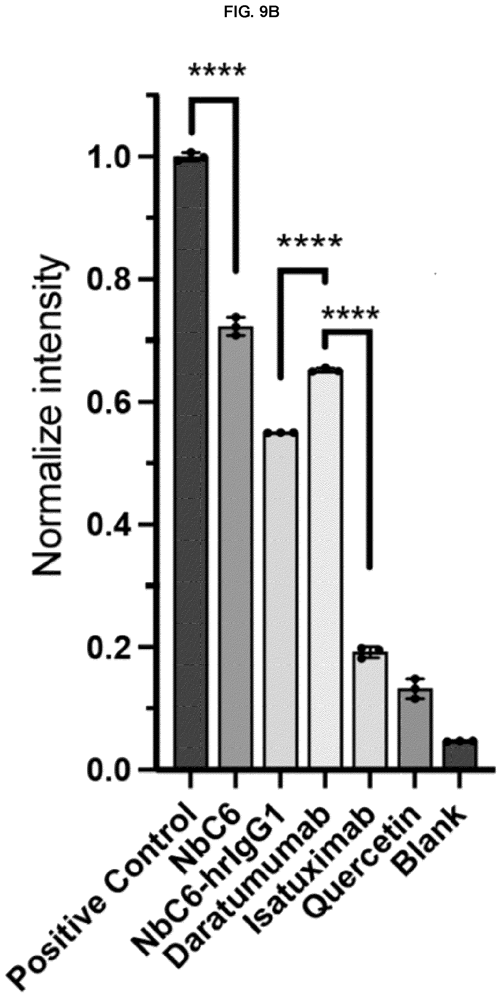

**Figure 9B** shows the data for all conditions at 8 minutes when the positive control reaches a plateau. Error bars represent the SD of 3 independent experiments. Note that NbC6-hrlgG1 inhibits cyclase activity significantly better than daratumumab. isatuximab inhibits ~80% of the cyclase activity, and NbC6-hrlgG1 simultaneously inhibits ~50%. One-Way ANOVA; **** = p< 0.0001.

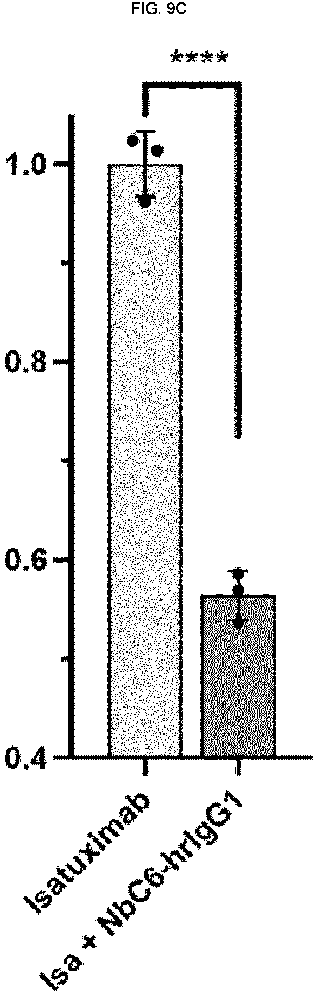

**Figure 9C** shows that combinatorial inhibition of isatuximab and NbC6-hrlgG1 results in a ~40% inhibition increase compared to the inhibition of isatuximab alone (the y-axis shows the normalized intensity). Thus, the combination of isatuximab and the minibodies of the present disclosure exhibits a strong synergistic effect.

### Example 10: Measuring Hydrolase activity of purified CD38

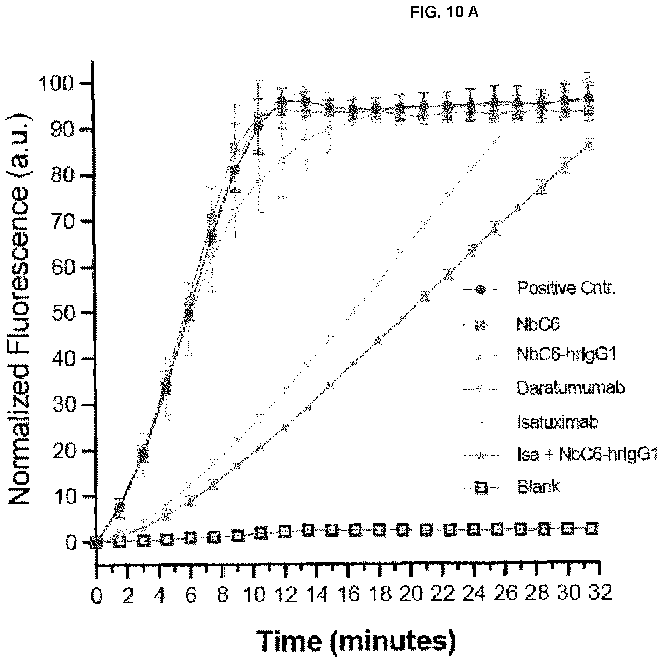

**Figure 10A** shows the hydrolase activity of purified CD38 in time. The positive control is CD38, which, without any binding molecule, is allowed to perform its hydrolase activity in the presence of the equal saturating amounts (100nM) of Nanobody C6 (NbC6), MinibodyC6 (NbC6-hrlgG1), Daratumumab, Isatuximab, combination of Isatuximab and NbC6-hrlgG1, or without CD38 (Blank).

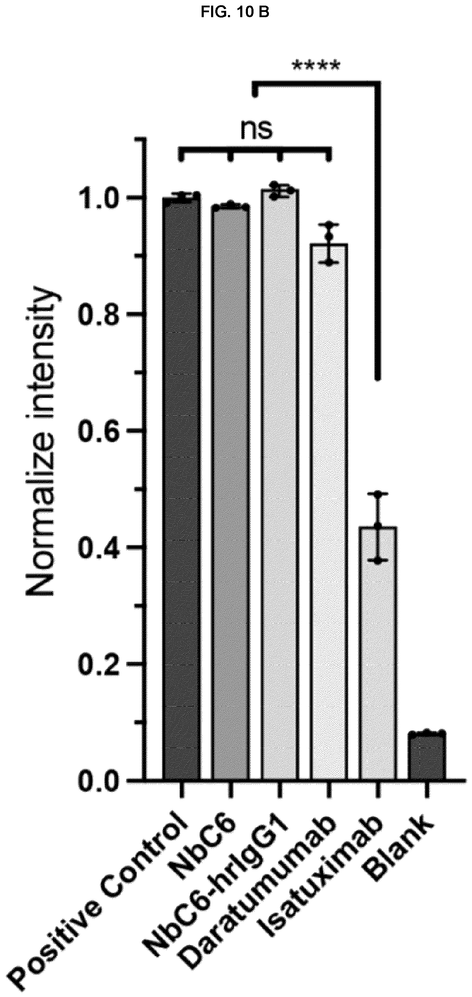

**Figure 10B** shows the data for all conditions at 12 minutes when the positive control reaches a plateau in **Figure 10A****.** Error bars represent the SD of 3 independent experiments. Note that only isatuximab can inhibit/slowdown the hydrolase activity of CD38. One-Way ANOVA; **** = p< 0.0001.

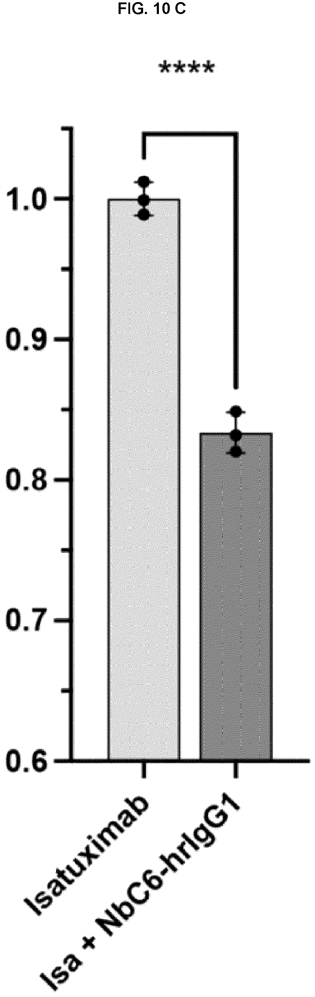

**Figure 10C** shows that combinatorial inhibition of Isatuximab and NbC6-hrlgG1 results in almost a 20% increase in inhibition compared to Isatuximab alone (the y-axis shows the normalized intensity). Thus, the combination of isatuximab and the minibodies of the present disclosure exhibits a strong synergistic effect.

### Example 11: Complement-Dependent Cytotoxicity (CDC) Assay using RAMOS cells - Comparison between Minibody C6 and Daratumumab

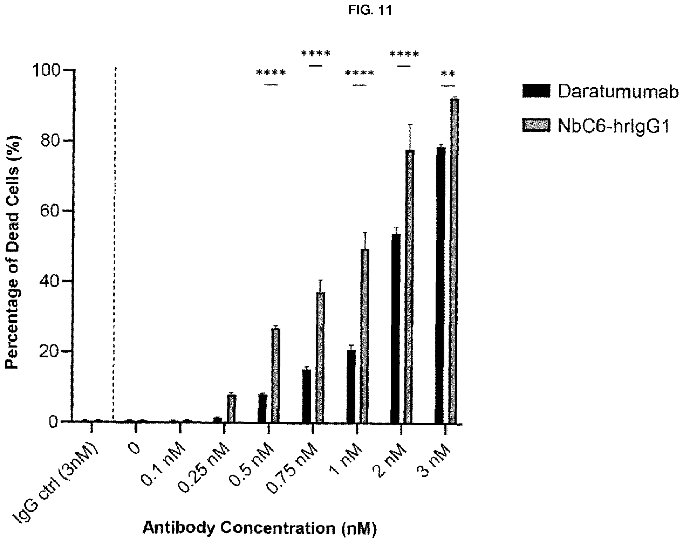

The minibody candidate hlgG1-NbC6, having a human IgG bivalent binding character, was challenged against daratumumab in a CDC assay to kill a human lymphoma cell line (CD38+). The results are depicted in **Figure 11****.** Different concentrations of antibodies in the presence of human Serum and B lymphoma cells (RAMOS cell line) have been used. NbC6-hrlgG1 outperforms Daratumumab at low concentrations. Significant differences are observed already at 0.25 nM, while, for example, at 1 nM, the minibody kills more than 50% of the cells and Daratumumab only ~20%. In one-way ANOVA statistical analysis, three independent experiments of each condition were performed. Tukey post-hoc test resulted in p>0.001 = **; p>0.0001 = ****. Cells were incubated for 6 hours at 37°C in 20% human serum. Flow cytometry assessed cell viability, labeling dead cells with a fluorescent dye. Negative control was used with a human IgG not binding to any epitope on these cells but in the presence of human Serum.

The results demonstrate that hlgG1-NbC6 outperformed daratumumab at lower concentrations in inducing CDC. Notably, CDC represents daratumumab's primary mechanism of killing tumour cells.

### Example 12: Complement-Dependent Cytotoxicity (CDC) for Combinatorial Treatment

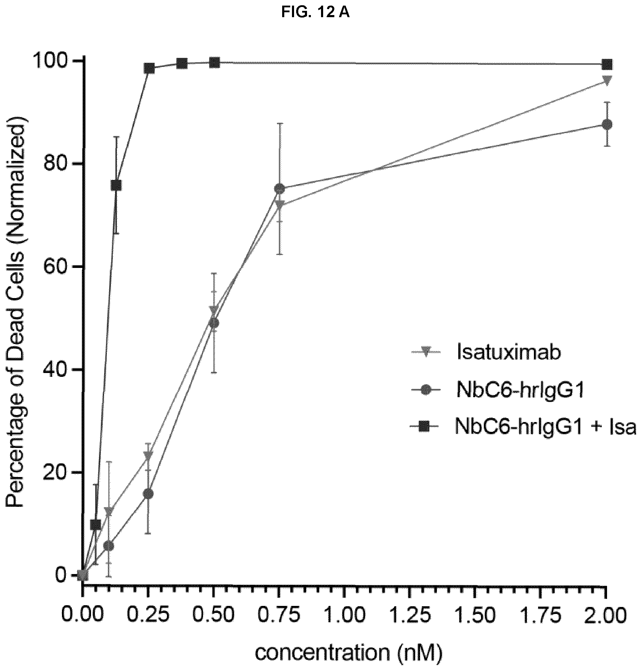

**Figure 12A** shows a Curve displaying the percentage (%) of dead cells (RAMOS; human B lymphoma cells) depending on the antibody concentration in the presence of human serum.

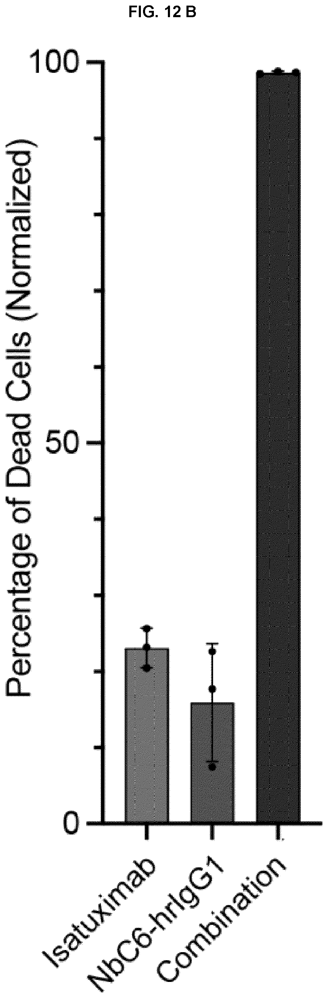

**Figure 12B** shows the killing saturation of combinatorial treatment using Isatuximab and NbC6-hrlgG1 at 0.25 nM (see arrows in Figure 12A) displayed in comparison to death obtained with the other individual therapies (-25%). The bar on the left represents isatuximab, the bar in the middle represents NbC6-hrlgG1, and the bar on the right represents the combinatorial treatment of NbC6-hrlgG1 and isatuximab.

### Example 13: Antibody-Dependent Cellular Cytotoxicity (ADCC) Assay Using RAMOS Cells Stably Expressing Yellow Fluorescent Protein (YFP)

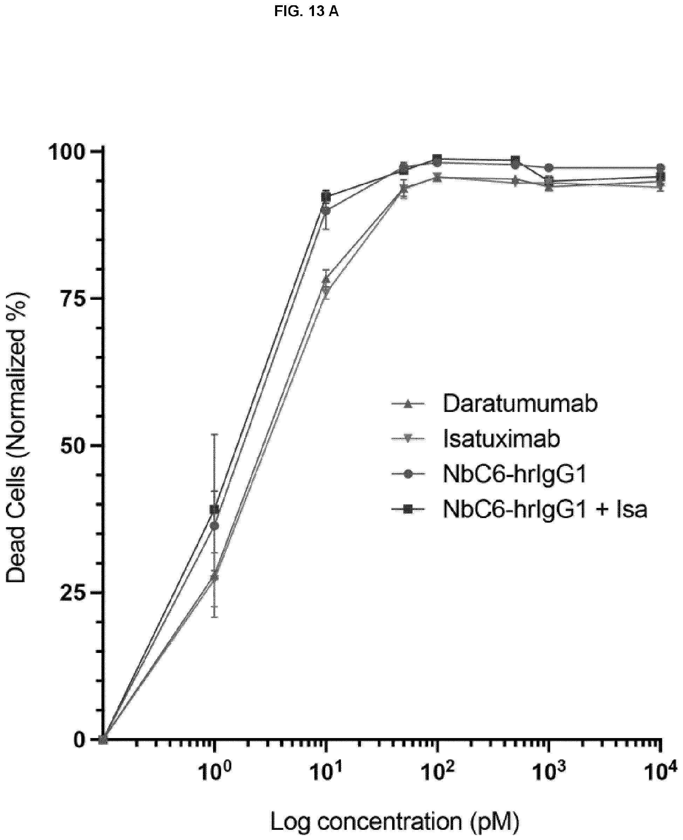

In **Figure 13A****,** RAMOS cells were pre-stained with CellTracker conjugated with PB450 dye and incubated overnight with primary NK cells at an effector-to-target (E:T) ratio of 10:1 in the presence of different antibodies: daratumumab, isatuximab, NbC6-hrlgG1, and a combination of NbC6-hrlgG1 with isatuximab, at varying concentrations. Control conditions included RAMOS cells alone and RAMOS + NK cells without antibodies. Cell death was assessed by flow cytometry, identifying the loss of YFP+ cells that were PB450+ and negative for LIVE/DEAD staining. Data were normalized to the RAMOS + NK condition without antibody.

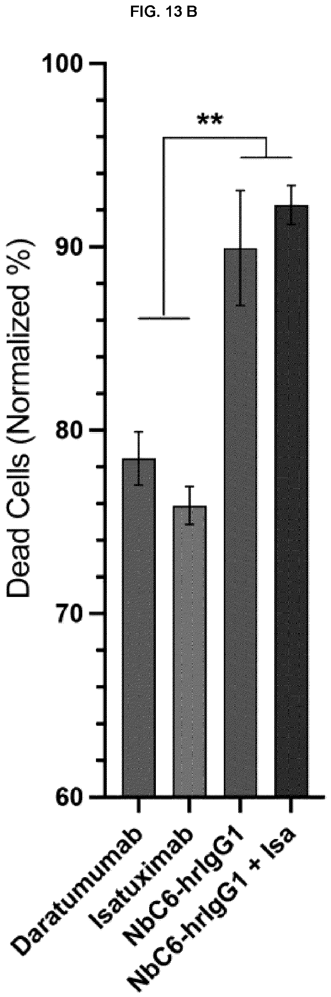

In **Figure 13B****,** detailed statistical analysis was performed to compare the efficacy of different treatments at 10 pM (One-Way ANOVA; ** = p< 0.01). As observed, minibody C6 (NbC6-hrlgG1) demonstrates superior antibody-dependent cytotoxicity (ADCC), especially at low concentrations (sub-50 pM), achieving over 90% cell killing, whereas daratumumab and isatuximab induce approximately 75% cell death at 10 pM.

Notably, the combinatorial treatment of the minibody C6 (NbC6-hrlgG1) and isatuximab achieves the most efficient ADCC values.

### Example 14: Live-cell staining of transfected Cos-7 cells expressing native or glycomutant hCD38

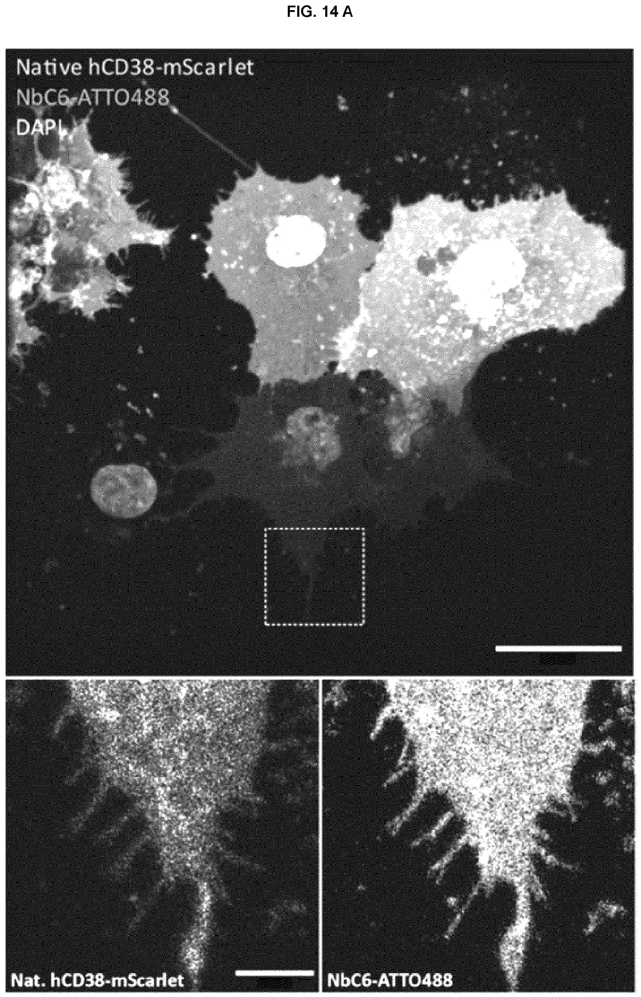

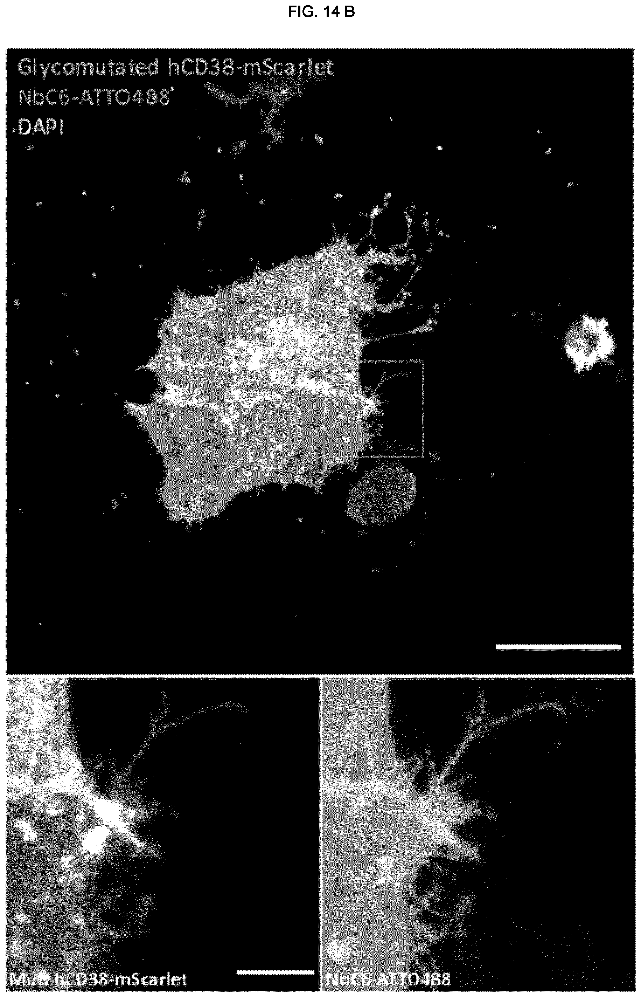

In **Figures 14** **A - B,** transfected Cos-7 cells expressing native full-length hCD38 or glycomutant (N100D, N164D, N209D, N219D) fused to mScarlet on their surface (hCD38-mScarlet) were live-stained with NbC6 conjugated to ATTO488 (NbC6.ATTO488) without permeabilization. Cells were then fixed with PFA, mounted, and imaged using a confocal microscope. Nuclei were stained with DAPI. Scale bars: 30 µm (overview) and 5 µm (zoomed-in regions). The staining confirms that NbC6 binds CD38 independent of glycosylation.

### Example 15: Evaluating the Ability of Single-Domain Antibodies to Block or Affect the Binding of Daratumumab or Isatuximab

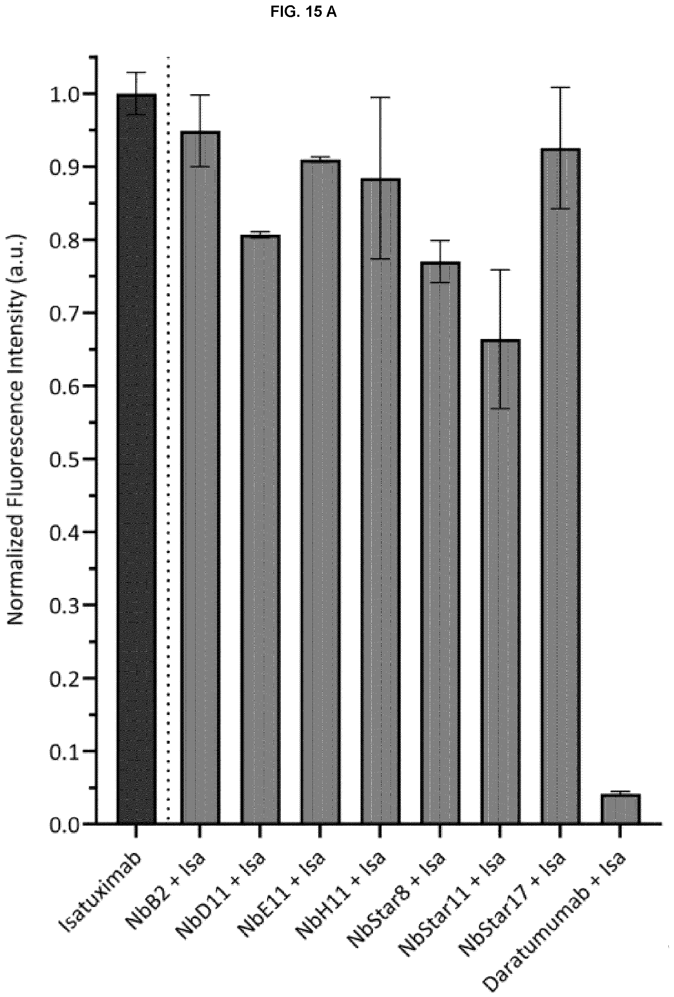

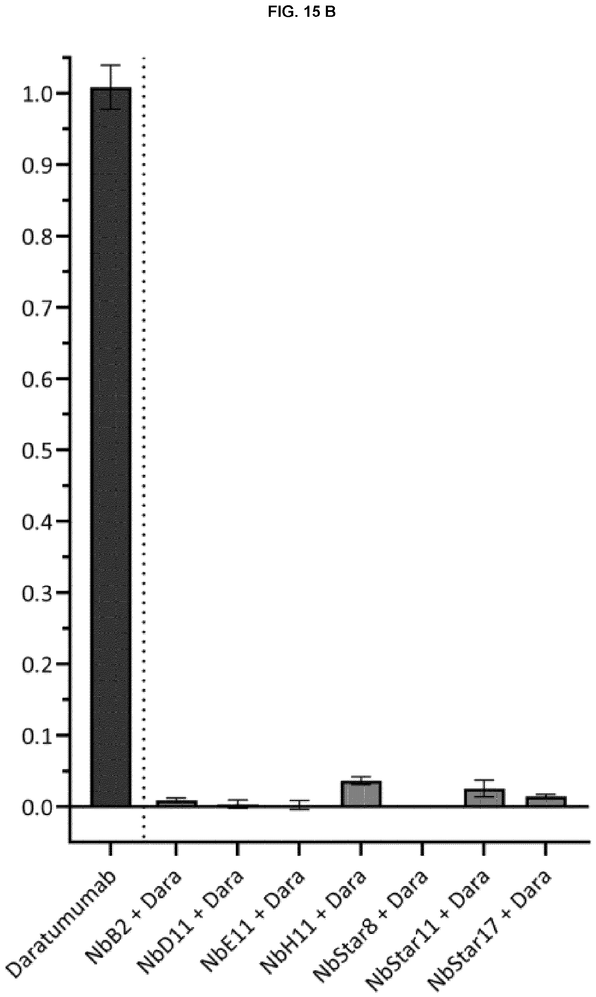

RAMOS cells were stained with fluorescently labelled isatuximab (**Figure 15A**) or daratumumab (**Figure 15B**), conjugated to fluorophore (ATTO643). Cells were pre-blocked using non-fluorescent single-domain antibody candidates. Pre-blocking was carried out by incubating cells with 200 nM of single-domain antibody for 45 minutes at 4°C, followed by adding fluorescently labelled isatuximab or daratumumab. Cells were washed twice with PBS and imaged live using consistent acquisition settings under a fluorescence microscope. The fluorescence intensity was quantified and normalized to the control condition (cells not pre-blocked, black bar). Image analysis was performed using ImageJ. Bars represent the mean of three measurements consisting of approximately 1,000 cells. Errors represent the standard deviation of the three measurements. Note that when daratumumab is used as the pre-blocking agent (**Figure 15A**), it efficiently blocks the binding of isatuximab, as demonstrated previously (Lee et al., 2021, DOI: 10.1016/j.bbrc.2020.12.048).

The results demonstrate that the single-domain antibody candidates of the present disclosure are suitable for use in a combinatorial therapy together with isatuximab, in contrast to the therapeutic antibody daratumumab of the prior art.

The presented method was developed to identify single-domain antibodies that target immune cell-based hematological malignancies, such as lymphomas, leukemias, and specifically Multiple Myeloma.

Alpacas were immunized with total B-cell membrane proteins isolated from a Burkitt's lymphoma cell line (RAMOS), known explicitly to overexpress CD38. Following immunization, a single-domain antibody library was constructed and subjected to live-cell phage display, enabling the simultaneous identification of single-domain antibodies that bound membrane proteins in their native conformations. Then individual single-domain antibody candidates were selected, sequenced and aligned, revealing distinct families, from which representative single-domain antibodies were cloned and produced. Immunoprecipitation using these single-domain antibodies purified the unknown target protein from the cell lysate (highlighted in a rectangle on the gel ), followed by mass spectrometry to identify and validate the target.

## Claims

1. An antibody or fragment thereof binding to CD38, selected from
(i) an antibody or fragment thereof competing with a reference antibody for binding to CD38, and
(ii) the reference antibody, wherein
the reference antibody has the sequence of SEQ ID No. 2.

2. The antibody or fragment thereof according to claim 1, the antibody or fragment thereof comprising
(i) complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8, optionally having framework regions, FR1, FR2, FR3 and FR4, wherein FR1 has a sequence identity of at least 90% with SEQ ID No. 3, FR2 has a sequence identity of at least 90% with SEQ ID No. 5, FR3 has a sequence identity of at least 90% with SEQ ID No. 7, and/or FR4 has a sequence identity of at least 90% with SEQ ID No. 9,
(ii) complementarity-determining regions CDR1, CDR2 and CDR3, wherein CDR1 has an amino acid sequence SEQ ID No. 13, CDR2 has an amino acid sequence SEQ ID No. 15, and CDR3 has an amino acid sequence SEQ ID No. 17, optionally having framework regions, FR1, FR2, FR3 and FR4, wherein FR1 has a sequence identity of at least 90% with SEQ ID No. 12, FR2 has a sequence identity of at least 90% with SEQ ID No. 14, FR3 has a sequence identity of at least 90% with SEQ ID No. 16, and/or FR4 has a sequence identity of at least 90% with SEQ ID No. 18,
(iii) complementarity-determining regions CDR1, CDR2 and CDR3, wherein CDR1 has an amino acid sequence SEQ ID No. 22, CDR2 has an amino acid sequence SEQ ID No. 24, and CDR3 has an amino acid sequence SEQ ID No. 26, optionally having framework regions, FR1, FR2, FR3 and FR4, wherein FR1 has a sequence identity of at least 90% with SEQ ID No. 21, FR2 has a sequence identity of at least 90% with SEQ ID No. 23, FR3 has a sequence identity of at least 90% with SEQ ID No. 25, and/or FR4 has a sequence identity of at least 90% with SEQ ID No. 27,
(iv) complementarity-determining regions CDR1, CDR2 and CDR3, wherein CDR1 has an amino acid sequence SEQ ID No. 31, CDR2 has an amino acid sequence SEQ ID No. 33, and CDR3 has an amino acid sequence SEQ ID No. 35, optionally having framework regions, FR1, FR2, FR3 and FR4, wherein FR1 has a sequence identity of at least 90% with SEQ ID No. 30, FR2 has a sequence identity of at least 90% with SEQ ID No. 32, FR3 has a sequence identity of at least 90% with SEQ ID No. 34, and/or FR4 has a sequence identity of at least 90% with SEQ ID No. 36,
(v) complementarity-determining regions CDR1, CDR2 and CDR3, wherein CDR1 has an amino acid sequence SEQ ID No. 40, CDR2 has an amino acid sequence SEQ ID No. 42, and CDR3 has an amino acid sequence SEQ ID No. 44, optionally having framework regions, FR1, FR2, FR3 and FR4, wherein FR1 has a sequence identity of at least 90% with SEQ ID No. 39, FR2 has a sequence identity of at least 90% with SEQ ID No. 41, FR3 has a sequence identity of at least 90% with SEQ ID No. 43, and/or FR4 has a sequence identity of at least 90% with SEQ ID No. 45,
(vi) complementarity-determining regions CDR1, CDR2 and CDR3, wherein CDR1 has an amino acid sequence SEQ ID No. 51, CDR2 has an amino acid sequence SEQ ID No. 53, and CDR3 has an amino acid sequence SEQ ID No. 55, optionally having framework regions, FR1, FR2, FR3 and FR4, wherein FR1 has a sequence identity of at least 90% with SEQ ID No. 50, FR2 has a sequence identity of at least 90% with SEQ ID No. 52, FR3 has a sequence identity of at least 90% with SEQ ID No. 54, and/or FR4 has a sequence identity of at least 90% with SEQ ID No. 56,
(vii) complementarity-determining regions CDR1, CDR2 and CDR3, wherein CDR1 has an amino acid sequence SEQ ID No. 60, CDR2 has an amino acid sequence SEQ ID No. 62, and CDR3 has an amino acid sequence SEQ ID No. 64, optionally having framework regions, FR1, FR2, FR3 and FR4, wherein FR1 has a sequence identity of at least 90% with SEQ ID No. 59, FR2 has a sequence identity of at least 90% with SEQ ID No. 61, FR3 has a sequence identity of at least 90% with SEQ ID No. 63, and/or FR4 has a sequence identity of at least 90% with SEQ ID No. 65, or
(viii) complementarity-determining regions CDR1, CDR2 and CDR3, wherein CDR1 has an amino acid sequence SEQ ID No. 71, CDR2 has an amino acid sequence SEQ ID No. 73, and CDR3 has an amino acid sequence SEQ ID No. 75, optionally having framework regions, FR1, FR2, FR3 and FR4, wherein FR1 has a sequence identity of at least 90% with SEQ ID No. 70, FR2 has a sequence identity of at least 90% with SEQ ID No. 72, FR3 has a sequence identity of at least 90% with SEQ ID No. 74, and/or FR4 has a sequence identity of at least 90% with SEQ ID No. 76.

3. The antibody or fragment thereof according to claim 1 or 2, the antibody or fragment thereof comprising
(i) complementarity-determining regions CDR1, CDR2 and CDR3, wherein CDR1 has an amino acid sequence SEQ ID No. 4, CDR2 has an amino acid sequence SEQ ID No. 6, and CDR3 has an amino acid sequence SEQ ID No. 8, optionally having framework regions, FR1, FR2, FR3 and FR4, wherein FR1 has a sequence identity of at least 95% with SEQ ID No. 3, FR2 has a sequence identity of at least 95% with SEQ ID No. 5, FR3 has a sequence identity of at least 95% with SEQ ID No. 7, and/or FR4 has a sequence identity of at least 95% with SEQ ID No. 9,
(ii) complementarity-determining regions CDR1, CDR2 and CDR3, wherein CDR1 has an amino acid sequence SEQ ID No. 13, CDR2 has an amino acid sequence SEQ ID No. 15, and CDR3 has an amino acid sequence SEQ ID No. 17, optionally having framework regions, FR1, FR2, FR3 and FR4, wherein FR1 has a sequence identity of at least 95% with SEQ ID No. 12, FR2 has a sequence identity of at least 95% with SEQ ID No. 14, FR3 has a sequence identity of at least 95% with SEQ ID No. 16, and/or FR4 has a sequence identity of at least 95% with SEQ ID No. 18,
(iii) complementarity-determining regions CDR1, CDR2 and CDR3, wherein CDR1 has an amino acid sequence SEQ ID No. 22, CDR2 has an amino acid sequence SEQ ID No. 24, and CDR3 has an amino acid sequence SEQ ID No. 26, optionally having framework regions, FR1, FR2, FR3 and FR4, wherein FR1 has a sequence identity of at least 95% with SEQ ID No. 21, FR2 has a sequence identity of at least 95% with SEQ ID No. 23, FR3 has a sequence identity of at least 95% with SEQ ID No. 25, and/or FR4 has a sequence identity of at least 95% with SEQ ID No. 27,
(iv) complementarity-determining regions CDR1, CDR2 and CDR3, wherein CDR1 has an amino acid sequence SEQ ID No. 31, CDR2 has an amino acid sequence SEQ ID No. 33, and CDR3 has an amino acid sequence SEQ ID No. 35, optionally having framework regions, FR1, FR2, FR3 and FR4, wherein FR1 has a sequence identity of at least 95% with SEQ ID No. 30, FR2 has a sequence identity of at least 95% with SEQ ID No. 32, FR3 has a sequence identity of at least 95% with SEQ ID No. 34, and/or FR4 has a sequence identity of at least 95% with SEQ ID No. 36,
(v) complementarity-determining regions CDR1, CDR2 and CDR3, wherein CDR1 has an amino acid sequence SEQ ID No. 40, CDR2 has an amino acid sequence SEQ ID No. 42, and CDR3 has an amino acid sequence SEQ ID No. 44, optionally having framework regions, FR1, FR2, FR3 and FR4, wherein FR1 has a sequence identity of at least 95% with SEQ ID No. 39, FR2 has a sequence identity of at least 95% with SEQ ID No. 41, FR3 has a sequence identity of at least 95% with SEQ ID No. 43, and/or FR4 has a sequence identity of at least 95% with SEQ ID No. 45,
(vi) complementarity-determining regions CDR1, CDR2 and CDR3, wherein CDR1 has an amino acid sequence SEQ ID No. 51, CDR2 has an amino acid sequence SEQ ID No. 53, and CDR3 has an amino acid sequence SEQ ID No. 55, optionally having framework regions, FR1, FR2, FR3 and FR4, wherein FR1 has a sequence identity of at least 95% with SEQ ID No. 50, FR2 has a sequence identity of at least 95% with SEQ ID No. 52, FR3 has a sequence identity of at least 95% with SEQ ID No. 54, and/or FR4 has a sequence identity of at least 95% with SEQ ID No. 56,
(vii) complementarity-determining regions CDR1, CDR2 and CDR3, wherein CDR1 has an amino acid sequence SEQ ID No. 60, CDR2 has an amino acid sequence SEQ ID No. 62, and CDR3 has an amino acid sequence SEQ ID No. 64, optionally having framework regions, FR1, FR2, FR3 and FR4, wherein FR1 has a sequence identity of at least 95% with SEQ ID No. 59, FR2 has a sequence identity of at least 95% with SEQ ID No. 61, FR3 has a sequence identity of at least 95% with SEQ ID No. 63, and/or FR4 has a sequence identity of at least 95% with SEQ ID No. 65, or
(viii) complementarity-determining regions CDR1, CDR2 and CDR3, wherein CDR1 has an amino acid sequence SEQ ID No. 71, CDR2 has an amino acid sequence SEQ ID No. 73, and CDR3 has an amino acid sequence SEQ ID No. 75, optionally having framework regions, FR1, FR2, FR3 and FR4, wherein FR1 has a sequence identity of at least 95% with SEQ ID No. 70, FR2 has a sequence identity of at least 95% with SEQ ID No. 72, FR3 has a sequence identity of at least 95% with SEQ ID No. 74, and/or FR4 has a sequence identity of at least 95% with SEQ ID No. 76.

4. The antibody or fragment thereof according to at least one of the preceding claims, the antibody or fragment thereof comprising
(i) a sequence of SEQ ID No. 2,
(ii) a sequence of SEQ ID No. 11,
(iii) a sequence of SEQ ID No. 20,
(iv) a sequence of SEQ ID No. 29,
(v) a sequence of SEQ ID No. 38,
(vi) a sequence of SEQ ID No. 47,
(vii) a sequence of SEQ ID No. 49,
(viii) a sequence of SEQ ID No. 58,
(ix) a sequence of SEQ ID No. 67,
(x) a sequence of SEQ ID No. 69, or
(xi) a sequence identity of at least 90%, 95%, 97%, or 99% with one of the above-mentioned sequences (i) to (x).

5. The antibody or fragment thereof according to at least one of the preceding claims, wherein cumulatively across all CDRs, optionally excluding CDR3, up to 3, up to 2 or up to 1 amino acid is eliminated or substituted, wherein the substitution optionally is an empirical, strictly conservative, conservative or moderately conservative substitution.

6. The antibody or fragment thereof according to at least one of the preceding claims, wherein cumulatively across all FRs up to 5, up to 3, up to 2 or up to 1 amino acid is eliminated or substituted, wherein the substitution optionally is an empirical, strictly conservative, conservative or moderately conservative substitution.

7. The antibody or fragment thereof according to at least one of the preceding claims, wherein
(i) the antibody or fragment thereof is independently chosen from the list consisting of: a monovalent single-domain antibody, a multivalent single-domain antibody, a monovalent human recombinant heavy chain antibody, and a multivalent human recombinant heavy chain antibody;
(ii) the antibody or fragment thereof is independently chosen from the list consisting of: IgG1, IgG2, IgG3, IgG4, IgA1, IgA2, IgM, IgD, IgE, Fc-engineered IgG, single-armed or monovalent IgG, pH-dependent antibodies or fragments, Fc-silenced IgGs, IgG fusion proteins, monovalent single-domain antibodies, multivalent single-domain antibodies, sdAb (single-domain antibodies), Fab (Fragment antigen-binding), Fab' (Fab prime), Fv (Fragment variable), scFv (single-chain variable fragment), F(ab')2, diabodies, tandem scFv, BiTE (bispecific T-cell Engager), TandAb (tandem diabody), triabody, antibody tetramer, pentabody, bivalent single-domain antibody, trivalent single-domain antibody, tetravalent single-domain antibody, pentavalent single-domain antibody, hexavalent single-domain antibody, hrHcAb (human recombinant heavy chain antibody), monovalent hrHcAb, multivalent hrHcAb, bivalent hrHcAb, trivalent hrHcAb, tetravalent hrHcAb, pentavalent hrHcAb, hexavalent hrHcAb, bispecific hrHcAb, trispecific hrHcAb, tetraspecific hrHcAb, monovalent minibodies, multivalent minibodies, bivalent minibodies, trivalent minibodies, tetravalent minibodies, pentavalent minibodies, hexavalent minibodies, bispecific minibodies, trispecific minibodies, tetraspecific minibodies, bivalent Chimeric Antigen Receptor (CAR), trivalent Chimeric Antigen Receptor (CAR), inverted IgG fragments, bispecific IgG (heterodimeric IgG), bispecific IgG1, bispecific IgG2, bispecific IgG3, bispecific IgG4, bispecific IgA1, bispecific IgA2, bispecific IgM, bispecific IgD, bispecific IgE, bispecific Fc-engineered IgG, bispecific pH-dependent antibodies or fragments, bispecific Fc-silenced IgG, bispecific IgG fusion proteins, bispecific diabodies, DART (Dual Affinity ReTargeting), DVD-Ig (Dual-Variable Domain Ig), Dual Action Fab, bispecific single-domain antibodies, Quadroma, orthogonal Fab, bispecific Chimeric Antigen Receptor (CAR), half-antibodies, trispecific IgG1, trispecific IgG2, trispecific IgG3, trispecific IgG4, trispecific IgA1, trispecific IgA2, trispecific IgM, trispecific IgD, trispecific IgE, TriKE (Trispecific Killer Engager), trispecific single-domain antibodies, trispecific Chimeric Antigen Receptor (CAR), trispecific Fc-engineered IgG, trispecific pH-dependent antibodies or fragments, trispecific Fc-silenced IgG, trispecific IgG fusion proteins, tetraspecific single-domain antibody, antibody-drug conjugates carrying MMAF, antibody-drug conjugates carrying MMAE, antibody-drug conjugates carrying DM1, antibody-drug conjugates carrying DM4, antibody-drug conjugates carrying pyrrolobenzodiazepine dimers, antibody-drug conjugates carrying duocarmycins, antibody-drug conjugates carrying SN-38, antibody-drug conjugates carrying amanitin, antibody-drug conjugates carrying immune stimulators, antibody-drug conjugates carrying microtubule inhibitors, antibody-drug conjugates carrying auristatins, antibody-drug conjugates carrying maytansinoids, antibody-drug conjugates carrying topoisomerase I inhibitors, antibody-drug conjugates carrying RNA polymerase II inhibitors, immunocytokines fusion proteins, antibody-enzyme fusion proteins, antibody-drug-metabolizing enzymes fusion proteins, immunotoxin fusion proteins, monomeric single-domain antibody, dimeric single-domain antibody, trimeric single-domain antibody, tetrameric single-domain antibody, pentameric single-domain antibody, hexameric single-domain antibody, multimeric single-domain antibody, bivalent scFv and CH3 domain of IgG fusion, bispecific scFv and CH3 domain of IgG fusion, bivalent scFv Fc domain fusion, bispecific scFv Fc domain fusion, trivalent scFv and trimerization domain fusion, trispecific scFv and trimerization domain fusion, tetravalent scFv and tetramerization domain fusion, tetraspecific scFv and tetramerization domain fusion, bivalent sdAb and CH3 domain of IgG fusion, bispecific sdAb and CH3 domain of IgG fusion, bivalent sdAb Fc domain fusion, bispecific sdAb Fc domain fusion, trivalent sdAb and trimerization domain fusion, trispecific sdAb and trimerization domain fusion, tetravalent sdAb and tetramerization domain fusion, tetraspecific sdAb and tetramerization domain fusion and/or variations thereof and/or recombinant variations thereof and/or humanized variations thereof.;
(iii) the antibody or fragment thereof is humanized, recombinant and/or isolated; and/or
(iv) the CD38 is human CD38 and/or comprises SEQ ID No. 77.

8. The antibody or fragment thereof according to at least one of the preceding claims, wherein the single-domain antibody binds to CD38 with an affinity of at most 1 µM, at most 1 nM, or at most 100 pM.

9. The antibody or fragment thereof according to at least one of the preceding claims, wherein
(i) the antibody or fragment thereof competes with daratumumab for binding to CD38, in particular human CD38,
(ii) the antibody or fragment thereof does not compete with isatuximab for binding to CD38, in particular human CD38,
(iii) the size of the antibody or fragment thereof ranges from 10.0 kDa to 170.0 kDa, such as from 10.0 kDa to 18.0 kDa, or from 70.0 kDa to 100.0 kDa, or from 130.0 kDa to 170.0 kDa,
(v) the antibody or fragment thereof is stable at temperatures of up to 65 to 70, preferred 70 to 75, most preferred 75 °C or higher, and/or
(vi) the antibody or fragment thereof is conjugated to at least one effector molecule, optionally selected from the group consisting of: a detectable label, a radionuclide, a cytotoxic drug or combinations thereof.

10. The antibody or fragment thereof according to one or more of the preceding claims for use in a method of therapy, diagnosis *in vivo* or *in vitro* or surgery, wherein
(iii) optionally the antibody or fragment thereof is for use in theranostics,
(iv) optionally the method comprises imaging by Positron Emission Tomography or Single-Photon Emission Computed Tomography, in particular when the antibody or fragment is conjugated to a radionuclide, and/or
(v) optionally, the *in vitro* method is a method for analysis or diagnosis of a disease in a sample, comprising contacting the antibody or fragment thereof according to one or more of claims 1 to 9 with the sample.

11. The antibody or fragment thereof according to one or more of claims 1 to 10 for use in
(v) a method of treating hematologic diseases, such as CD38 positive cancer, cancer overexpressing CD38, Multiple Myeloma (MM), Chronic Lymphatic Leukemia (CLL), Acute Lymphoblastic Leukemia (ALL), Non-Hodgkin's Lymphoma (NHL), Diffuse Large B-cell Lymphoma (DLBCL) or Light Chain Amyloidosis;
(vi) a method of treating autoimmune diseases such as Rheumatoid Arthritis (RA);
(vii) a method of treating neurodegenerative diseases such as Alzheimer's Disease (AD), Parkinson's Disease (PD), Amyotrophic Lateral Sclerosis (ALS) or Multiple Sclerosis (MS); or
(viii) a method of immunosuppression, e.g. preventing transplant rejection following organ transplantation,
wherein
• optionally the antibody or fragment thereof is used in combination with isatuximab;
• optionally the antibody or fragment thereof is used as an RNA-vaccine; and/or
• optionally the antibody or fragment thereof is used in CAR T-cell therapy.

12. A nucleic acid molecule comprising at least one nucleic acid sequence encoding at least partially the antibody or fragment thereof according to one or more of claims 1 to 9.

13. A host cell comprising a vector, the vector comprising at least one nucleic acid molecule comprising at least one nucleic acid sequence encoding at least partially the antibody or fragment thereof according to one or more of claims 1 to 9, wherein optionally the cell is a eukaryotic or prokaryotic cell.

14. A method of manufacturing the antibody or fragment thereof according to one or more of claims 1 to 9, comprising expressing the antibody or fragment thereof in a host cell, optionally in a host cell according to claim 13, and isolating the antibody or fragment thereof.

15. A pharmaceutical composition comprising the antibody or fragment thereof according to one or more of claims 1 to 9, or the nucleic acid molecule according to claim 12, and a pharmaceutically acceptable excipient.
